# EUROPEAN PATENT SPECIFICATION

(11) **EP 2 890 809 B1**
(45) Date of publication and mention of the grant of the patent: **01.02.2017**
(21) Application number: 13750557.4
(22) Date of filing: 16.08.2013
(51) Int. Cl.: C12Q 1/68, C12N 15/10

(54) **METHOD OF DETERMINING THE PRESENCE OR ABSENCE OF A TARGET NUCLEIC ACID IN A CELL SAMPLE**
VERFAHREN ZUR BESTIMMUNG DES VORHANDENSEINS ODER NICHTVORHANDENSEINS EINER TARGET-NUKLEINSÄURE IN EINER ZELLPROBE
PROCÉDÉ DE DÉTERMINATION DE LA PRÉSENCE OU DE L'ABSENCE D'UN ACIDE NUCLÉIQUE CIBLE DANS UN ÉCHANTILLON CELLULAIRE

(30) Priority: 30.08.2012 EP 12182339; 30.08.2012 US 201261695042 P
(43) Date of publication of application: 08.07.2015
(73) Proprietor: Qiagen GmbH, 40724 Hilden (DE)
(72) Inventor: SPRENGER-HAUSSELS, Markus, 40724 Hilden (DE); KUPFER, Christian, 40724 Hilden (DE); SCHATZ, Peer, 40724 Hilden (DE)
(74) Representative: Roth, Carla
(86) International application number: PCT/EP2013/067117
(87) International publication number: WO 2014/032990

(56) References cited:
- WO-A1-00/70040
- WO-A2-2004/101809
- QIAGEN: "QIASymphony DSP AxpH DNA Kit Handbook", QIAGEN , 1 April 2012 (2012-04-01), XP002693806, Retrieved from the Internet: URL:http://www.qiagen.com/resources/Downlo ad.aspx?id={B30E3BFF-A9AB-49D3-9B76-655AA4 DEDA52}&lang=en&ver=1 [retrieved on 2013-03-13]
- QIAGEN: "H2C High Risk HPV DNA Test", QIAGEN , 1 March 2008 (2008-03-01), XP002693807, Retrieved from the Internet: URL:http://www.qiagen.com/resources/Downlo ad.aspx?id={DED1FC02-4A2E-4E6E-9C27-1E0519 605B5D}&lang=en&ver=1 [retrieved on 2013-03-14]

## Description

### FIELD OF THE INVENTION

The present invention pertains to a method for detecting the presence or absence of a target nucleic acid in a cell containing liquid sample.

### BACKGROUND OF THE INVENTION

For detecting the presence or absence of a target nucleic acid several methods are known in the prior art that are based on capturing a hybrid comprising the target nucleic acid to be detected, herein referred to as hybrid capturing assay. The basis for the respective technology is for example described in WO 93/10263 and further developments thereof in WO 2010/127223. The respective technology is used for detecting and characterising specific nucleic acid sequences and sequence changes for example for detecting the presence or absence of viral or bacterial nucleic acid sequences indicative of an infection, the presence of variants of alleles of mammalian genes associated with disease and cancer and identification of the source of nucleic acid found in forensic samples, as well as in fetal diagnostics for example in paternity determinations. Said technology that is based on capturing a hybrid which can be used for detecting DNA or RNA (reverse hybrid capturing) is wide spread, in particular in the field of clinical diagnostics. An FDA approved assay is the *digene* HC2 HPV DNA Test, which uses a manual conversion procedure for processing both PRESERVCYT® and SUREPATH® LBC media for subsequent testing by the digene HC2 High-Risk HPV DNA TEST assay. The respective manual protocols require a time consuming, manual conversion method, including many repetitive motions. To collect the cells from the clinical samples, they must be centrifuged, the supernatant must be removed and the cells are then prepared for the subsequent assay. High volume laboratories which process 500 to 2500 specimens per day face due to the repetitive manual steps a significant number of ergonomic related injuries such as for example carpal tunnel syndrome, which exists at lesser extend also in lower volume accounts as well. Thus, there is a high demand to avoid manual steps and to automate the sample preparation.

An automated method for preparing samples for a respective hybrid capturing assay was developed by QIAGEN under the name of QIASYMPHONY® AXpH DNA procedure. This is an anion exchange chromatography-based DNA extraction protocol, which results in automated sample preparation of up to 96 samples in 4.5 to 6.5 hours depending on the used cytology medium. Samples that were collected in SUREPATH® medium require due to formaldehyde fixation a modified protocol with extended lysis time and the addition of proteinase K to purify the DNA from the formaldehyde fixed SUREPATH® samples. This prolonged run time does not allow customers to process a full plate of 88 samples plus calibrators and controls including the hc2 assay within one shift (8 hours). Thus, there is an unaddressed customer need to achieve faster turnaround times at high throughput HPV test sides and to reduce the amount of manual interventions.

The QIASYMPHONY® AXpH DNA technology is discussed in the QIASYMPHONY® DSP AXpH DNA kit handbook.

The document entitled "H2C High-Risk HPV DNA Test" (QIAGEN) relates to an *in vitro* nucleic acid hybridization assay for HPV detection. WO 00/70040 A1 discloses *inter alia* methods for using paramagnetic particles to concentrate or harvest cells.

It is the object of the present invention to provide a method for detecting the presence or absence of a target nucleic acid in a cell sample, which is rapid and suitable for automation.

### SUMMARY OF THE INVENTION

The invention can be defined by the appending claims.

The present invention provides a rapid and automatable method, wherein cells, such as epithelial cells originating from cervical swap samples, are collected from the surrounding liquid medium by binding them to an anion exchange surface. The present invention is particularly suitable for collecting cells from a liquid-based cytology medium. The cells bind directly with high affinity and quick kinetics to the anion exchange surface which preferably is provided by magnetic particles carrying anion exchange moieties. The cells that are bound to the anion exchange surface can be easily separated from the surrounding medium and can be directly resuspended in a composition that is suitable for a subsequent hybrid capturing assay which can be performed to detect nucleic acids such as e.g. pathogen nucleic acids, in particular viral nucleic acids or cellular nucleic acids. Additionally, it was surprisingly found that it is not necessary to actively remove, respectively separate the anion exchange surface prior to performing the hybrid capturing assay. This significantly reduces the necessary preparation steps. Therefore, the method according to the present invention provides a considerable improvement over existing manual or automated sample preparation protocols in that it is rapid, automatable and requires only few preparation steps. In contrast to prior art methods, no time-consuming manual cell preparation steps are required and furthermore, no specific pre-treatments such as an adjustment of binding conditions are necessary. This saves chemistry and furthermore, has the advantage that the whole operating volume of a robotic system can be used for the sample volume. As is shown by the examples, the method provides reliable results and is not susceptible to errors which are important characteristics in the field of diagnostics. Thus, the present invention provides a solution to the above described customer needs.

According to a first aspect, a method of determining the presence or absence of a target nucleic acid in a cell sample is provided, said method comprising:
a) contacting magnetic particles having a surface comprising anion exchange moieties with the cell sample under conditions suitable to induce binding between the cells and said surface;
b) separating the magnetic particles with the bound cells from the remaining sample to collect the cells, wherein the magnetic particles with the bound cells are processed by the aid of a magnetic field;
c) releasing nucleic acids from the cells;
d) generating a hybrid between the target nucleic acid and a probe specific for the target nucleic acid in the presence of the magnetic particles;
e) detecting the presence or absence of the hybrid.

As is shown by the examples, the respective method allows to rapidly prepare cell samples for a hybrid capturing assay and furthermore, allows to rapidly perform a hybrid capturing assay. The method is reliable, rapid and suitable for automation. Therefore, it is particularly suitable in the field of diagnostics. Furthermore, the method is particularly useful for high-throughput applications.

Also provided is the use of a kit in the method according to the invention, wherein the kit comprises:
a) magnetic particles, wherein the surface of the magnetic particles comprise anion exchange moieties;
b) a denaturation agent which is an alkaline solution comprising a base; and
c) a composition comprising a chaotropic agent.

Disclosed is a kit suitable for use in the method according to the present invention, comprising:
a) magnetic particles, wherein the surface of the magnetic particles comprise anion exchange moieties;
b) a denaturation agent which is an alkaline solution comprising a base, preferably NaOH or KOH; and
c) a composition comprising a chaotropic agent and optionally one or more additives selected from the group of chelating agents, buffering agents and preservatives.

A respective kit is useful for rapidly preparing a cell sample for a hybrid capturing assay. Preferably, said kit is provided in form of a cartridge, wherein the kit components a) to c) are comprised in separate troughs of said cartridge. A respective cartridge is suitable for use in a robotic system that is capable of processing magnetic particles.

Also disclosed is a kit for performing a hybrid capturing assay, comprising:
a) magnetic particles, wherein the surface of the magnetic particles comprise anion exchange moieties;
b) at least one probe suitable for hybridizing to a target nucleic acid; and
c) at least one binding agent which is capable of binding a double-stranded nucleic acid hybrid.

Other objects, features, advantages and aspects of the present application will become apparent to those skilled in the art from the following description and appended claims.

### BRIEF DESCRIPTION OF THE FIGURES

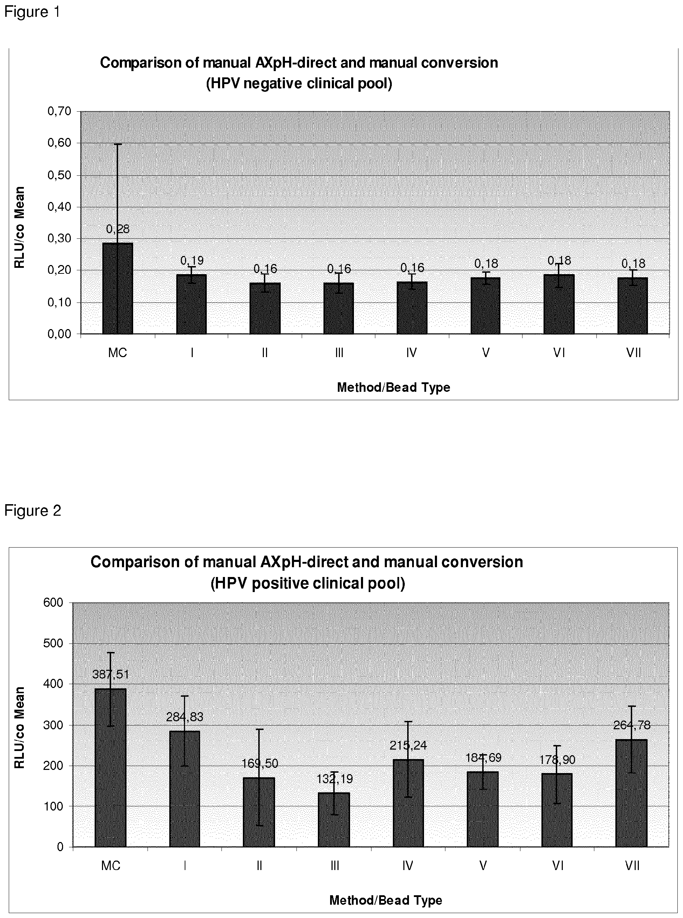

**Figure 1****:** Comparison of manual AXpH-direct and manual conversion I. The HPV assay results (RLU/CO [MW]) are shown for HPV negative clinical samples when applying different bead suspensions I to VII. Manual conversion (MC) served as reference.
**Figure 2****:** Comparison of manual AXpH-direct and manual conversion II. The HPV assay results (RLU/CO [MW]) are shown for HPV positive clinical samples when applying different bead suspensions I to VII. Manual conversion (MC) served as reference.

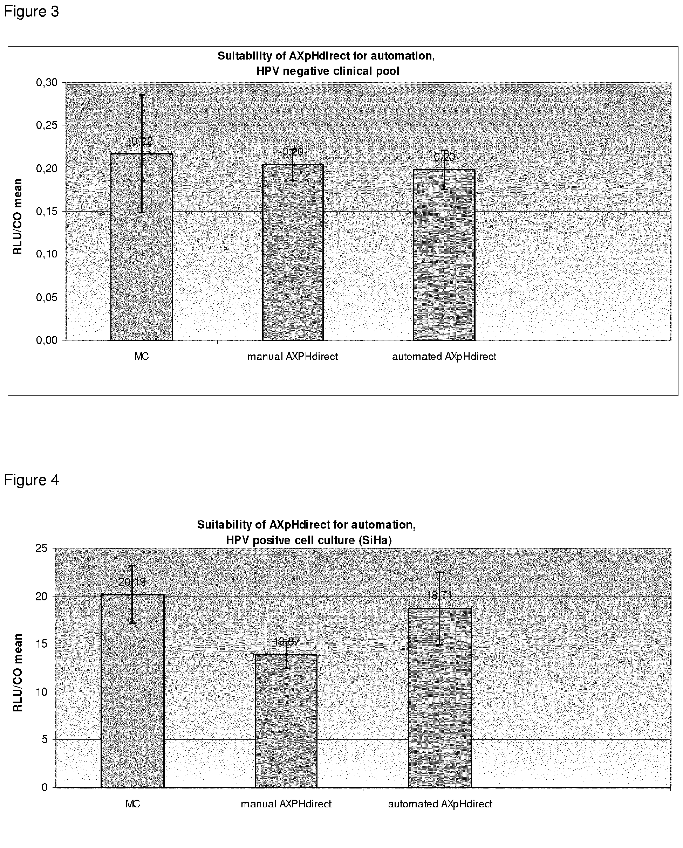

**Figure 3****:** Suitability of AXpH-direct for automation I. The HPV assay results (RLU/CO [MW]) are shown for HPV negative clinical samples when applying manual conversion (MC) as reference, a manual AXpH-direct protocol and an automated AXpH-direct protocol.
**Figure 4****:** Suitability of AXpH-direct for automation II. The HPV assay results (RLU/CO [MW]) are shown for HPV positive SiHa cell culture when applying manual conversion (MC) as reference, a manual AXpH-direct protocol and an automated AXpH-direct protocol.

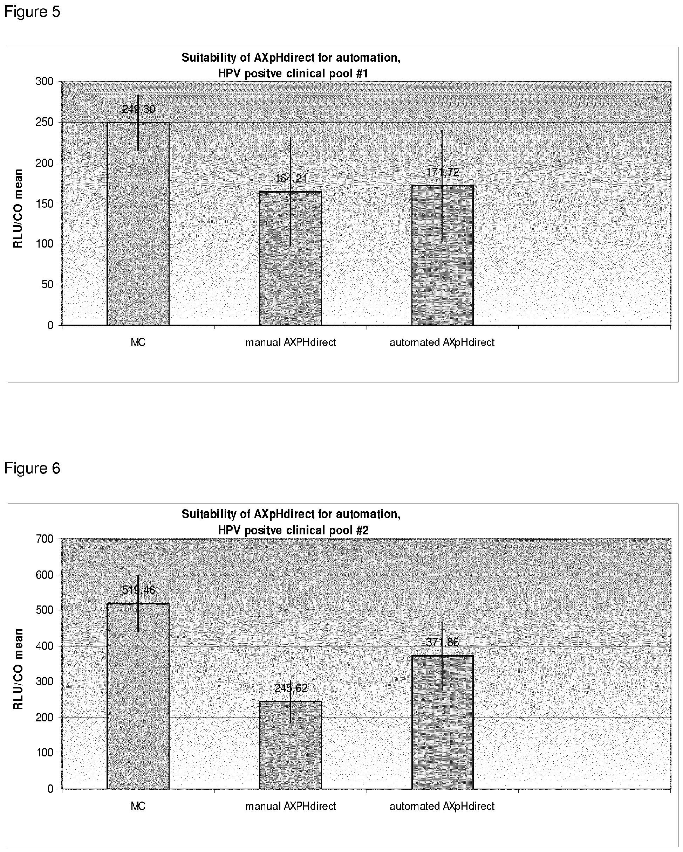

**Figure 5****:** Suitability of AXpH-direct for automation III. The HPV assay results (RLU/CO [MW]) are shown for HPV positive samples from a clinical pool (pool 1) when applying manual conversion (MC) as reference, a manual AXpH-direct protocol and an automated AXpH-direct protocol.
**Figure 6****:** Suitability of AXpH-direct for automation IV. The HPV assay results (RLU/CO [MW]) are shown for HPV positive samples from another clinical pool (pool 2) when applying manual conversion (MC) as reference, a manual AXpH-direct protocol and an automated AXpH-direct protocol.

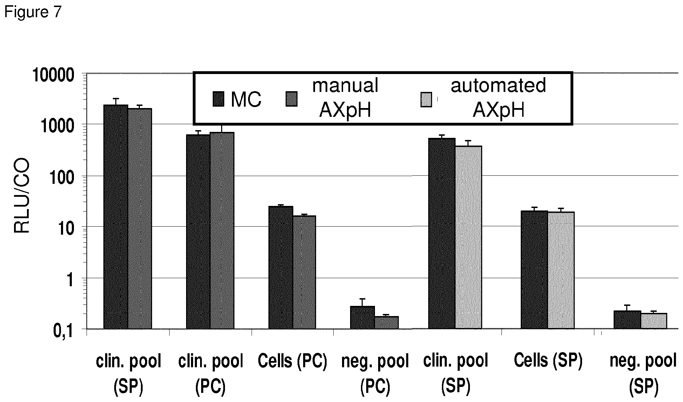

**Figure 7****:** Suitability of AXpH-direct for PRESERVCYT^{®} and SUREPATH^{®} samples. The HPV assay results (RLU/CO) are shown for different HPV positive (clin. pool) and negative clinical samples (neg. pool) as well as for HPV positive cell culture (Cells) when applying manual conversion (MC) as reference, a manual AXpH-direct protocol or an automated AXpH-direct protocol. The samples contained PRESERVCYT^{®} (PC) or SUREPATH^{®} (SP) medium.

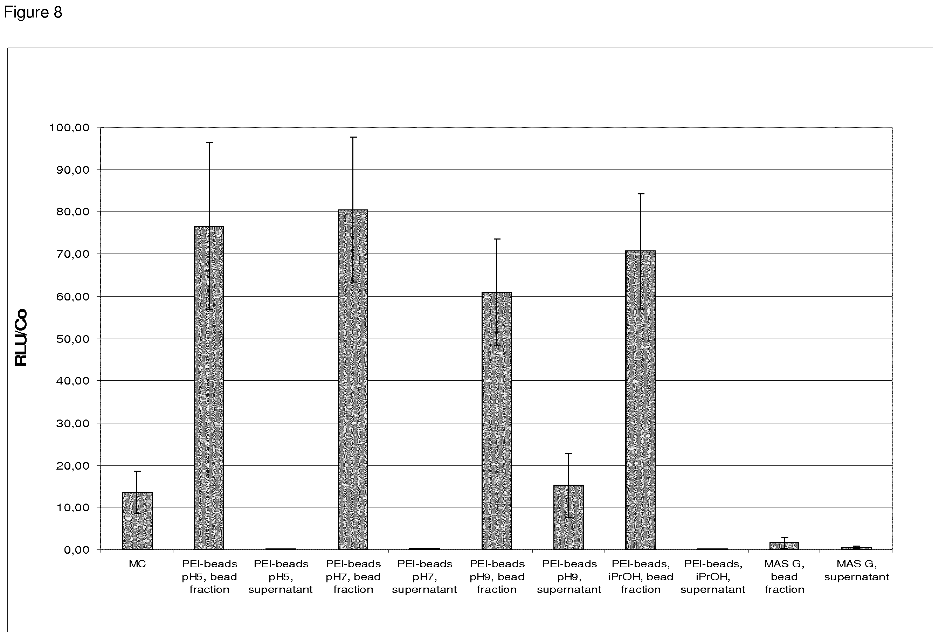

**Figure 8****:** Parameters responsible for the binding of the cells to the beads I. The HPV assay results (RLU/CO) are shown for the bead fractions and the supernatant fractions (SN) as well when applying different conditions for the binding of the cells to the beads (pH 5, 7 or 9, iPrOH = isopropanol). PEI modified beads or silica beads (MAS G) were used.

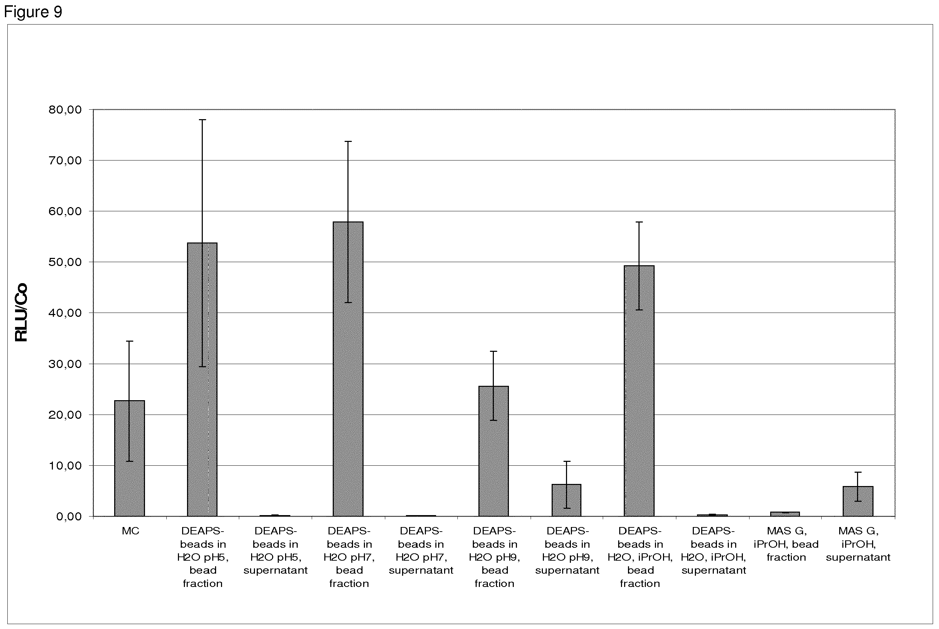

**Figure 9****:** Parameters responsible for the binding of the cells to the beads II. The HPV assay results (RLU/CO) are shown for the bead fractions and the supernatant fractions as well when applying different conditions for the binding of the cells to the beads (pH 5, 7 or 9, iPrOH = isopropanol). DEAPS modified beads or silica beads (MAS G) were used.

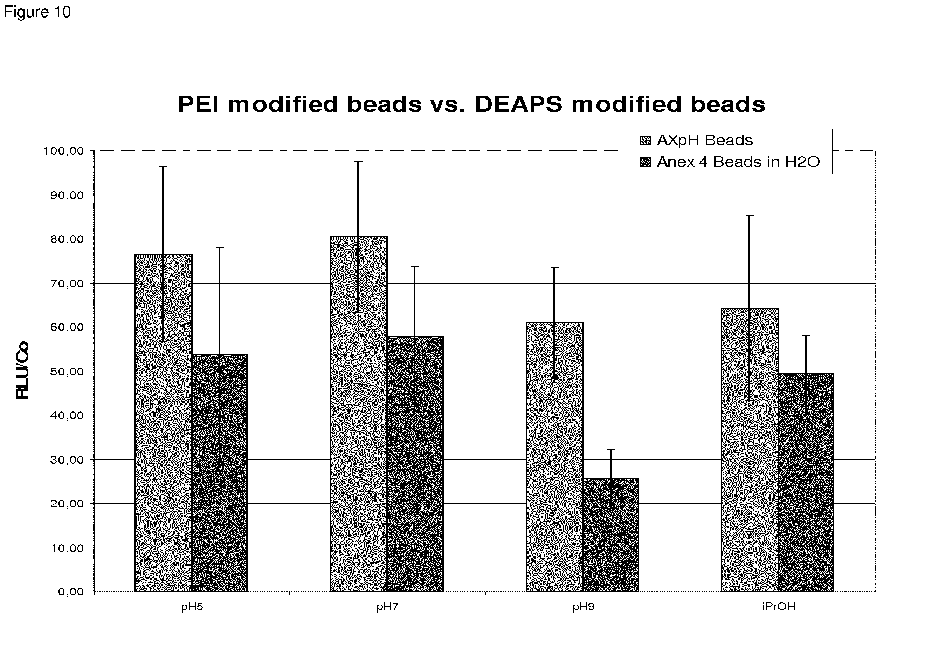

**Figure 10****:** Parameters responsible for the binding of the cells to the beads III. The HPV assay results (RLU/CO) are shown for the bead fractions when applying different conditions for the binding of the cells to the beads (pH 5, 7 or 9, iPrOH = isopropanol). PEI modified beads and DEAPS modified beads were compared.

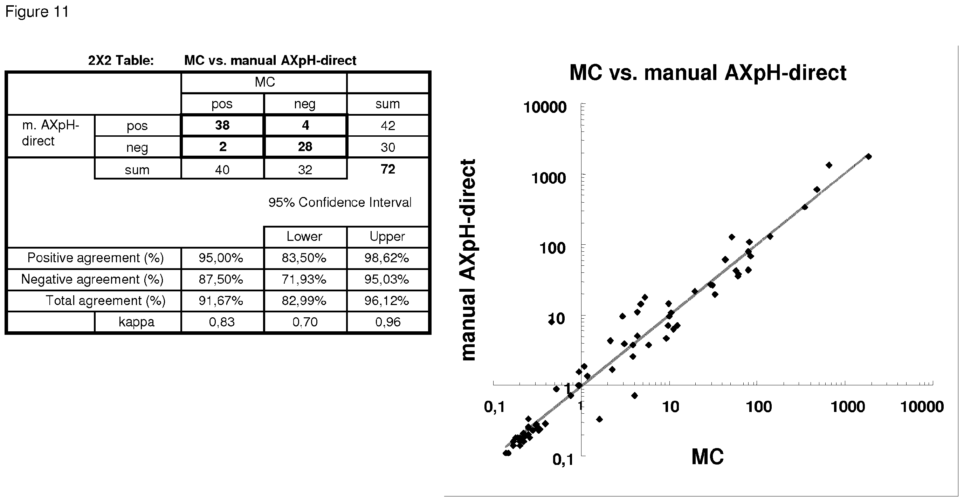

**Figure 11****:** Conformity of HPV assay results I. The HPV assay results (RLU/CO = 0,1-10,000) are shown for replicates of 72 individual samples. For comparison between the manual conversion (MC) and the manual AXpH-direct protocol, the respective RLU/CO values were depicted in a x-y-diagram. r² is the correlation coefficient.

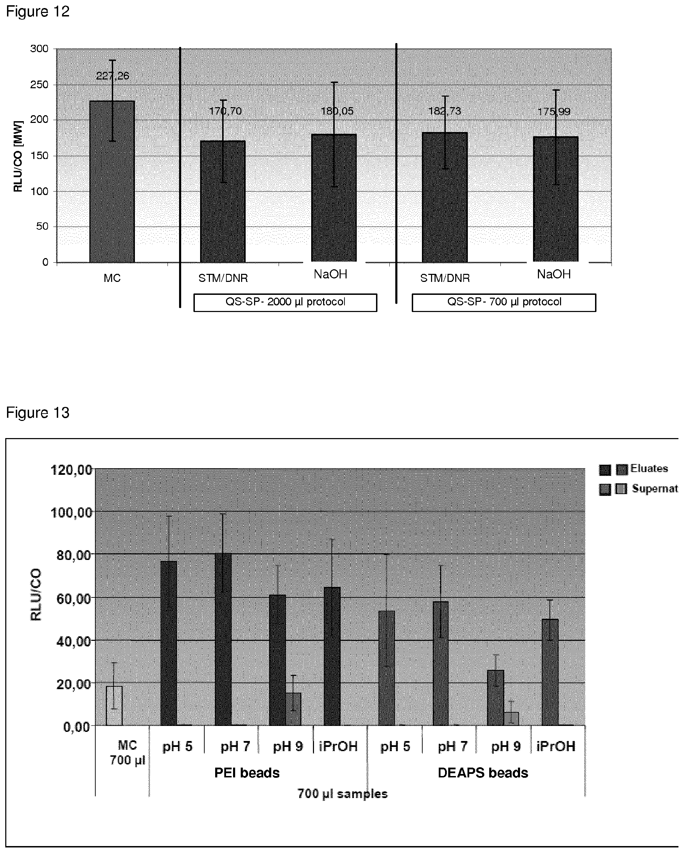

**Figure 12****:** Influence of elution buffer. The obtained HPV assay results (RLU/CO [MW]) are shown when subjecting 2000 µl or 700 µl of sample to the automated AXpH-direct protocol (QS-SP-2000 µl protocol or 700 µl protocol, respectively) and applying STM/DNR or NaOH as elution buffer. Manual conversion (MC) served as reference.
**Figure 13****:** Parameters responsible for the binding of the cells to the beads IV. The HPV assay results (RLU/CO) are shown for the bead fractions when applying different conditions for the binding of the cells to the beads (pH 5, 7 or 9, iPrOH = isopropanol). PEI modified beads and DEAPS modified beads were compared. Manual conversion (MC) with a sample input of 700 µl served as reference.

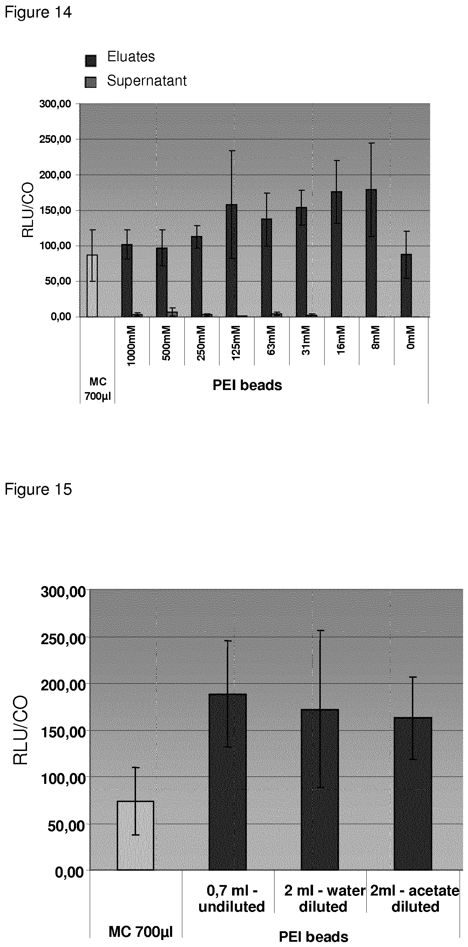

**Figure 14****:** Effect of conductivity during cell binding. The HPV assay results (RLU/CO) are shown for the eluate fractions and the supernatant fractions as well when applying different concentrations (0-1000 mM) of KH₂PO₄ during binding of the cells to the PEI modified beads. Manual conversion (MC) served as reference.
**Figure 15****:** Influence of sample acidification and dilution. The HPV assay results (RLU/CO) are shown for the eluate fractions when subjecting undiluted, water or acetate diluted samples to the manual AXpH-direct protocol. PEI modified beads were used. Manual conversion (MC) served as reference.

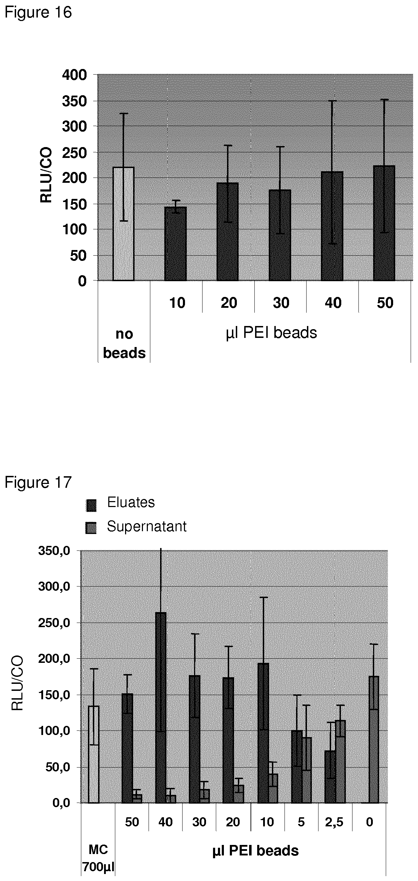

**Figure 16****:** Optimizing bead amounts I. The HPV assay results (RLU/CO) are shown for the eluate fractions when applying different volumes of suspensions of PEI modified beads. Manual conversion (no beads) served as reference.
**Figure 17****:** Optimizing bead amounts II. The HPV assay results (RLU/CO) are shown for the eluate and for the supernatant fractions as well when applying different volumes of suspensions of PEI modified beads. Manual conversion (MC) served as reference.

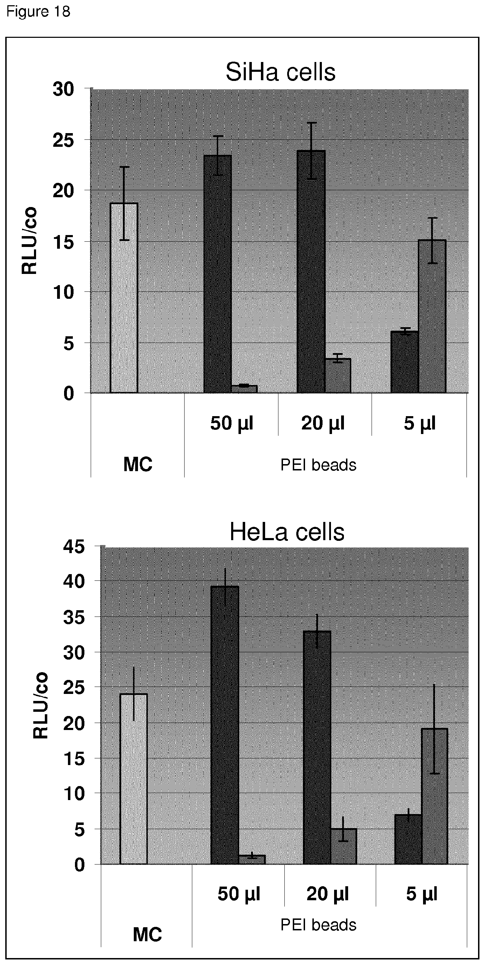

**Figure 18****:** Binding of cell culture controls. The HPV assay results (RLU/CO) are shown for the eluate and for the supernatant fractions as well when applying different volumes of suspensions of PEI modified beads. SiHa and HeLa cells were used as samples. Manual conversion (MC) served as reference.

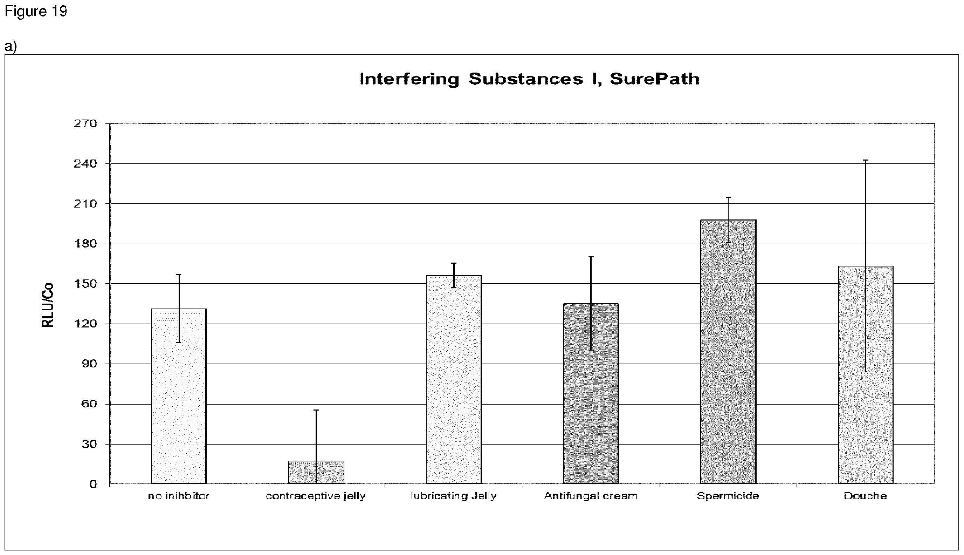

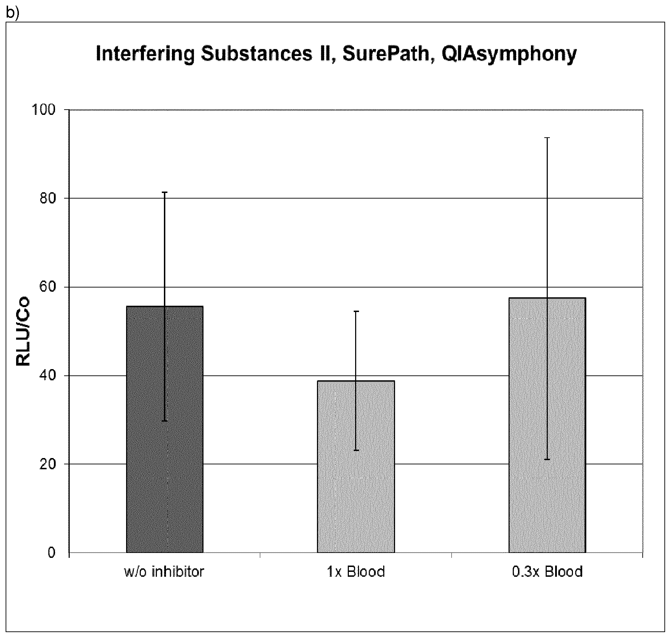

**Figure 19 a)** **and b):** Interfering substances. The obtained HPV assay results (RLU/CO) are shown when adding various interfering substances (CJ = contraceptive jelly, LJ = lubricant jelly, AC = antifungal cream, CS = contraceptive spermicide, D = douche) to the samples and applying the AXpH-direct method. Respective samples without interfering substances served as reference (w/o Inh.).

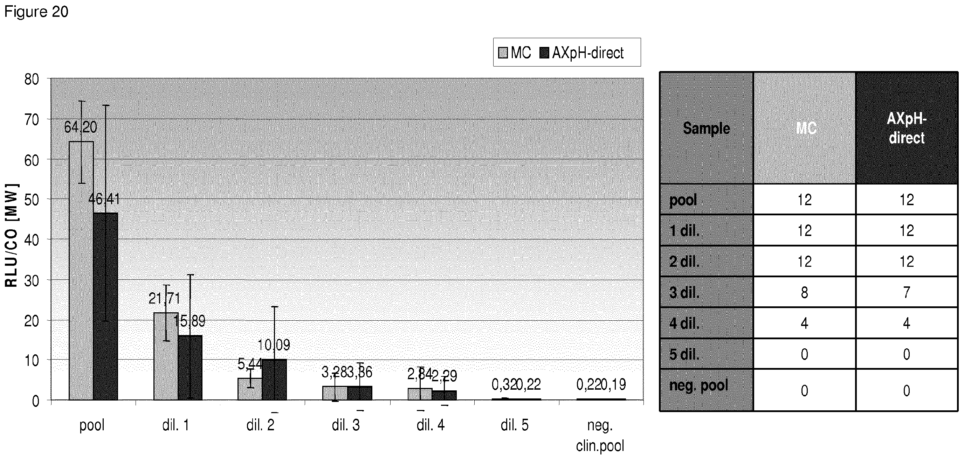

**Figure 20****:** Limit of detection. The obtained HPV assay results (RLU/CO) are shown when using various dilutions of samples from a HPV positive clinical pool (pool, dil. 1-5) and applying manual conversion (MC) or the AXpH-direct method. For every dilution, the samples from the positive clinical pool detected as positive may be seen from the table on the right. A HPV negative clinical pool (neg. pool) served as control.

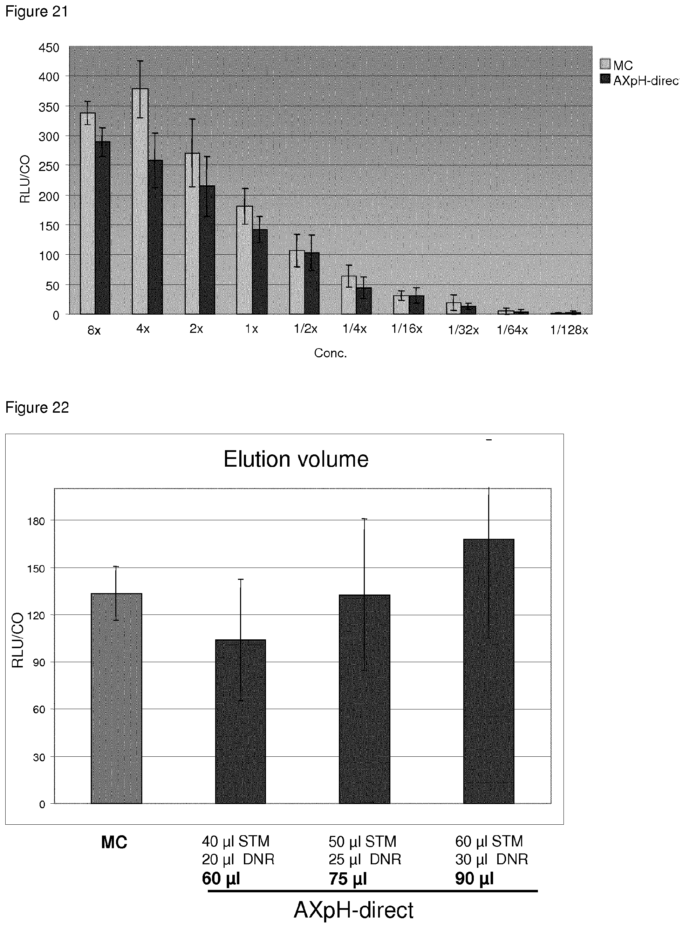

**Figure 21****:** Influence of cellularity on cell binding. The obtained HPV assay results (RLU/CO) are shown when using various dilutions of samples from a HPV positive clinical pool (8x - 1/128x) and applying manual conversion (MC) or the automated AXpH-direct method. A HPV negative clinical pool (neg. pool) served as control.
**Figure 22****:** Influence of elution volume. The obtained HPV assay results (RLU/CO) are shown when applying different volumes of STM/DNR for elution. Manual conversion (MC) served as reference.

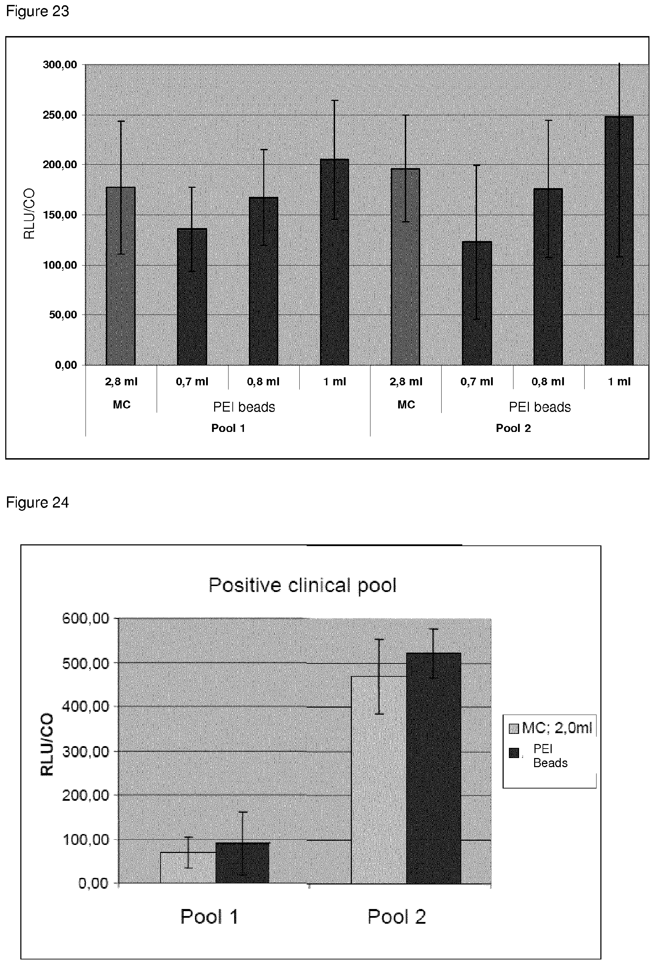

**Figure 23****:** Influence of sample input volume. The obtained HPV assay results (RLU/CO) are shown when using various volumes of sample input. Samples from two different clinical pools were employed (Pool 1 and pool 2). AXpH beads were used and manual conversion (MC) served as reference.
**Figure 24****:** Use of PEI modified beads for preparation of PRESERVCYT^{®} samples. The obtained HPV HC assay results (RLU/CO) are shown for two different HPV positive clinical pools (pool 1 and pool 2). Manual conversion (MC) with a sample input of 2.0 ml served as reference.

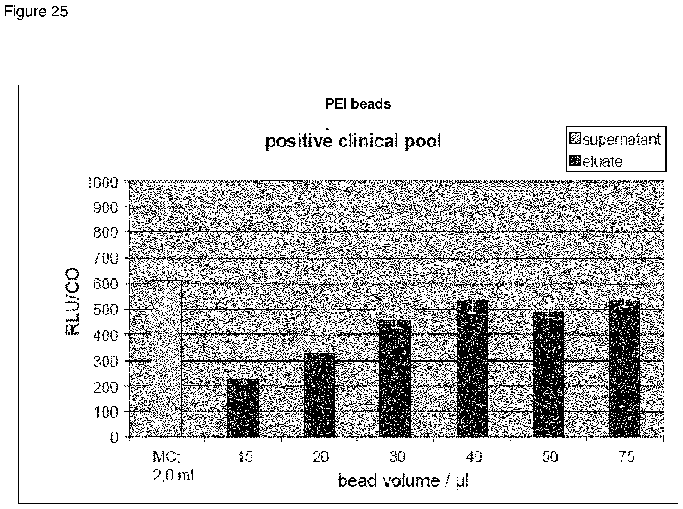

**Figure 25****:** Analysis of binding efficiency for PEI modified beads (PRESERVCYT^{®} samples). The obtained HPV HC assay results (RLU/CO) are shown for the supernatant and eluate fractions of samples from a clinical pool applying different volumes of bead suspensions (bead volume). Manual conversion (MC) with a sample input of 2.0 ml served as reference.

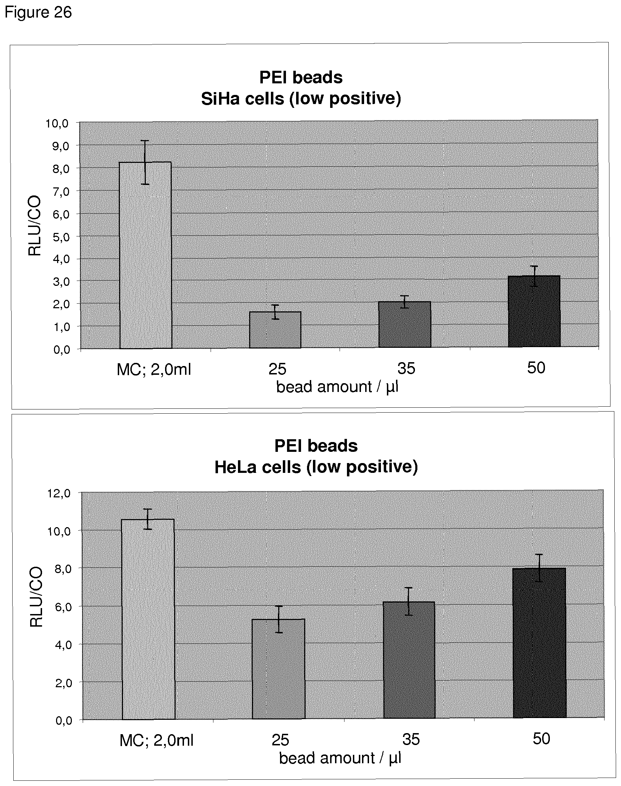

**Figure 26****:** Fine tuning of bead amounts for automated sample preparation I (PRESERVCYT^{®} samples). The obtained HPV HC assay results (RLU/CO) are shown for samples from a HPV low positive SiHa (top) as well as HeLa cell culture (bottom) applying different volumes of PEI modified bead suspensions (bead volume). Manual conversion (MC) with a sample input of 2.0 ml served as reference.

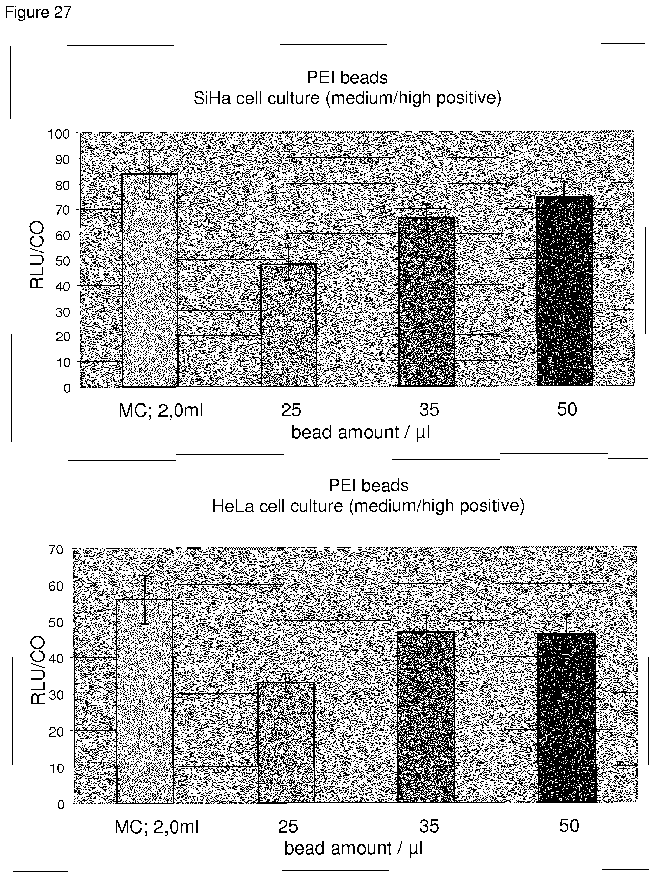

**Figure 27****:** Fine tuning of bead amounts for automated sample preparation II (PRESERVCYT^{®} samples). The obtained HPV HC assay results (RLU/CO) are shown for samples from a HPV medium/high positive SiHa (top) as well as HeLa cell culture (bottom) applying different volumes of PEI modified bead suspensions (bead volume). Manual conversion (MC) with a sample input of 2.0 ml served as reference.

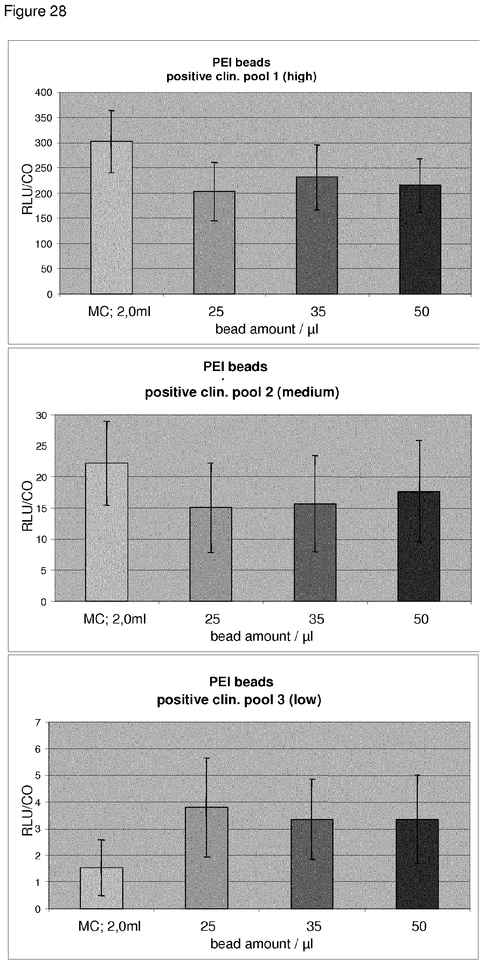

**Figure 28****:** Fine tuning of bead amounts for automated sample preparation III (PRESERVCYT^{®} samples). The obtained HPV HC assay results (RLU/CO) are shown for samples from a HPV high (top), medium (middle) as well as low (bottom) positive clinical pool applying different volumes of PEI modified bead suspensions (bead volume). Manual conversion (MC) with a sample input of 2.0 ml served as reference.

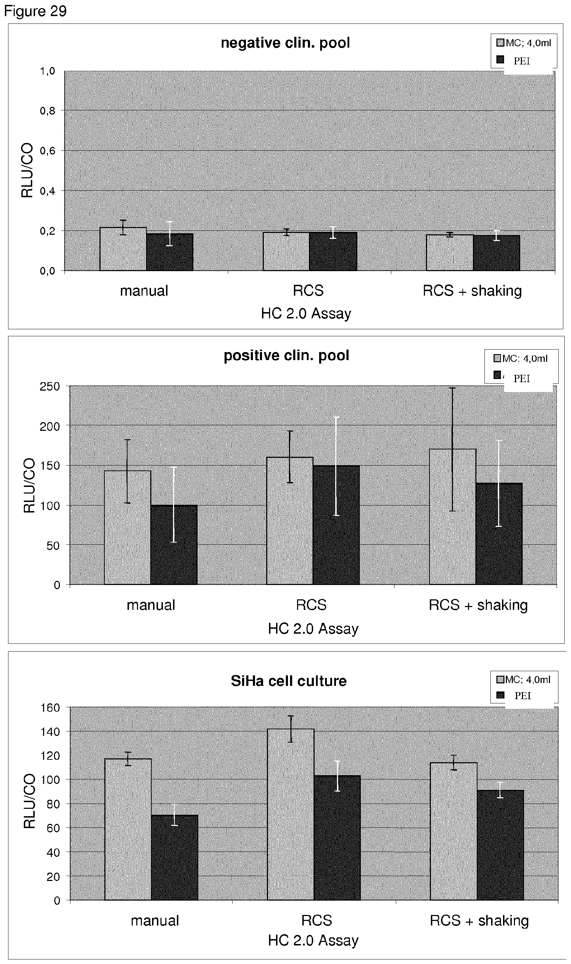

**Figure 29****:** Link between automated AXpH-direct method (AXpH) and RCS (Rapid Capture System) (PRESERVCYT^{®} samples). The obtained HPV HC assay results (RLU/CO) are shown for samples from a negative and a positive clinical pool as well as from SiHa cell culture. The execution of the HC 2.0 Assay was varied. Manual conversion (MC) with a sample input of 2.0 ml served as reference.

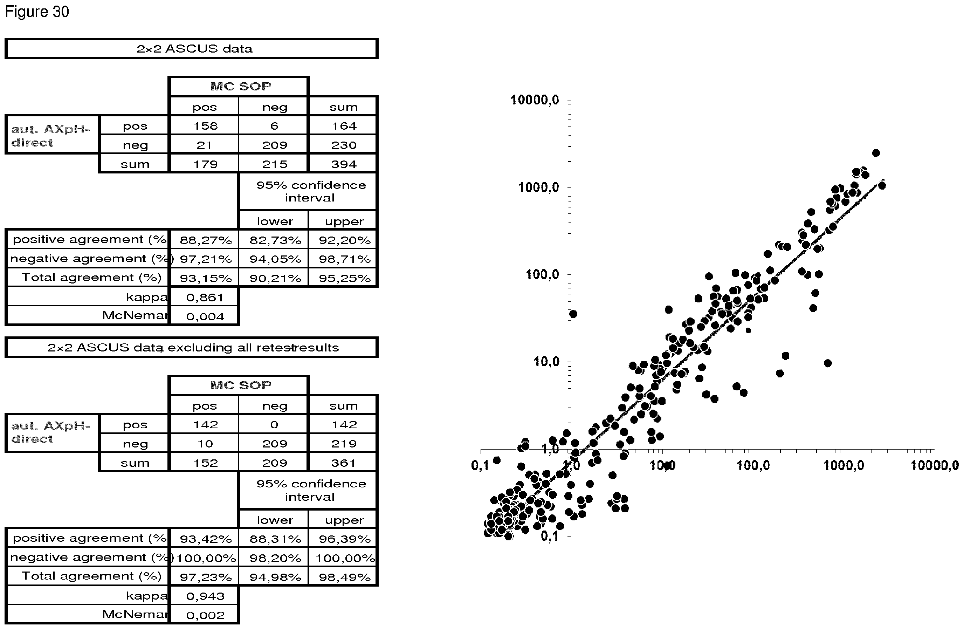

**Figure 30****:** Conformity with manual conversion (MC) referring to HPV HC assay results (PRESERVCYT^{®} samples). The HPV assay results are shown for replicates of 394 individual samples. For comparison between the manual conversion (MC) and the automated AXpH-direct protocol, the respective RLU/CO values were depicted in a x-y-diagram. r² is the correlation coefficient.

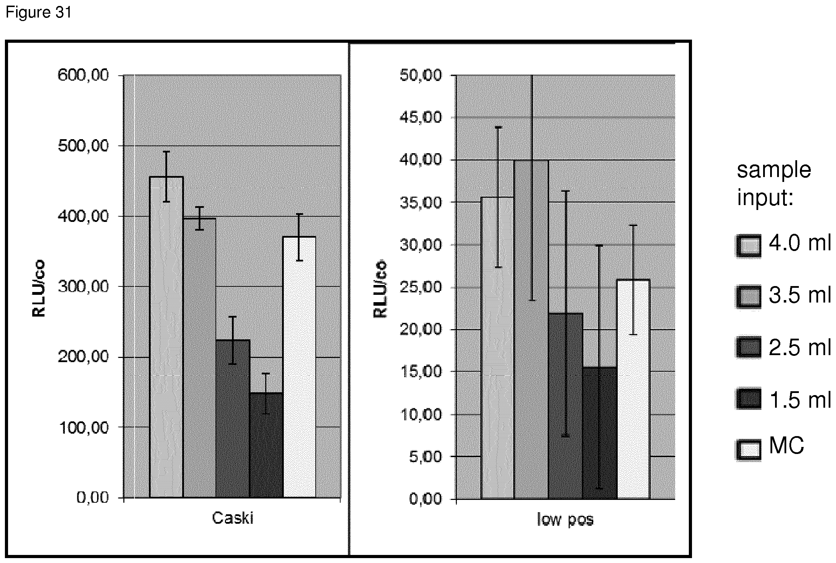

**Figure 31****:** Increased sensitivity of HPV HC assay by increased sample input (PRESERVCYT^{®} samples). The obtained HPV assay results (RLU/CO) are shown for samples from Caski cell culture and from a low positive clinical pool when applying different volumes of sample input. Manual conversion (MC) served as reference.

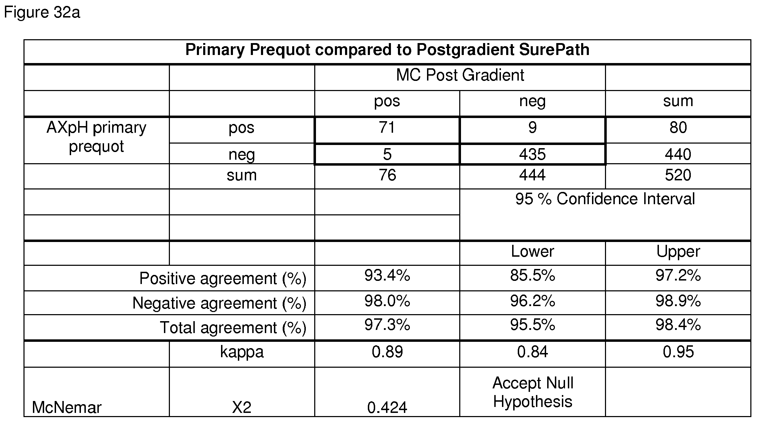

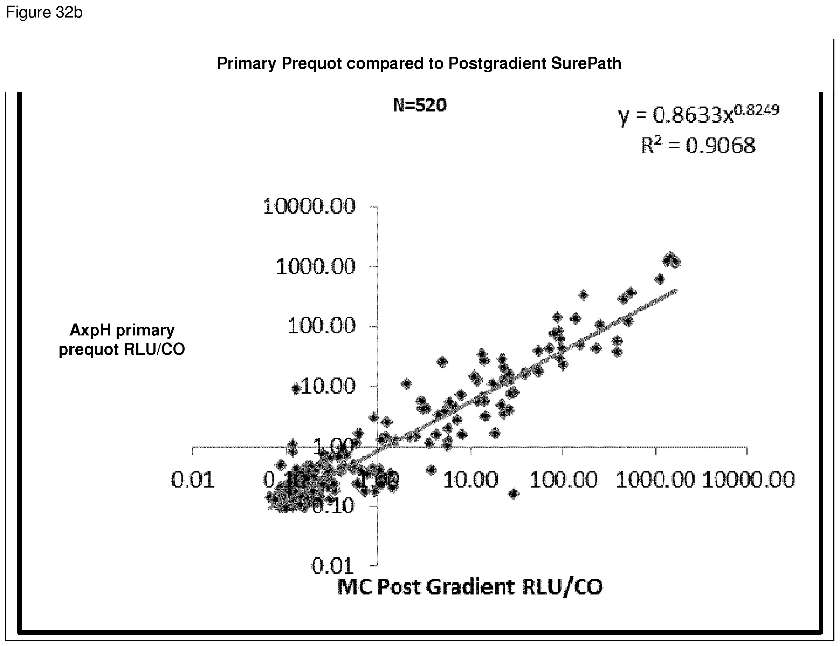

**Figure 32a** **and b:** 2X2 and Scatter plots analysis for manual conversion vs. AXpH - direct method. Prequot SUREPATH® samples were analysed.

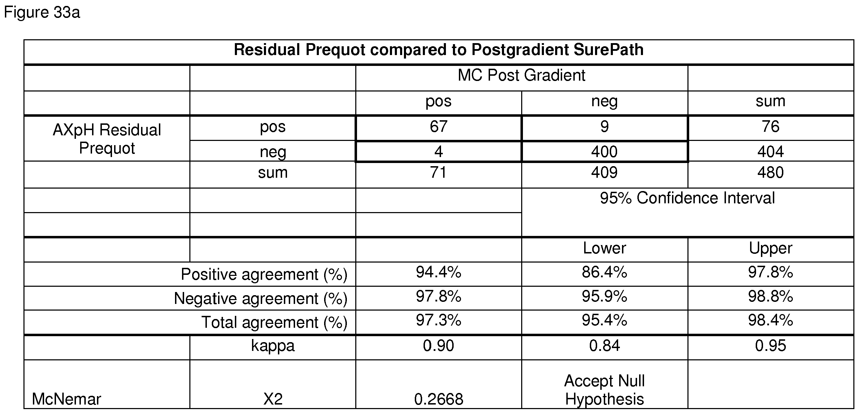

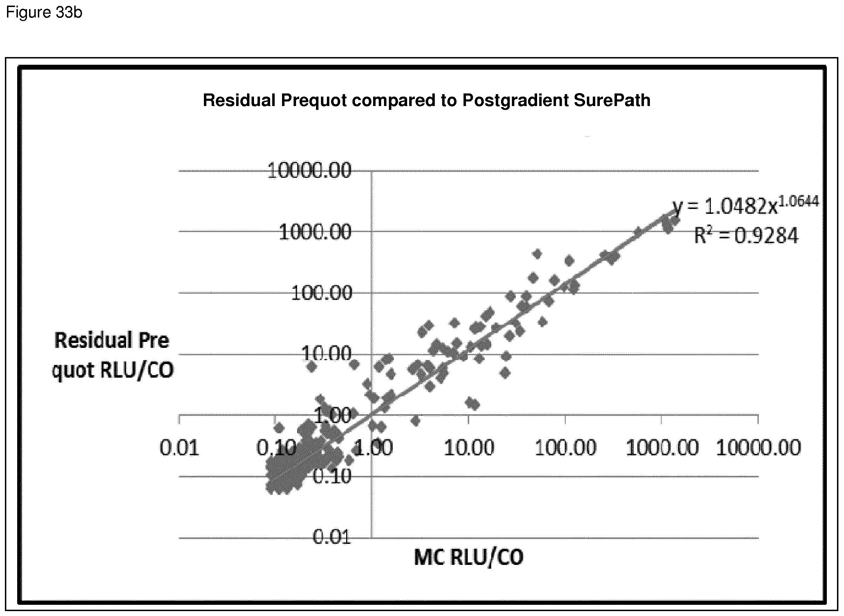

**Figure 33a** **and b:** 2X2 and scatter plots analysis for manual conversion vs. AXpH-direct method. Residual Prequot SUREPATH® samples were analysed.

### DETAILED DESCRIPTION OF THE INVENTION

The present disclosure provides a method that allows the collection of cells using an anion-exchange surface to prepare the cells for the subsequent target nucleic acid detection. The method is useful for, for example, laboratory research and clinical diagnostic purposes, including but not limited to the detection and identification of pathogenic organisms or viruses, the detection of a genetic predisposition to a particular disease and in the field of tumor or other disease diagnostics. In particular, it can be used in the field of virus detection, in particular HPV detection.

According to a first aspect, the present disclosure provides a method of determining the presence or absence of a target nucleic acid in a cell sample, said method comprising:
a) contacting a surface comprising anion exchange moieties with the liquid cell sample under conditions suitable to induce binding between the cells and said surface;
b) separating the surface with the bound cells from the remaining sample to collect the cells;
c) releasing nucleic acids from the cells;
d) generating a hybrid between the target nucleic acid and a probe specific for the target nucleic acid
e) detecting the presence or absence of the hybrid.

The present invention can be defined by the appending claims.

Each individual step, the used materials as well as suitable and preferred embodiments are subsequently described in detail.

### STEP A)

In step a) the cell sample is contacted with a surface comprising anion exchange moieties under conditions suitable to induce binding between the cells and said surface.

### The surface

The surface that is used in step a) is solid or quasi-solid and comprises anion exchange moieties. The term "surface" as used herein in particular refers to the portion of a solid phase which comes into contact with a liquid when the solid phase is contacted therewith. The solid phase provides a surface that provides, e. g. carries, anion exchange moieties. The anion exchange moieties will be described in detail below. Suitable solid phases may be made of or may comprise in particular at their surface a material selected from the following group
- a material comprising or consisting of silicon such as silica and polysilicic acid materials, quartz, borosilicates, silicates, diatomaceous earth or glasses,
- a material comprising or consisting of a polymer such as poly(meth)acrylate, polyurethane, polystyrene, polystyrol, polyacrylamide, a divinylbenzene polymer, a styrene divinylbenzene polymer, polyethylene, polypropylene, polyvinylidene fluoride, polyacrylonitrile, polyvinylchloride, polyacrylate, polyacrylamide, polymethacrylate or a methyl methacrylate polymer;
- a material comprising or consisting of a polysaccharide such as agarose, cellulose, dextrans or sepharose;
- a material comprising or consisting of a mineral;
- a material comprising or consisting of a metal oxide such as aluminum oxide, magnesium oxide, titanium oxide or zirconium oxide;
- a material comprising or consisting of a metal such as gold or platinum; and
- a material comprising or consisting of a derivative of the foregoing.

Furthermore, the solid phase may also comprise more than one of the above described materials. Preferably, at least the surface comprising the anion exchange moieties is composed of one of the described materials or a mixture thereof. Also any solid phase suitable for anion exchange chromatography may be used as solid phase to provide the surface that comprises, e.g. is functionalized with, anion exchange moieties.

Preferred formats of the solid phase include but are not limited to particles such as beads, membranes, filters, plates, columns, vessels and dipsticks. According to one embodiment, the surface comprising anion exchange moieties is provided by a vessel, for example the inner surface of a vessel that is intended to receive the cell sample. The inner surface of the surface or portions thereof can be functionalized, e.g. coated, with anion exchange moieties. Examples of respective vessels that can be functionalized with anion exchange moieties include but are not limited to microtubes and wells of a microplate. In this embodiment, the cells bind due to the provided anion exchange moieties to the inner surface of the vessel, e.g. of a well and are thereby can be easily collected from the sample.

According to a preferred embodiment, the surface is provided by a solid phase that can be provided as suspension and can be separated from a liquid phase. When contacted with a liquid phase such as for example a cell containing liquid based cytology medium as example of a cell sample, the surface comprising the anion exchange moieties is in contact with the liquid phase in order to allow cell binding. Preferably, the surface comprising anion exchange moieties is provided by particles which comprise anion exchange moieties at their surface. The particles may have an average size that is selected from a range of 100 nm to 50 µm, 200 nm to 40 µm, 300 nm to 35 µm, 400 nm to 30 µm, 450 nm to 25 µm, 500 nm to 20 µm, 550 nm to 15 µm, 600 nm to 12.5 µm, 650 nm to 10 µm, 700 nm to 7.5 µm, 750 nm to 5 µm, 800 nm to 3.5 µm, 800nm to 3 µm, 800 to 2,5 µm, 800nm to 2 µm and 800 nm to 1,5 µm. Particles of the respective sizes and in particular of a smaller size such as 10µm or less, 7.5µm or less, preferably 5µm or less, 2.5µm or less or 1.5µm or less are easy to handle and can be well resuspended in the cell sample. Furthermore, respective small particles provide a large surface area that can bind and accordingly can efficiently collect the cells from the remaining sample such as e.g. a liquid-based cytology collection medium. Furthermore, smaller particles can be easily removed during step d) without the need for a magnetic separation step. Suitable materials for providing or making the particles are described above. The particles may also comprise more than one of the above described materials, e.g. comprising two or more layers comprising or consisting of different materials to provide the particle body. Suitable embodiments are also described subsequently.

Preferably, magnetic particles are used to provide an anion exchange surface that binds cells. Using magnetic particles has the advantage, that they can be processed and moved by the aid of a magnetic field. The magnetic particles can for example have superparamagnetic, paramagnetic, ferrimagnetic or ferromagnetic characteristics. Preferably, superparamagnetic particles are used. The magnetic particles may comprise a magnetic material that is incorporated in the particles and/or is associated with the particles. To avoid leaching of the magnetic material, the magnetic material is preferably completely encapsulated e. g. by the material providing the surface such as e.g. silica, polysilicic acid, glass or a polymeric material such as polyacrylate. The magnetic material may provide the core(s) of the particles, may be comprised in the core and/or may be applied onto the core of the particle.

According to one embodiment, the particle comprises a polymer core e.g. made of polystyrol, which is surrounded by at least one polymeric layer, preferably at least two polymeric layers, preferably a polyethylenimine layer and/or a polyacrylate layer. Preferably, the particle surface is made of or comprises a polyacrylate. Said particle surface is then functionalized with anion exchange moieties, preferably polyethylenimine. If magnetic particles are used, a magnetic material such as e.g. iron oxide can be deposited on the polystyrol core. Afterwards, at least one, preferably at least two polymeric layers as described above are applied and the surface of the obtained magnetic particle is then functionalized with anion exchange moieties, preferably polyethylenimine, to provide the final magnetic particle providing an anion exchange surface.

### Anion exchange moieties

The surface that is used in the present invention to bind the cells and thus collect the cells from the cell sample comprises anion exchange moieties. Anion exchange moieties comprise one or more groups capable of anion exchange such as e.g. amine groups. Under appropriate conditions, in particular appropriate pH conditions, anion exchange moieties are capable of binding anions. However, they do not need to be associated with an anion.

According to one embodiment, the anion exchange moieties are provided by the surface material of the solid phase. This embodiment is suitable, if the surface is made of a material that comprises anion exchange groups that are accessible to the cells if the surface is in contact with a liquid. The anion exchange moieties may also form part of a compound or composition which is bound to the surface of the solid phase. Preferably, the surface is functionalized with one or more anion exchange moieties comprising one or more anion exchange groups. The term "moiety" does not include any restrictions with respect to size. The same or different anion exchange groups may be present within one moiety if the moiety comprises more than one anion exchange group. Examples of suitable anion exchange moieties include but are not limited to monoamines, diamines, polyamines, and nitrogen-containing aromatic or aliphatic heterocyclic groups as well as cyclic amines, aromatic amines and heterocyclic amines. Preferably, the anion exchange moiety comprises at least one primary, secondary and/or tertiary amino group. In preferred embodiments, the anion exchange moiety comprises or consists of a primary, secondary or tertiary amine of the formula

R₃N, R₂NH, RNH₂ and/or X-(CH₂)ₙ-Y

wherein

X is R₂N, RNH or NH₂,

Y is R₂N, RNH or NH₂,

R is independently of each other a linear, branched or cyclic alkyl, alkenyl, alkynyl or aryl substituent which may comprise one or more heteroatoms, preferably selected from O,
N, S and P, and
n is an integer in the range of from 0 to 20, preferably 0 to 18.

Hence, the anion exchange moieties comprises an anion exchange group and optionally may have more than one anion exchange group which may be the same or different from each other. An anion exchange group preferably is a chemical group which is neutral or uncharged at a high pH value and is protonated at a low pH value, thereby having a positive charge. At which pH value an anion exchange group becomes positively charged depends on its pKa value. The pKa value of the anion exchange group may lie in the range of from about 7.5 to 14, 8 to about 14, preferably from about 8.5 to about 13.5, 8.75 to 13, more preferably from 9 to 12.5, 9.25 to 12, 9.5 to 11.5 or from about 9.5 to about 11. A pKa value of at least 8, preferably at least 8.5, more preferred at least 9, most preferred at least 9.5 is advantageous, because the anion exchange moieties are positively ionisable and thus positively charged already at low to moderate pH values. This ensures strong cell binding over a broad pH range and thus pH values that are regularly provided by standard cell samples. Therefore, specific adjustments of the binding conditions are in most cases obsolete because the cell sample provides a pH value or pH milieu wherein at least a portion of the anion exchange groups are positively charged, thereby allowing cell binding.

The anion exchange moieties are positively ionisable at appropriate pH values thereby enabling attraction and binding of negatively charged molecules and in particular enabling attraction and binding of cells that are comprised in the cell sample. As is demonstrated by the examples, the anion exchange moieties enable efficient binding of cells, such as in particular cervical cells. Preferably, the anion exchange surface used in the method of the present invention is resistant to degradation by lytic enzymes, proteinases, chemicals and heat up to 85°C, preferably up to 90°C, more preferred up to 95°C. E.g. PEI modified surfaces fulfil the respective requirements. Using respective resistant anion exchange surfaces has the advantage that they are not degraded during step d), if the anion exchange surface is not removed, respectively separated from the remaining sample prior to step d).

The amino groups may bear alkyl, alkenyl, alkynyl and/or aromatic substituents, including cyclic substituents and substituents which together with the nitrogen atom form a heterocyclic or heteroaromatic ring. The substituents preferably comprise 1 to 20 carbon atoms, more preferably 1 to 12, 1 to 8, 1 to 6, 1 to 5, 1 to 4, 1 to 3 or 1 or 2 carbon atoms. They may be linear or branched and may comprise heteroatoms such as oxygen, nitrogen, sulfur, silicon and halogen (e.g. fluorine, chlorine, bromine) atoms and may be substituted as well. Preferably, the substituents comprise not more than 4, more preferably not more than 3, not more than 2 or not more than 1 heteroatom.

In one embodiment the anion exchange moiety carries 1 to 30, 1 to 25, 1 to 20, 1 to 15, 1 to 10, 2 to 8 or 2 to 6 amino groups.

Particular examples of anion exchange moieties include but are not limited to aminomethyl (AM), aminoethyl (AE), aminoalkyl, alkylaminoalkyl, dialkylaminoalkyl such as diethylaminoethyl (DEAE), N,N-diethylaminopropyltrimethoxysilane (DEAPS), ethylendiamine, diethylentriamine, triethylentetraamine, tetraethylenpentaamine, pentaethylenhexaamine, trimethylamino (TMA), triethylaminoethyl (TEAE), linear or branched polyethylenimine (PEI), carboxylated or hydroxyalkylated polyethylenimine, jeffamine, spermine, spermidine, 3-(propylamino)propylamine, polyamidoamine (PAMAM) dendrimers, polyallylamine, polyvinylamine, N-morpholinoethyl, polylysine, and tetraazacycloalkanes. Preferred anion exchange moieties include dialkylamino groups, especially diethylamino groups or linear or branched polyethylenimine (PEI). Most preferred, the surface comprises, respectively is functionalized with polyethylenimine to provide the anion exchange moieties. Linear polyethyleneimines (PEIs) contain secondary amines, in contrast to branched PEIs which contain primary, secondary and tertiary amino groups. According to one embodiment, the anion exchange moiety is no polypeptide or protein.

In one embodiment, the anion exchange moieties comprises an entity selected from the group consisting of primary, secondary and tertiary mono- and poly-amines of the formula
R₁R₂R₃N,
R₁R₂N(CH₂)ₙNR₃R₄,
R₁R₂N(CH2)ₙNR₃(CH2)ₘNR₄R₅,
R₁R₂N(CH2)ₙNR₃(CH2)ₘNR₄(CH2)ₒNR₅R₆
R₁R₂N(CH2)ₙNR₃(CH2)ₘNR₄(CH2)ₒNR₅(CH2)ₚNR₆R₇
R₁R₂N(CH2)ₙNR₃(CH2)mNR₄(CH2)ₒNR₅(CH2)ₚNR₆(CH2)_{q}NR₇R₈
R₁R₂N(CH2)ₙNR₃(CH2)ₘNR₄(CH2)ₒNR₅(CH2)ₚNR₆(CH2)_{q}NR₇(CH2)ᵣNR₈R ₉
R₁R₂N(CH2)ₙNR₃(CH2)ₘNR₄(CH2)ₒNR₅(CH2)ₚNR₆(CH2)_{q}NR₇(CH2)ᵣNR₈(CH2)ₛNR₉R₁₀
wherein
m, n, o, p, q, r and s independently from one each other can be 2 to 8, R₁, R₂, R₃, R₄, R₅, R₆, R₇, R₈, R₉ and R₁₀ can be identical or different and are chosen from the group H, alkyl (branched or unbranched, saturated or unsaturated, preferably comprising 1 to 10 C atoms) and aryl.

In one embodiment, the anion exchange moiety comprises N-propyl-1,3-propandiamine or pentaethylene hexamine. In certain embodiments, the anion exchange moieties are selected from spermine and spermidine. As described above, it is preferred though to functionalize the surface, preferably the surface of particles, more preferred magnetic particles, with polyethylenimine.

According to the present invention the surface may comprise and can accordingly be functionalized with more than one type of anion exchange moieties. Thus, also two or more different anion exchange moieties can be present on the surface. Accordingly, it is within the scope of the present invention that the surface comprises a mixture of different anion exchange moieties. However, it is also within the scope of the present invention to predominantly or exclusively use one type of anion exchange moiety.

For functionalizing a surface with anion exchange moieties, several methods are feasible. The anion exchange moieties may be bound directly to the surface, either covalently or non-covalently, electrostatically and/or may form part of a polymer or other composition which forms a surface coating or which is provided at the surface of the solid phase. The anion exchange moieties may also be precipitated on the solid phase. According to one embodiment, the anion exchange moieties are provided in form on a coating on the solid phase.

According to one embodiment, a covalent coupling strategy is used. According to one embodiment, the solid phase comprises at its surface functional groups that are suitable for covalent attachment of the anion exchange moieties. For example, the surface may comprise functionalities selected from the group consisting of Si-O-Si, Si-OH, alcohol, diol or polyol, carboxyl groups, amine, phosphate or phosphonate. The anion exchange moieties may be attached to the solid phase, for example, by using epoxides, carboxyl groups, in particular activated carboxyl groups, silanes, acid anhydrides, acid chlorides, formyl groups, tresyl groups, pentafluorophenyl groups sulfonyl chloride or maleinimide groups. The functional groups that are used for coupling of the anion exchange moieties may be attached directly to the solid phase or via (linear or branched) spacer groups, e.g. hydrocarbons such as -(CH₂)ₙ-groups, carbohydrates, polyethylenglycols and polypropylenglycols. Alternatively, also a polymer composed of monomers comprising the anion exchange groups such as an amino functional group can be used to provide anion exchange moieties at the surface of the solid phase. In certain embodiments, the solid phase material has a silicon containing surface such as a polysilicic acid surface and the anion exchange moieties are coupled to said surface using a silane group. According to a preferred embodiment, the surface of the solid phase comprises carboxyl groups which can be used for covalent coupling of the anion exchange moieties such as PEI. The respective carboxyl groups are preferably provided by a polyacrylate layer.

The anion exchange moieties can be attached to the surface of the solid phase via a linker group. The linker group preferably is a linear, branched or cyclic alkylen, alkenylen or alkynylen group which preferably comprises 1 to 20 carbon atoms, more preferably 1 to 12, 1 to 8, 1 to 6, 1 to 5, 1 to 4, 1 to 3 or 1 or 2 carbon atoms. It may further comprise heteroatoms such as oxygen, nitrogen, sulfur, silicon and halogen (e.g. fluorine, chlorine, bromine) atoms, preferably not more than 4, more preferably not more than 3, not more than 2 or not more than 1 heteroatom. In preferred embodiments, the linker group is an alkylene group, in particular a propylene group.

According to one embodiment, for producing the anion exchange surface, the surface is activated to promote or enable coupling of the anion exchange moieties. Preferably, a chemical activation is performed. Chemical activation includes but is not limited to the use of oxidation reagents. According to one embodiment which is preferred if COOH groups are used for coupling, a carbodiimide such as EDC (1-ethyl-3-(3-dimethylaminopropyl) carbodiimide) is used to activate the surface, in particular a surface carrying COOH groups. EDS is a suitable carboxyl activating agent for the coupling of amines to yield amide bonds. However, EDC can also be used to activate phosphate groups. EDC can be used in combination with N-hydroxysuccinimide (NHS) or sulfo-NHS to increase the coupling efficiency and/or create a stable amine-reactive product.

### Additional functionalization of the surface

In certain embodiments, the surface comprises further functional groups in addition to the anion exchange moieties. Non-limiting examples are described below. Said functional groups can be provided by the surface material itself, e.g. acidic groups such as carboxyl groups (e.g. if the surface is made of a polyacrylate) or respective functional groups can be provided by ligands that are attached covalently or non-covalently to the surface. The functional groups may be applied to the surface as is described herein for the anion exchange moieties.

According to one embodiment, the pKa value of said functional groups lies in a range of 0 to 7, preferably 1 to 5. Suitable examples include ion exchangers, in particular cation exchangers, preferably acidic groups such as carboxyl groups. Further suitable groups include betains, sulfonate, phosphonate and phosphate groups. As described herein, the solid phase may comprise carboxyl groups in order to allow the attachment of the anion exchange moieties. When attaching the anion exchange moieties, the concentration of the anion exchange moieties can be chosen such that at least a portion of the carboxyl groups is not bound to anion exchange moieties and accordingly, wherein the carboxyl groups are accessible at the surface.

According to one embodiment, the surface comprises inert ligands. In particular, the inert ligands are neutral or uncharged at a pH range of 2 to 14, 3 to 13, 4 to 12 and preferably are hydrophilic. The inert ligands may be attached to the surface as is described herein for the anion exchange moieties. Preferably, the inert ligands are organic moieties, in particular alkyl, alkenyl, alkynyl or aromatic moieties which may be linear, branched or cyclic and which preferably comprise at least one heteroatom such as oxygen, nitrogen, sulfur, silicon and halogen (e.g. fluorine, chlorine, bromine) atoms. The inert ligand preferably comprises 1 to 20 carbon atoms, more preferably 1 to 12, 1 to 8, 1 to 6, 1 to 5, 1 to 4, 1 to 3 or 1 or 2 carbon atoms, and up to 4, more preferably up to 3, up to 2, or up to 1 heteroatom. Particularly preferred are inert ligands comprising at least one hydroxyl group, in particular at least 2 hydroxyl groups, such as ligands comprising a 2,3-dihydroxypropyl group. A specific example of the inert ligands is a (2,3-dihydroxypropyl)oxypropyl group. The inert ligands can be used to reduce the amount of anion exchange moieties that can attach to the solid phase during coupling of the anion exchange moieties. Hence, the density of the anion exchange moieties on the surface of the solid phase can be controlled and adjusted to the subsequent application of the solid phase. In particular, the solid phase may comprise anion exchange moieties and inert ligands in a ratio in the range of from about 1:1 to about 1:10, preferably from about 1:2 to about 1:5, more preferably about 1:3.

In another embodiment, the solid phase does not comprise such inert ligands. According to one embodiment, the solid phase only comprises the anion exchange moieties and does not comprise other ligands. According to one embodiment, the solid phase comprises a silicon containing surface, preferably a polysilicic acid surface and is derivatized with a silane compound comprising the anion exchange groups and is not derivatized with another silane compound.

Examples of suitable solid phases, anion exchange groups, functional groups and inert ligands (comprising a neutral group N) are described in WO 2010/072834, WO 2010/072821, DE10 2008 063 001A1 and DE 10 2008 063 003.

### Preferred embodiment of an anion exchange surface

According to a particularly preferred embodiment, the anion exchange surface is a surface, preferably a carboxylated surface, comprising anion exchange moieties, preferably comprising amine groups. Preferably, polyethyleneimine (PEI) is used as anion exchange moiety to bind the cells from a liquid sample. Preferably, said anion exchange surface is provided in form of particles, more preferred in the form of magnetic particles. This preferred embodiment of an anion exchange surface is now explained in further detail.

Polyethyleneimine (PEI) is a polymer whose molecules are built from repeating units of ethyleneimine (also known as aziridine) whose three-membered rings open during polymerization.

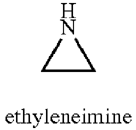

An exemplary PEI molecule has formula as follows

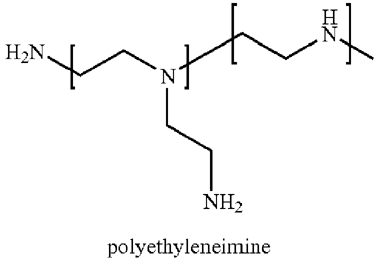

PEI is thus typically a branched molecule built up of units of ethyleneimine that bond through a nitrogen atom. In certain embodiments, the form of PEI used is a product purchased from the Sigma-Aldrich Chemical Company ("Sigma-Aldrich"), product number 408719 (100 ml). This molecule is ethylenediamine end-capped polyethyleneimine, having (according to Sigma-Aldrich) an average molecular weight of ∼800 when measured by light scattering (LS), and an average molecular weight of ∼600 when measured by gel permeation chromatography (GPC). Ethyleneimine (CH2CH2NH) has a molecular weight of 43 and, assuming the average molecular weight of the PEI molecule to be 800 as reported by Sigma-Aldrich, this molecule has approximately 18 units of CH2CH2NH, some of which may be branched. In essence it has approximately 18 primary (-NH2), secondary (-NHR) or tertiary (-NR2) amine groups present (wherein R is a carbon-containing group bonding to the amine nitrogen through a carbon atom).

The form of PEI suitable for use herein is of course not limited to that product available from Sigma-Aldrich. PEI, being polymeric in nature, admits of a wide range of forms, controlled at least in part by the extent of polymerization permitted during its synthesis. Thus, many variants of PEI, having variously, different numbers of repeating units, and different amounts of branching, are suitable for use herein. For example, one having from 10-30 units of CH2CH2NH is suitable, as is one having from 12-24 units, as are those having from 16-20 units. In general, there is a range of lengths that is suitable for cell capture. Additionally, different end-caps from ethylene diamine may be used to make a variant of PEI suitable for use herein. Such end caps may include, without limitation, 1,2-propylene diamine, 1,3-propylene diamine, 1,2-butylene diamine, 1,3-butylene diamine, and 1,4-butylenediamine.

Molecules of PEI suitable for use as anion exchange moiety in the present invention may be characterized by molecular weight. In particular, suitable PEI molecules have weights in the range 600-800 Da. When measured by LS, suitable PEI molecules have measured weights in the range 700-900 Da, and when measured by GPC, suitable PEI molecules have measured weights in the range 500-700 Da.

To provide a surface comprising PEI as anion exchange moiety, PEI is typically immobilized on, such as bound to the surface of, a solid support such as particles, preferably magnetic particles. The particles are typically formed of a material to which the PEI can be easily associated. Exemplary materials from which such particles can be formed include polymeric materials that can be modified to attach a ligand. Typically, such a solid support itself may be derivatized to yield surface functional groups that react easily with PEI molecules to create a chemical bond between the surface and the PEI. A frequently-employed- and desirable-surface functional group is the carboxyl (-COOH) group. Exemplary polymeric materials that provide, or can be modified to provide, carboxyl groups and/or amino groups available to attach PEI include, for example, polystyrene, latex polymers (e.g., polycarboxylate coated latex), polyacrylamide, polyethylene oxide, polyacrylate, and derivatives thereof. Polymeric materials that can used to form suitable particles are described in U.S. Pat. No. 6,235,313 to Mathiowitz et al.. Other materials include glass, silica, agarose, amino-propyl-tri-ethoxy-silane (APES) modified materials and the materials described above.

During the process of reaction of a PEI molecule with a carboxylated particle, such as a magnetic particle, one of the amine groups out of the total possible amine groups on a PEI molecule, such as 18 possible groups in the aforementioned product from Sigma Aldrich, is consumed to react with the COOH group of the surface of the particle to form a carbodiimide bond. The remainder of the total number amine groups, such as 17 groups in the aforementioned product from Sigma Aldrich, are available for protonation and accordingly can provide the anion exchange functionality. In some embodiments, a synthesis protocol comprises: washing a quantity of microspheres with carbonate and MES buffer; preparing sulfo-NHS and EDAC; incubating the microspheres with sulfo-NHS and EDAC for 30 minutes; washing the microspheres with MES and borate buffer; contacting the microspheres with PEI for 8-10 hours; and rinsing unbound PEI from the microspheres. Suitable methods for preparing PEI functionalized particles as are preferably used in the method according to the present invention are described in US 2010/0009351.

There are a variety of sources of particles that can be used to bind PEI, and used in the processes described herein, for example: Seradyn Magnetic carboxyl modified magnetic beads (Part #3008050250, Seradyn), Polysciences BioMag carboxyl beads, Dynal polymer encapsulated magnetic beads with a carboxyl coating, and Polybead carboxylate modified microspheres available from Polyscience, catalog no. 09850.

The high density of the PEI molecules on the particle surface permits even a small quantity of particles to be used to efficiently capture the cells from the sample.

As described above, the particles are preferably magnetic. Magnetic particles are advantageous because centrifugation is generally not required to separate them from a solution in which they are suspended. Magnetic particles can be moved by applying a magnetic field and thus, are suitable for automation. Suitable and preferred particle sizes were described above and also apply to this preferred embodiment, wherein PEI groups are used as anion exchange moiety.

Because a nitrogen atom is trivalent and due to the conditions of polymerization, each molecule of PEI can have a mixture of primary, secondary, and tertiary amine groups. Therefore, polyethylene molecules exhibit multiple pKa's over a range of values roughly consonant with the range of pKa's spanned by primary, secondary, and tertiary aliphatic amines. Accordingly, PEI typically has a pKa in the range greater than 9.0. Thus, at low pH PEI is typically positively charged-and may even carry multiple positive charges per molecule arising from protonations of the amine groups at pH's lower than its pKa -and is therefore able to bind strongly to cells in solution. Thus, the use of PEI functionalized particles is particular efficient to concentrate and thus harvest cells from a cell containing sample.

PEI molecules as are preferably used as anion exchange moiety in the present invention are furthermore advantageous because they are resistant to, e.g., are immune to, degradation by lytic enzymes, harsh chemicals such as detergents, and heat up to 95° C. This is a particular advantage as it allows that the anion exchange surface comprising PEI as anion exchange moiety, preferably the PEI functionalized magnetic particles described herein, may remain present in the sample/processing mixture(s) even after the cells were lysed and may remain present during nucleic acid denaturation and generation and capture of the hybrid. Thus, as is shown in the examples, it is not necessary to separate magnetic particles carrying PEI as anion exchange moiety that were used for collecting the cells prior to performing the lysis and denaturing and prior to performing the hybrid capturing assay. This is an important advantage as it saves handling steps and makes the method very convenient for the user. That this embodiment is suitable and functions well as is shown by the examples was highly surprising as PEI can itself function as an inhibitor of enzymatic processes or can capture released nucleic acids. Therefore, it was not expected that an anion exchange surface which comprises PEI groups as anion exchange moieties can remain in the sample/processing mixture during nucleic acid release, denaturation and generation and capture of the hybrid as, however, is shown herein is possible.

### Particle suspension

The particles carrying anion exchange moieties as described herein can be provided in form of a suspension. Preferably, they are comprised in an aqueous solution. In a simple embodiment, the particles are provided in water. According to one embodiment, the suspension comprising the particles has a pH value of 13 or less, 12 or less, 11 or less, 10 or less, 9 or less, 8.5 or less, 8 or less, 7.5 or less, 7 or less and preferably has a pH value below 6.5. Preferred pH values can be selected from the range of 3.5 to 10, 3.75 to 9, 4 to 8, 4.25 to 7, 4.5 to 6.5, 5 to 6.25 and 5.5 to 6.25. According to one embodiment, said solution comprises one or more additives selected from the group consisting of buffers, in particular biological buffers as described herein (e.g. TRIS, MES or MOPS), detergents, preferably non-ionic detergents (e.g. in a concentration of 2.5% or less, more preferred 1% or less, 0.5% or less, 0.25% or less or 0,175% or less) and preservatives such as azides (e.g. in a concentration of 0.5% or less, 0.25% or less, 0.2%or less, 0.1% or less, 0.05% or less). The design of the solution influences the sedimentation characteristics of the particles. A solution comprising all of the aforementioned additives has good sedimentation characteristics and is in particular suitable for particles comprising amine groups, in particular magnetic particles that are functionalized with polyethylenimines. The solution that is used for providing the particle suspension is preferably storage stable and thus allows storing the particle suspension for at least 3 months, at least 6 months and preferably for at least 12 months. These characteristics are fulfilled by the above described solution that can be used for providing the particle suspension. Suitable buffers for providing a particle suspension are also described in US 2010/0009351.

According to one embodiment, the particle suspension comprises the particles which provide the anion exchange surface which preferably are magnetic particles as described above in a concentration selected from 1mg/ml to 100mg/ml, 1mg/ml to 75mg/ml, 2mg/ml to 60mg/ml, 2mg/ml to 50mg/ml, 2.5mg/ml to 40mg/ml, 2.5mg/ml to 35mg/ml, 3mg/ml to 30mg/ml, 4mg/ml to 27.5mg/ml, 5mg/ml to 25mg/ml, 7.5mg/ml to 22.5mg/ml, 10mg/ml to 20mg/ml and 12.5mg/ml to 17.5mg/ml. Good results are achieved in a concentration of 10 to 50mg/ml, preferably 10mg/ml to 20mg/ml. Most preferred, the particles are comprised in the suspension in a concentration of approx. 15mg/ml. Suitable and preferred particle sizes and examples of magnetic particles are described above.

### The cell sample

The cell sample comprises cells. The cells may be prokaryotic or eukaryotic. Preferably, the cells are eukaryotic cells, more preferably the cells are mammalian cells. According to a preferred embodiment, the cells are human cells. The cells can be derived for example from a biological, in particular a clinical sample. The cell sample can be collected from a human, for example a patient, using a cell collection device such as a swab, cervical broom, cervical brush, spatula, floqswab or similar collection devices or by collecting a body fluid, such as blood, urine, liquor, semen and the like. Accordingly, basically any sample may be used in which cells may be present, including, without limitation: cell suspensions, a cell containing specimen or culture (e.g., cellular and tissue cultures);clinical and laboratory biological samples; food and agricultural samples; and forensic samples, including semen, hair, blood, skin, and saliva samples; cell containing samples obtained from a mammalian, preferably human, in particular body samples such as body fluids and tissues, in particular whole blood, bone marrow aspirates, lymphatic fluid, urine, amniotic fluid, semen/seminal fluid, bronchoalveolar lavages, body secretions, nasal secretions, vaginal secretions, wound secretions or cell-containing samples obtained from the aforementioned body fluids and tissues. Samples may be from any mammal, including a human, and explicitly include fluid, solid (e.g., stool) or tissue, as well as liquid and solid food and feed products and ingredients such as dairy items, meat and meat by-products, and waste. Explicitly included are samples taken directly from a mammal, as well as samples that have been stored in a preservative, including but not limited to paraffin-embedded tissue samples and cellular and tissue or cell samples stored in a liquid-based cytology medium. The cells are comprised in a liquid which can at least partially originate from the biological sample itself (e.g. in the case of blood or a cell culture) or can be partially or fully provided by a collection medium that is used to receive the cells and/or that is used to receive the cell-containing biological sample. E.g. a cell sample that was obtained by using a swab or other cell collection device (such as spatula or brush) can be contacted with a collection medium, for example a liquid-based cytology medium. This is in particular preferred when processing epithelial cells, such as cervix cells. According to one embodiment, the cells are epithelial cells. Respective cells can be collected for example from a cell culture or can be obtained from a patient by using an appropriate collection device. According to one embodiment, the cells are cervix cells. Respective cells can be collected from women e.g. by using a swab or other suitable collection device (such as spatula or brush). The collected cells are then usually contacted with a liquid-based cytology medium to preserve the cells during shipping and/or storage. Cells comprised in or cell samples contacted with a respective collection and/or storage medium and in particular cells comprised in a liquid-based cytology medium are also encompassed by the term "cell sample" as used herein.

In an embodiment, the cell sample comprises at least one fixative agent. In an embodiment, the sample comprises a cross-linking and/or a non-cross-linking fixative agent. Cross-linking fixatives function by making chemical bonds between proteins in the sample, leading to their precipitation and immobilization within the sample. Exemplary crosslinking fixatives include, but are not limited to, formaldehyde and paraformaldehyde. Non-cross-linking fixatives do not chemically alter the proteins in the sample; rather they simply precipitate them where they are found in the sample. Non-cross-linking fixatives include ethanol, acetone, methanol and mixtures thereof.

In a preferred embodiment, the cells are comprised in a liquid-based cytology medium. Liquid-based cytology media are well known and are widely used in the prior art and thus, need no detailed description here. Exemplary liquid-based cytology media include, but are not limited to PRESERVCYT® (Hologic, Inc., Bedford, MA) (a methanol-based liquid-based cytology medium); SUREPATH® (Becton, Dickinson and Company, Franklin Lakes, NJ) (a liquid-based cytology medium comprising alcohol and aldehyde), M4 (Remel), M4RT (Remel), Copan UTM (Universal Transport Medium) and Eagle Minimum Essential Medium (EMEM). The used liquid-based cytology medium preserves the cells and accordingly does not or does not substantially induce cell lysis. As is shown by the examples, a primary sample material collected in the liquid-based cytology medium can be analysed using the method of the invention. If desired, a portion of the cells can be removed from the primary sample material in advance for other purposes. These cells can be used e.g. for other analytical purposes such as e.g. cytology tests. According to one embodiment, cells comprised in the sample material are suspended prior to processing, e.g. by vortexing. This is e.g. feasible if only an aliquot of the primary sample material is processed. According to one embodiment, the cell sample is a SUREPATH® pre-quot sample. In SUREPATH® pre-quot samples, the primary material collected in SUREPATH® medium is processed. E.g. a cell containing portion of this primary material can be processed using the method of the invention. Processing the primary material has advantages as it avoids sample preparation steps, thereby saving handling steps. According to one embodiment, the cell sample is a SUREPATH® post-quot sample. SUREPATH® post-quot samples are obtained from the primary SUREPATH® sample by a sample preparation step to allow e.g. the parallel analysis of the collected cells by cytology, e.g. a PAP test. E.g. the collected cells such as cervix cells are obtained from a density gradient and suspended again in SUREPATH® medium. This enriched cell suspension is referred to in the art as "post-quot" or "post-gradient" sample. As is shown by the examples, both types of SUREPATH® samples can be processed with the method of the invention. Furthermore, residual pre-quot samples can be processed as is shown by the examples.

### Contacting conditions

In step a), the cell sample is contacted with a surface comprising anion exchange moieties under conditions that are suitable to induce binding between the cells and said surface. Binding is mediated by a direct interaction between the cells and the surface, in particular between the cells and the anion exchange moieties. Thus, cell binding to the surface is not mediated by specific binding partners such as antibodies or similar binding molecules. The cells are brought into contact with the anion exchange surface over a sufficiently long period of time, i.e. a period of time which suffices to allow the cells to bind/attach themselves to the anion exchange surface. Such a period of time should be at least 10 seconds, preferably at least 15 seconds, more preferred at least 20 seconds, and preferably is selected from at least 30 seconds, at least one minute, at least 1.5minutes, at least 2 minutes, at least 2.5 minutes, at least 3 minutes, at least 4 minutes, at least 5 minutes or at least 6 minutes. Suitable incubation times can also be determined by the skilled person. As is shown by the examples, already short incubation periods are suitable as the cells bind rapidly to the anion exchange surfaces described herein.

According to one embodiment, an aliquot of the above described particle suspension is contacted with the cell sample wherein the cells are comprised in a liquid, preferably a liquid-based cytology medium as described above. According to one embodiment, the cell sample including the liquid has a volume that is selected from 0.05ml to 10ml, 0.1ml to 7.5ml, 0.2ml to 6ml, 0.25ml to 5ml, 0.3ml to 4ml, 0.35ml to 3.5ml, 0.4ml to 3ml and 0.5 to 2.75ml. It is a particular advantage of the method according to the present invention that small as well as large sample volumes can be processed. Furthermore, it is possible to capture cells from diluted samples. The option to increase the sample volume has the important advantage that the overall sensitivity of the method can be increased. Furthermore, as regularly no chemistry is needed to adjust the cell binding conditions and in particular to adjust the pH value for binding the cells, the whole process volume of a robotic system basically can be used for the sample volume. The chosen volume of the sample is contacted with an aliquot of the particle suspension. Suitable aliquot sizes for the particle suspension can be e.g. selected from 5µl to 150µl, 7.5µl to 125µl, 10µl to 100µl, 15µl to 85µl, 20µl to 75µl, 25µl to 65µl, 30µl to 55µl and 35µl to 50µl. Preferably, the particle suspension comprises the particles, which preferably are magnetic particles, in a concentration selected from a range described above for the particle suspension. Larger aliquot volumes of at least 15µl, at least 20µl, at least 25µl and preferably at least 30µl can be processed more easily by robotic systems and thus are preferred. The particle suspension and the sample can be contacted in any order. E.g. the particle suspension respectively an aliquot thereof providing the desired amount of particles can be provided in a vessel and the sample is added to the particle suspension or vice versa. The resulting mixture comprising the sample and the particle suspension preferably comprises the particles in a concentration selected from 50µg/ml to 2000 µg/ml, 75µg/ml to 1750 µg/ml, 100 µg/ml to 1500 µg/ml, 125 µg/ml to 1250 µg/ml, 140 µg/ml to 1200 µg/ml, 150 µg/ml to 1150 µg/ml and 165 µg/ml to 1100 µg/ml. These concentrations are preferred when particles, preferably magnetic particles, having an average size of 5µm or less, preferably 3µm or less and preferably having a size that lies in the range of 500 nm to 2 µm, preferably 800 nm to 1,5 µm are used. Most preferred, the average size of the magnetic particles is about 1 µm. As described above, preferably, magnetic particles functionalized with polyethylenimine are used.

As described above, in a preferred embodiment, the cell sample comprises epithelial cells such as cervix cells that are collected in a liquid-based cytology medium. If processing a cell sample wherein the liquid-based cytology medium comprises a cross-linking fixative agent (e.g. a SUREPATH® sample) it is preferred that the mixture comprising the cell sample and the particle suspension comprises the particles which preferably are magnetic particles as described above, in a concentration selected from 300 µg/ml to 2000 µg/ml, 400 µg/ml to 1500 µg/ml and 500 µg/ml to 1250 µg/ml. E.g. a sample (e.g. a SUREPATH® sample) having a volume of 0.5ml to 1ml can be contacted with 35µl to 50µl of the above described particle suspension which preferably comprises the particles in a concentration of 10 to 50mg/ml, preferably 10 to 20mg/ml, in particular approx. 15mg/ml. Respective particle concentrations are also suitable if a liquid-based cytology medium is used which comprises the cells in a higher concentration. If processing a respective cell sample wherein the liquid-based cytology medium does not comprise a cross-linking fixative agent (e.g. a PRESERVCYT® sample) it is preferred that the mixture comprising the sample and the particle suspension comprises the particles which preferably are magnetic particles as described above, in a concentration selected from 100 µg/ml to 750 µg/ml, 125 µg/ml to 600 µg/ml, 150 µg/ml to 500 µg/ml and 165µg/ml to 400µg/ml. E.g. a cell sample (e.g. a PRESERVCYT® sample) having a volume of 2ml to 3ml can be contacted with 35µl to 50µl of the above described particle suspension which preferably comprises the particles in a concentration of 10 to 20mg/ml, in particular 15mg/ml. Respective concentrations are also suitable if a liquid-based cytology medium is used which comprises the cells in lower concentrations. As described above, preferably, magnetic particles functionalized with polyethylenimine are used. Said particles may also comprise carboxyl groups at their surface. Preferably, the magnetic particles have an average size of 5µm or less, preferably 3µm or less and preferably have an average size that lies in the range of 750 nm to 2 µm, preferably 800 nm to 1,5 µm. Most preferred, the average size of the magnetic particles is about 1 µm.

As is shown by the examples, using an anionic exchange surface according to the teachings described herein allows binding the cells under various conditions. This is a particular advantage as it contributes to the fact that the method according to the present invention is robust and insensitive to errors. As is shown by the examples, many contaminants that can be comprised in cell samples and in particular in cervix cell samples do not significantly disturb the performance of the cell binding. This is an important advantage in the field of diagnostics, in particular HPV diagnostics, wherein the cell samples such as cervix samples can comprise contaminants.

Furthermore, as is shown by the examples, binding of the cells to the anion exchange surface can occur at various pH values. The suitable pH range also depends on the pKa value of the anion exchange groups of the anion exchange moieties, as the anion exchange groups should be positively charged to ensure efficient cell binding. Therefore, according to one embodiment, binding occurs at a pH value wherein anion exchange groups of the anion exchange moieties are positively charged. According to one embodiment, binding of the cells occurs in step a) at a pH value that is selected from a pH value of 9.5 or less, a pH value of 9 or less, a pH value of 8.75 or less, a pH value of 8.5 or less, a pH value of 8.25 or less, a pH value of 8 or less, a pH value of 7.75 or less, a pH value of 7.5 or less, a pH value of 7.25 or less, a pH value of 7 or less, a pH value of 6.75 or less, a pH value of 6.5 or less and a pH value of 6 or less. Preferably, cell binding occurs, depending on the cell sample, at a pH value selected from a pH range of 3 to 8.5, 3.5 to 8.0, 3.5 to 7.75, 3.5 to 7.5, 3.5 to 7.25, 3.75 to 7 or 4 to 6.5. As is shown by the examples it is preferred that binding occurs at a pH value of 8.5 or less, preferably 8 or less and more preferred 7.5 or less and in particular at a neutral or acidic pH value of 7 or less or 6.5 or less as here, the binding efficiency of the cells to the anion exchange surface is improved and binding is very rapid. The described pH values and ranges are particularly preferred when functionalizing the surface with amines such as polyethylenimine. It is a particular advantage of the method according to the present invention that the binding of the cells to the anion exchange surface can occur under a variety of conditions and in particular at a variety of pH values. Thereby, the method according to the present invention can be applied to a high number of cell samples without the need for specific adaptions, in particular pH adjustments, of the liquid that comprises the cells. For example, epithelial cells such as cervix cells are usually collected/preserved in a collection/preservation medium. Said media may have, depending on the collection/preservation medium used, a pH value from moderately or slightly basic to acidic. Thus, pH values as described above are usually provided or are present if the cells are comprised in a collection medium such as a liquid-based cytology medium. For example, the standard collection medium SUREPATH has a pH value of approximately 7, whereas the standard collection medium PRESERVCYT® has a pH value of 4 to 5. As is shown by the examples, the method according to the present invention can be used in conjunction with both collection media even though they significantly differ in their pH value. When contacting cells comprised in a respective collection medium with an anion exchange surface as described herein, the cells bind directly and with quick kinetics to said surface. Accordingly, when processing such samples no specific adaptions of the binding conditions are necessary when using the technology according to the present invention. It is not necessary to adjust the binding conditions by adding a binding buffer to the cell sample or by adjusting the pH value to achieve a specific pH range that allows binding the cell to the anion exchange surface. This is an important advantage, as it saves handling steps, saves costs and makes the method suitable for many different cell sample types and reduces the risk that handling errors falsify the results. Thus, according to one embodiment, no adaption of the binding conditions and in particular no adaption of the pH value occurs and the anion exchange surface, preferably the magnetic particles comprising anion exchange moieties, is simply contacted with the cell sample and is then incubated to allow cell binding.

However, as is shown by the examples, binding can also occur in the presence of additives such as e.g. alcohols. Accordingly, if desired, respective additives such as a branched or unbranched alcohol having 1 to 5 carbon atoms, e.g. isopropanol, or mixtures of respective alcohols can be added during the binding step. However, it is not necessary that respective additives are added and it is shown by the examples that the binding of the cells to the anion exchange surface is not necessarily improved thereby. Thus, for the easy of simplicity it is preferred that no alcohol or other additives are added to the cell sample to support the binding of the cells to the anion exchange surface. As is described above and demonstrated by the examples, it is within the scope of the present invention and preferred to provide the anion exchange surface, in particular magnetic particles comprising anion exchange moieties , in form of a suspension. The liquid that is used for providing the particle suspension is accordingly present during the binding step. This liquid can be used to make certain pH adjustments, if e.g. rather alkaline cell samples are processed. By using an acidic suspension medium for suspending the anion exchange particles, the pH value of the binding mixture comprising the particles and the cell sample can be lowered, without, however, increasing the handling steps and without the need to add separate binding agents.

After step a) the cells are bound to the surface comprising anion exchange groups.

### STEP B)

In step b), the surface to which the cells are bound is separated from the remaining sample, thereby collecting the cells. Here, different modes of operations are feasible in order to separate the surface with the bound cells from the remaining sample which also depend on the used solid phase. Non-limiting examples are described subsequently.

For example, if a vessel is used, wherein the inner wall of the vessel is at least partially functionalized with anion exchange moieties as described herein, the remaining sample can be discarded by decanting or it can be removed by aspiration or similar methods. The bound cells remain associated to the inner surface of the vessel due to the anion exchange moieties. Respective separation steps for separating the remaining sample can be performed using a robotic system.

According to a preferred embodiment, particles comprising anion exchange moieties and in particular particles functionalized with polyethylenimines at their surface are used to provide the anion exchange surface. If the particles are non-magnetic they can be collected for example by filtration or sedimentation which can according to one embodiment be assisted by centrifugation. It is preferred though to use magnetic particles, because magnetic particles including the bound cells can be processed easily by the aid of a magnetic field, e.g. by using a permanent magnet. This embodiment is preferred as it is compatible with established robotic systems capable of processing magnetic particles. Here, different robotic systems exist in the prior art that can be used in conjunction with the present invention to process the magnetic particles to which the cells were bound. According to one embodiment, the magnetic particles are collected at the bottom or the side of the reaction vessel and the remaining liquid sample is removed from the reaction vessel, leaving behind the collected magnetic particles to which the cells are bound. Removal of the remaining sample can occur by decantation or aspiration. Such systems are well known in the prior art and thus need no detailed description here.

In an alternative system that is known for processing magnetic particles a magnet which is usually covered by a cover or envelope plunges into the reaction vessel to collect the magnetic particles. The magnetic particles that carry the bound cells can then be transferred for example into a new reaction vessel e.g. comprising a resuspension solution, preferably a denaturing composition as will be described in the following. As respective systems are well-known in the prior art and are also commercially available (e.g. QIASYMPHONY®; QIAGEN), they do not need any detailed description here.

In a further alternative system that is known for processing magnetic particles, the sample comprising the magnetic particles can be aspirated into a pipette tip and the magnetic particles can be collected in the pipette tip by applying a magnet e.g. to the side of the pipette tip. The remaining sample can then be released from the pipette tip while the collected magnet particles which carry the bound cells remain due to the magnet in the pipette tip. The collected magnetic particles can then be processed further. Such systems are also well-known in the prior art and are also commercially available (e.g. BioRobot EZ1, QIAGEN) and thus, do not need any detailed description here.

It is within the scope of the present invention and preferred to contact the bound cells after step b) with a liquid composition. This embodiment has the advantage that it may *inter alia* support the collection of the cells if magnetic particles are used, in particular if a robotic system is used wherein the magnet plunges into the reaction vessel for collecting the magnetic particles. By contacting the collected cells which are still bound to the magnetic particles with a liquid composition, it is ensured in this embodiment that the particles with the bound cells are efficiently removed from the magnet and are redispersed in the liquid composition. However, contacting the collected cells with a liquid composition has additional general advantages also when using other magnetic separation systems or other anion exchange surfaces as described herein. The liquid composition can be *inter alia* used for preparing the collected cells for the hybrid capturing assay. Thus, the liquid composition is compatible with the subsequent downstream processing, in particular the hybrid capturing assay that is performed in steps d) and e). The liquid composition may be added to the collected cells that are bound to the anion exchange surface, e.g. it may be added to the magnetic particles carrying the bound cells or *vice versa.* According to one embodiment contacting the anion exchange surface carrying the bound cells with the liquid composition results in that the collected cells are at least partially released and thus eluted from the anion exchange surface. If desired, the anion exchange surface can then be separated from the released cells. Here, any mode of separation is feasible and suitable modes include but are not limited to collecting the liquid composition comprising the released cells e.g. by aspiration or, which is feasible if magnetic particles are used, collecting and separating the magnetic particles from the remaining liquid composition comprising the released cells using a magnetic field. However, as is shown by the examples, it is not mandatory to remove, respectively separate the anion exchange surface prior to steps c) and d) and this is a particular advantage of the present invention as this again saves handling steps. The cells may even remain bound to the anion exchange surface as long as the nucleic acids are released therefrom (see step c).

### STEP C)

In step c), nucleic acids are released from the collected cells and preferably are also denatured. Here, several options exist and suitable and preferred embodiments will be described in the following.

According to a preferred embodiment, a liquid composition is contacted with the collected cells as is described in the preceding paragraph. As described above, said liquid composition may assist the release and thus the elution of the cells from the anion exchange surface, what is, however, not mandatory. Preferably, however, said liquid composition is a composition that assists the release of nucleic acids from the cells in step c).

According to one embodiment, the collected cells are contacted with a liquid composition which comprises a chaotropic agent. Any chaotropic agent can be used for this purpose that causes disorder in a protein or nucleic acid by, for example, but not limited to altering the secondary, tertiary or quaternary structure of a protein or a nucleic acid. Preferably, a chaotropic salt is used. The chaotropic salt preferably comprises guanidinium, thiocyanate, isothiocyanate, perchlorate, trichloroacetate and/or trifluoroacetate as chaotropic ion. Preferably, the chaotropic agent is selected from the group consisting of guandinium salts, guanidinium hydrochloride, guanidinium thiocyanate, guanidinium isothiocyanate, sodium thiocyanate, sodium iodide, sodium perchlorate, sodium trichloroacetate, sodium trifluroacetate and urea. Also a mixture of chaotropic agents can be used. Preferably, a guanidinium salt such as guanidinium hydrochloride, guanidinium thiocyanate or guanidinium isothiocyanate is used as chaotropic agent in the composition that assists the release of the nucleic acids in step c). The liquid composition may comprise the chaoptropic agent, which preferably is a chaotropic salt as mentioned above, in a concentration that lies in a range selected from about 0,1M up to the saturation limit, about 0,2M to 5M, about 0,3M to 4M, about 0,4M to 3M, about 0,5M to 2.5M, about 0,6M to about 2M, about 0,6M to about 1.5M and 0,6M to about 1M. Preferably, the liquid composition is a lysis solution such as a lysis buffer.

According to one embodiment, the composition comprises a chelating agent, preferably EDTA. A chelating agent is an organic compound that is capable of forming coordinate bonds with metals through two or more atoms of the organic compound. Suitable chelating agents include, but are not limited to diethylenetriaminepentaacetic acid (DTPA), ethylenedinitrilotetraacetic acid (EDTA), ethylene glycol tetraacetic acid (EGTA) and N,N-bis(carboxymethyl)glycine (NTA). According to a preferred embodiment, EDTA is used. As used herein, the term "EDTA" indicates *inter alia* the EDTA portion of an EDTA compound such as, for example, K₂EDTA, K₃EDTA or Na₂EDTA.

According to one embodiment, the composition comprises a detergent. The detergent may be non-ionic, ionic, anionic, cationic or zwitterionic.

Furthermore, the composition may comprise a preservative such as sodium azide. Furthermore, the composition may comprise a buffering agent. Preferably, a biological buffer such as HEPES, MES, MOPS, TRIS, BIS-TRIS Propane and others is comprised in the composition. Preferably, a Tris buffer is used.

In step c), the released nucleic acids are preferably also denatured. This can be achieved e.g. by adding a denaturation agent such as a base and/or heating as will be described in the following. In particular if the target nucleic acid is a double-stranded molecule such as a DNA molecule, it is preferred that in step c) the double-stranded target nucleic acid is converted to a single stranded target nucleic acid molecule to make the nucleic acids accessible to hybridization in step d).

Preferably, the liquid composition that contributes to or achieves the release of the nucleic acids from the cells has an alkaline pH value to achieve or support the denaturation of the released nucleic acids. Additionally, it was found that using a liquid composition having an alkaline pH value also assists in releasing and thus eluting the bound cells from the anion exchange surface probably due to charge shifts induced by the alkaline pH value. According to one embodiment, the liquid composition has a pH value that is selected from a pH value of 10 or more, a pH value of 11 or more, a pH value of 11.5 or more, a pH value of 12 or more, a pH value of 12.5 or more, a pH value of 12.75 or more, a pH value of 13 or more and a pH value of 13.25 or more. A high alkaline pH value supports the release of the nucleic acids and furthermore, denatures the released nucleic acids, thereby preparing the nucleic acids for step d). This embodiment is particularly preferred if the target nucleic acid is DNA because such basic pH will both nick and degrade a majority of the internal RNA in the specimen. Preferably, the denaturation is supported by heating as will be described below. In addition, alkaline treatment can disrupt interactions between peptides and nucleic acids to improve accessibility of the target nucleic acid and degrade protein. Furthermore, alkaline treatment of proteins effectively homogenizes the specimen to ensure reproducibility of analysis results for a given sample. It can also reduce the viscosity of the sample to increase kinetics, homogenize the sample, and reduce background by destroying any endogenous single stranded RNA nucleic acids, DNA-RNA hybrids or RNA-RNA hybrids in the sample. It also helps inactivate enzymes such as RNases and DNases that may be present in the sample. One skilled in that art would appreciate that if RNA is the target nucleic acid (as opposed to DNA), different reagents may be preferable. For establishing an alkaline pH value as described above, a base, preferably a chemical base such as e.g. sodium or potassium hydroxide can be added, respectively may be comprised in the liquid composition.

Using a strong alkaline liquid composition as described above is particularly suitable if the target nucleic acid is DNA. If the target nucleic acid is RNA, more moderate conditions are preferred in order to preserve the integrity of the RNA. E.g. the liquid composition may have a pH value of 7.5 to 10, 8 to 9.5 or 8.5 to 9, in particular if using an anion exchange surface which allows e.g. to release the cells at a pH value of 8 or below, preferably 7 or below.

The liquid composition that is contacted with the collected cells can be provided by one composition, e.g. one solution or may be prepared by contacting the cells with two or more separate compositions. According to one embodiment, the collected cells are contacted with two or more separate compositions in order to provide the release and denaturation conditions described above. According to one embodiment, two or more compositions are mixed to provide a liquid composition as described above prior to contracting said composition with the anion exchange surface to which the cells are bound (preferably magnetic particles). According to one embodiment, a first composition comprises the chaotropic agent and optionally the other components described above (e.g. the composition STM can be used as first composition, see examples) and a separate second composition comprises the denaturation agent (e.g. DNR as second composition, see examples). Preferably, said second composition is an alkaline solution. Any alkali that can bring the liquid composition that results from the mixing of the first and second composition to a pH range described above is suitable. Suitable concentrations of alkali include from about 1.0 N to about 2.0 N or from about 1.25 N to about 1.75 N. However, also higher concentrated alkaline solutions can be used to establish a pH value that lies in the pH range described above. Without being limited, suitable alkali include NaOH and KOH. Mixing the two (or more) compositions together provides the liquid composition that assists the release and denaturation of the nucleic acids and thus provides the conditions described above and in particular establishes the alkaline pH value described above. Preparing the liquid composition by mixing two or more separate compositions to establish the conditions described above is preferred and it is in particular preferred to add the denaturation agent as described above separately. According to one embodiment, the second composition comprising the denaturating agent is provided first and the first composition comprising the chaotropic agent is added to the second composition to provide the liquid composition described above. Preferably, the respectively obtained liquid composition is then contacted with the collected cells that are bound to the anion exchange moieties.

According to one embodiment, the surface is provided by magnetic particles, and the collected cells that are bound to the magnetic particles are contacted with an amount of the liquid composition described above that is selected from 25µl to 500µl, 30µl to 400µl, 35µl to 300µl, 40µl to 250µl, 50µl to 200µl, 60µl to 175µl, 70µl to 150µl, 80µl to 125µl and 85µl to 100µl. According to one embodiment, the liquid composition is added in a volume to achieve a particle concentration after resuspension that lies in a range selected from 2000µg/ml to 15000µg/ml, 2500µg/ml to 12500µg/ml, 3000µg/ml to 10000µg/ml; 3500µg/ml to 9500µg/ml, 4000µg/ml to 9000µg/ml, 4500µg/ml to 8750µg/ml and 5000µg/ml to 8500µg/ml. Particularly good results were achieved when resuspending the magnetic particles with the bound cells in a liquid composition volume so that 1 mg particles or less are comprised in a volume of 150µl, preferably 125µl, more preferred 100µl or less. As is shown by the examples, respective volumes and in particular a volume of the liquid composition of more than 60µl, more than 70µl and preferably more than 80µl provides particularly good results in the subsequent hybrid capturing assay. This, particularly, if the magnetic particles are not removed prior to performing step d). A range of 85µl to 100µl for the liquid composition is most preferred.

Instead of or preferably in addition to the denaturation agent described above, other methods of denaturation may be employed such as utilizing a heating step, for example, heating the sample to at least 80°C or at least 95°C to separate the nucleic acid strands. Adding an alkaline denaturation agent, which can be comprised in the liquid composition as described above, and performing a heating step is preferred to efficiently denature the released nucleic acids.

According to one embodiment, after the cells were contacted with the liquid composition described above, which preferably is an alkaline liquid composition, the resulting mixture is heated to assist the denaturation of the nucleic acids. Preferably, said mixture is heated to at least 55°C, preferably at least 60°C. Suitable temperature ranges include 55°C to 90°C, preferably 60°C to 85°C and more preferred 65°C to 80°C. Preferably, heating occurs for at least 30min, preferably at least 35min, more preferred at least 40min. Suitable time periods can be selected from 30min to 150min, 35min to 130min, 40min to 120min and 45min to 100min. The described time and temperature conditions shall provide an efficient denaturation of the nucleic acids in an acceptable amount of time, while leaving the target nucleic acid in a suitable condition for carrying out step d). The suitable time period also depends on the processed sample. E.g. when processing cell containing samples comprising specific fixatives such as SUREPATH® samples, longer incubation times of 90min (or longer if desired) at a temperature of at least 60°C, preferably at about 65°C are preferred. When processing cell-containing samples that do not comprise cross-linking fixatives such as PRESERVCYT® samples, shorter incubation times of e.g. 45min (or longer if desired) at a temperature of at least 60°C, preferably at about 65°C are sufficient. Suitable time periods can also be determined by the skilled person. It will be readily understood by one of ordinary skill in the art that longer periods of incubation at lower temperatures, or shorter periods of incubation at higher temperatures, may be balanced to provide a similar effect to the conditions described herein. The release of the nucleic acids can also be assisted by shaking. E.g. the sample treated with the liquid composition described above can be mixed by hand mixing or mechanical shaking at about 800 rpm, about 900 rpm, about 1000 rpm, between about 600 and about 1000 rpm, or between about 600 and 1200 rpm.

According to one embodiment, the sample mixture comprising the liquid composition, the cells and optionally the anion exchange surface, e.g. the magnetic particles, or an aliquot of said mixture is transferred into a new vessel, preferably a multi-well device such as a 96 well plate, prior to heating. This embodiment is advantageous as it can be easily integrated in established hybrid capturing assay work-flows and can also be integrated in automated processing systems as therein respective multi-well devices are usually processed and accordingly, existing equipment can be used.

After performing step c), a sample is obtained which comprises the released and preferably also denatured nucleic acids. Said sample optionally also comprises the anion exchange surface, e.g. magnetic particles if the magnetic particles were not separated prior to or during step c) what is preferred in order to safe handling steps.

The sample obtained after performing step c) may be stored e.g. at 4°C or below prior to performing steps d) and e).

### STEP D) AND STEP E)

In step d), a hybrid is generated between the target nucleic acid and a probe specific for the target nucleic acid. Preferably, a hybrid capturing assay is performed and the hybrid is preferably a RNA/DNA hybrid. As mentioned before, it is possible that the anion exchange surface which preferably is provided by magnetic particles comprising anion exchange moieties such as preferably PEI is not removed, respectively separated prior to step d). That the anion exchange surface may remain present during step d) was very surprising as it is known that anion exchange surfaces bind nucleic acids. Thus, there were concerns that the released nucleic acids, the nucleic acids probes and/or the hybrids could bind to the anion exchange surface and thereby would not be available for hybridization and capture. However, as is shown by the examples, it is possible that the anion exchange surface may remain present during the generation and capture of the hybrids in particular if a surface functionalized with PEI is used as anion exchange surface. This was specifically surprising as it was known that PEI can also disturb enzymatic activities and amplification reactions.

Suitable, non-limiting embodiments for carrying out steps d) and e) are described subsequently.

According to one embodiment, the method according to the present invention comprises the following steps d) and e):
*Step d):*
   - contacting the sample comprising the released and denatured nucleic acids with one or more probes specific for the target nucleic acid under conditions that allow the probes and single-stranded target nucleic acid molecules to hybridize forming double-stranded nucleic acid hybrids;
*Step e):*
   - detecting the presence or absence of double-stranded nucleic acid hybrids.

Preferably, the method according to the present invention comprises the following steps d) and e):
*Step d):*
   - contacting the sample comprising the released and denatured nucleic acids and magnetic particles that were used for binding the cells with one or more probes specific for the target nucleic acid under conditions that allow the probes and single-stranded target nucleic acid molecules to hybridize forming double-stranded nucleic acid hybrids;
*Step e):*
   - detecting the presence or absence of double-stranded nucleic acid hybrids.

As described above, the magnetic particles that were used for binding the cells preferably comprise PEI at their surface.

Preferably, the method according to the present invention comprises the following steps d) and e):
*Step d):*
   - contacting the sample comprising the released and denatured nucleic acids with one or more probes specific for the target nucleic acid under conditions that allow the probes and single-stranded target nucleic acid molecules to hybridize forming double-stranded nucleic acid hybrids;
   - capturing the double stranded nucleic acid hybrids;
   - optionally separating the double-stranded nucleic acid hybrids from un-bound nucleic acids;
*Step e)*
   - detecting the presence or absence of double-stranded nucleic acid hybrids.

Preferably, the method according to the present invention comprises the following steps d) and e):
*Step d):*
   - contacting the sample comprising the released and denatured nucleic acids and magnetic particles that were used for binding the cells with one or more probes specific for the target nucleic acid under conditions that allow the probes and single-stranded target nucleic acid molecules to hybridize forming double-stranded nucleic acid hybrids;
   - capturing the double stranded nucleic acid hybrids;
   - separating the double-stranded nucleic acid hybrids from un-bound nucleic acids thereby also removing at least a portion of the magnetic particles;
*Step e):*
   - detecting the presence or absence of double-stranded nucleic acid hybrids.

As described above, the magnetic particles that were used for binding the cells preferably comprise PEI at their surface.

According to one embodiment, the method according to the present invention comprises the following steps d) and e):
*Step d):*
   - contacting the sample comprising the released and denatured nucleic acids with one or more probes specific for the target nucleic acid under conditions that allow the probes and single-stranded target nucleic acid molecules to hybridize forming double-stranded nucleic acid hybrids;
   - capturing the double stranded nucleic acid hybrids using a first binding agent binding the double-stranded nucleic acid hybrid, thereby forming a double-stranded nucleic acid hybrid/first binding agent complex;
   - separating the double-stranded nucleic acid hybrid/first binding agent complex from unbound nucleic acids;
   - binding said complex with a further binding agent that is labelled with a detectable marker to form a double-stranded nucleic acid hybrid/first binding agent/labelled binding agent complex;
   - optionally washing the double-stranded nucleic acid hybrid/first binding agent/labelled binding agent complex;
*Step e):*
   - detecting the presence or absence of the label of the further binding agent thereby indicating the presence or absence of the target nucleic acid.

According to one embodiment, the method according to the present invention comprises the following steps d) and e):
*Step d):*
   - contacting the sample comprising the released and denatured nucleic acids and magnetic particles that were used for binding the cells with one or more probes specific for the target nucleic acid under conditions that allow the probes and single-stranded target nucleic acid molecules to hybridize forming double-stranded nucleic acid hybrids;
   - capturing the double stranded nucleic acid hybrids using a first binding agent binding the double-stranded nucleic acid hybrids, thereby forming a double-stranded nucleic acid hybrid/first binding agent complex;
   - separating the double-stranded nucleic acid hybrid/first binding agent complex from unbound nucleic acids thereby also removing at least a portion of the magnetic particles;
   - binding the complex with a further binding agent that is labelled with a detectable marker to form a double-stranded nucleic acid hybrid/first binding agent/labelled binding agent complex;
   - washing the double-stranded nucleic acid hybrid/first binding agent/labelled binding agent complex, wherein said washing removes remaining magnetic particles if still present;
*Step e):*
   - detecting the presence or absence of the label of the further binding agent thereby indicating the presence or absence of the target nucleic acid.

As described above, the magnetic particles that were used for binding the cells preferably comprise PEI at their surface.

According to one embodiment, the method according to the present invention comprises the following steps d) and e):
*Step d):*
   - contacting the sample comprising the released and denatured nucleic acids with one or more probes specific for the target nucleic acid under conditions that allow the probes and single-stranded target nucleic acid molecules to hybridize forming double-stranded nucleic acid hybrids;
   - capturing the double stranded nucleic acid hybrids on a solid support coated with a first binding agent binding the double-stranded nucleic acid hybrid, thereby forming a double-stranded nucleic acid hybrid/solid support complex;
   - separating the double-stranded nucleic acid hybrid/solid support complex from unbound nucleic acids;
   - binding the complex with a further binding agent that is labelled with a detectable marker to form a double-stranded nucleic acid hybrid/solid support/labelled binding agent complex;
   - optionally washing the double-stranded nucleic acid hybrid/solid support/labelled binding agent complex;
*Step e):*
   - detecting the presence or absence of the label of the further binding agent thereby indicating the presence or absence of the target nucleic acid.

According to one embodiment, the method according to the present invention comprises the following steps d) and e):
*Step d):*
   - contacting the sample comprising the released and denatured nucleic acids and magnetic particles that were used for binding the cells with one or more probes specific for the target nucleic acid under conditions that allow the probes and single-stranded target nucleic acid molecules to hybridize forming double-stranded nucleic acid hybrids;
   - capturing the double stranded nucleic acid hybrids on a solid support coated with a first binding agent binding the double-stranded nucleic acid hybrid, thereby forming a double-stranded nucleic acid hybrid/solid support complex;
   - separating the double-stranded nucleic acid hybrid/solid support complex from unbound nucleic acids thereby removing at least a portion of the magnetic particles;
   - binding the complex with a further binding agent that is labelled with a detectable marker to form a double-stranded nucleic acid hybrid/solid support/labelled binding agent complex;
   - washing the double-stranded nucleic acid hybrid/solid support/labelled binding agent complex with a wash buffer, wherein said washing removes remaining magnetic particles if still present;
*Step e):*
   - detecting the presence or absence of the label of the further binding agent thereby indicating the presence or absence of the target nucleic acid.

As described above, the magnetic particles that were used for binding the cells preferably comprise PEI at their surface.

Suitable and preferred embodiments and aspects of steps d) and e) will also be described in the following:

### The probes and hybridization conditions

After release and denaturation of the nucleic acids which occurs in step c) as is described above, the denatured target nucleic acids are contacted with one or more probes under conditions suitable for the one or more probes to hybridize to the target nucleic acid to form a double-stranded nucleic acid hybrid. The probe is preferably a polynucleotide probe. The probe can be full length, truncated, or synthetic DNA or full length, truncated, or synthetic RNA. Furthermore, probes comprising RNA and DNA nucleotides or comprising modified nucleotides and/or analogs of nucleotides can be used, as long as a hybrid is formed. If the target nucleic acid is DNA, then preferably the probe is RNA and if the target nucleic acid is RNA, then preferably the probe is DNA. Accordingly, a RNA/DNA hybrid is preferably formed. The probes are designed to hybridize or bind with the target nucleic acid molecules. In one aspect, the probes are capable of hybridizing or binding to HPV and HPV high risk variants. In an additional aspect, the probes are specific for HPV and HPV high risk variants. High risk (HR) probes can include probes for HPV high risk types 16, 18, 31, 33, 35, 39, 45, 51, 52, 56, 58, 59, 66, 68 and 82.

The probes may vary in amount from about 7.5 ng to about 60 ng per HPV type per assay, or from about 20 ng to about 45 ng per HPV type per assay, or about 30 ng of probe for each HPV type per assay is used. Thus, in one aspect the HR probes consist of or consist essentially of one or more probes for HPV high risk types 16, 18, 31, 33, 35, 39, 45, 51, 52, 56, 58, 59, 66, 68, and 82 or low risk HPV types 6, 11, 40, 43, 53, 61, 67, 69, 70, 71, 72, 81, and 83. The RNA probes may be short synthetic RNA probes that specifically bind only to the target nucleic acid molecule.

In a non-limiting aspect, the one or more probe used is capable of hybridizing or binding to target nucleic acid molecules that are at least 70 percent, at least 80 percent, at least 85 percent, at least 90 percent, at least 95 percent, at least 96 percent, at least 97 percent, at least 98 percent, at least 98 percent, at least 99 percent, or 100 percent identical to nucleic acid molecules associated with HPV, genetic variants of HPV, HPV DNA of a high risk HPV type, or HPV RNA of a high risk HPV type, or any one of high risk HPV types 16, 18, 31, 33, 35, 39, 45, 51, 52, 56, 58, 59, 66, 68, and 82 or any one of low risk HPV types 6, 11, 40, 43, 53, 61, 67, 69, 70, 71, 72, 81, and 83. In another aspect, the one or more probes used is complementary to HPV, genetic variants of HPV, HPV DNA of a high risk HPV type, HPV RNA of a high risk HPV type, or any one of high risk HPV types 16, 18, 31, 33, 35, 39, 45, 51, 52, 56, 58, 59, 66, 68, and 82 or any one of low risk HPV types 6, 1 1, 40, 43, 53, 61, 67, 69, 70, 71, 72, 81, and 83. Also DNA or RNA fragments of the target nucleic acids can be used.

According to one embodiment, the denatured sample is neutralized prior to or during the addition of the probes if denaturation occurred under alkaline conditions. According to one embodiment, the one or more probes are diluted in a probe diluent that also can act as a neutralizing hybridization buffer. This embodiment is advantageous in order to neutralize the alkaline pH value that was used in step c) to release and denature the nucleic acids. The probe diluent used for DNA or RNA probes will differ due to the different requirements necessary for DNA versus RNA stability. For example, if the probes are RNA, it is preferred to neutralize the sample first and then add the one or more probes or alternatively, add the RNA probe and a neutralizing agent (probe diluent) to the sample at the same time as strong alkaline pH values can destroy RNA. The probe diluent can be used to dissolve and dilute the probe and also help restore the sample to about a neutral or weakly alkaline pH, e.g., about pH 6 to about pH 9, preferably 6.5 to 8, more preferred 6.5 to about 7.5 to provide a more favorable environment for hybridization. Sufficient volume of probe diluent, e.g. one-half volume of the sample, may be used to neutralize a base-treated sample.

In an aspect, the probe diluent comprises a buffer, polyacrylic acid, NaOH and sodium azide. The probe diluent may comprise acetic acid. In one aspect, the probe diluent comprises 2.2 M BES (N,N-Bis(2-hydroxyethyl)-2-aminoethanesulfonic acid), 2.6 percent polyacrylic acid (PAA), 0.7 N NaOH and 0.05 percent sodium azide. The probe diluent may contain from about 1.2 M to about 2.6 M BES, from about 1.5 M to about 2.5 M BES; from about 1.75 M to about 2.25 M BES; from about 2 M to 2.4 M BES, or about 2.2 M BES, as well as any number within the recited ranges. In one aspect the probe diluent may contain from about 2 percent to about 3.0 percent PAA or, as well as any number within the recited ranges. In another aspect, the PAA concentration is from about 2.2 percent to about 2.7 percent. In yet another aspect, the PAA concentration is about 2.6 percent. In a further aspect the probe diluent may contain from about 0.6 N to about 0.8 N NaOH, for example, about 0.7 N NaOH. The concentration of NaOH generally increases as the amount of BES increases.

For large probes, a heated alkaline solution may be added to the sample, then probe diluent may be added to the sample at room temperature, and then the sample may be reheated. Such a process can inhibit secondary structure from forming. Binding agents such as antibodies tend to bind to structures with secondary structure. When using non-full length probes such as truncated or synthetic probes, heating the solutions or sample may not be necessary because secondary structures issues are not present. In an aspect, the sample is not heated when used with truncated or synthetic probes. After treatment with the denaturation reagent, an aliquot of neutralization buffer, in an aspect the probe diluent described, in which the one or more probes are dissolved, can be added to the sample under appropriate conditions to allow hybridization or binding of the probe and the target nucleic acid to occur. The neutralization buffer may contain a single buffering salt. In an aspect, the neutralization buffer does not contain more than a single buffering salt. The hybridization condition is sufficient to allow the one or more polynucleotide probes to anneal to a corresponding complementary nucleic acid sequence, if present, in the sample to form a double-stranded nucleic acid hybrid.

Hybridization conditions suitable for the particular probes and diluents used are employed. For example, the probes and sample nucleic acids can be incubated for a hybridization time, preferably at least about 5 to about 120 minutes, about 10 to about 100 minutes, or from about 20 to about 80 minutes, or from about 30 minutes to about 60 minutes, as well as any number within the recited ranges sufficient to allow the one or more polynucleotide probes to anneal to a corresponding complementary target nucleic acid sequence. The hybridization conditions can include a hybridization temperature of at least about 55°C, at least about 60°C, preferably from about 60°C to about 75°C, preferably 65°C to about 70°C as well as any number within the recited ranges. For a given target nucleic acid and a given probe, one of ordinary skill in the art can readily determine desired hybridization conditions and hybridization times by routine experimentation. One of ordinary skill in the art will further appreciate that the time and temperature of hybridization must be optimized, one with respect to the other. Without being limited, stringent hybridization conditions may be controlled by increasing the temperature, increasing the ionic conditions to above 0.5M (for example, NaCl), or reducing the concentration of PAA. As a non-limiting example, stringent hybridization conditions may include performing a hybridization reaction at elevated temperatures, such as of at least about 65°C.

### Capture

After the one or more probes were allowed to hybridize to the target nucleic acid molecule and to form a double-stranded nucleic acid hybrid, the hybrid is captured by a molecule that binds to the double-stranded nucleic acid hybrid formed. Such a molecule is referred to herein as first binding agent or anti-hybrid binding agent. Thereby, a double-stranded nucleic acid hybrid/first binding agent complex is formed. Binding agents specific for the double stranded nucleic acid hybrids include, but are not limited to, monoclonal antibodies, polyclonal antibodies, proteins such as but not limited to RNAse H, nucleic acids including but not limited to aptamers, or sequence specific nucleic acids. In one aspect an antibody binding the formed double-stranded nucleic acid hybrid is used as binding agent, respective antibodies are also known as anti-hybrid antibodies. Accordingly, the double-stranded nucleic acid hybrids formed in accordance with the present invention can be captured and detected using antibodies or antibody fragments that are specific to double-stranded nucleic acid hybrids. Subsequently, we will describe suitable and preferred embodiments by referring to antibodies. However, said description equally applies to antibody fragments such as Fab fragments capable of binding the hybrids or other suitable anti-hybrid binding agents that can be used as first binding agent.

The antibody is specific to double-stranded hybrids, such as but not limited to RNA/DNA hybrids; DNA/DNA hybrids; RNA/RNA hybrids; and mimics thereof, where mimics refer to molecules that behave similarly to RNA/DNA, DNA/DNA, or RNA/RNA hybrids. The anti-double-stranded nucleic acid hybrid antibody, i.e., the anti-hybrid antibody that is utilized will depend on the type of double-stranded nucleic acid hybrid formed. In one aspect, the anti-hybrid antibody is immunospecific to RNA/DNA hybrids. It will be understood by those skilled in the art that either polyclonal or monoclonal anti-hybrid antibodies can be used and/or coupled and/or immobilized on a support in the present method as described below. In one aspect, monoclonal antibodies support high stringency incubation temperatures during the capture step. The first and further binding agents, which preferably are antibodies may be the same for capture and detection or may be different from each other. In one aspect, the first and further binding agent (which can be labelled, see below), which preferably are both monoclonal antibodies, used for capture and/or detection are the same and are specific for RNA-DNA hybrids. As described above, also suitable as binding agents are immunofragments or derivatives of antibodies that are specific for double-stranded hybrids where such fragments or derivatives contain the binding regions of the antibody.

In an aspect of the present invention, a monoclonal anti-RNA/DNA hybrid antibody derived from a hybridoma cell line is used. Such hybridoma cell lines are described in U.S. Pat. No. 4,865,980, U.S. Pat. No. 4,732,847, and U.S. Pat. No. 4,743,535. Hybrid-specific monoclonal antibodies may also be prepared using techniques that are standard in the art. The hybrid-specific monoclonal antibody may be used for both capturing and detecting the target nucleic acid. Also other binding agents suitable of specifically binding the formed hybrid can be used as first binding agent for capturing the hybrid.

In one aspect, a first anti-hybrid binding agent such as an anti-hybrid antibody is immobilized onto a support using techniques that are standard in the art. Examples of suitable immobilization technologies include covalent linkages or adsorption, for example, protein-protein interactions, protein-G beads, biotin- streptavidin interaction, EDAC to link to a carboxyl or tosyl group, etc., hybridization directly onto the solid support using, for example, sequence specific nucleic acids in an affinity column or coating strategies.

Supports include but are not limited to reaction vessels, including microtiter plates wherein one or more wells are functionalized with the molecule that binds the hybrid, preferably are functionalized with an anti-hybrid antibody, particles, magnetic particles, columns, plates, filter paper and dipsticks. Any support can be used as long as it allows removal, e.g. extraction of a liquid phase. Magnetic particles are useful in that they can be left in the solution and the liquid phase can be extracted or decanted, if a magnetic field is applied to immobilize the particles or the magnetic particles with the bound hybrid can be removed using a system as described above. Particles that are small and have a high surface area are preferable, such as particles about 0.5µm to 10µm, 0.75µm to 7.5µm, 0.75µm to 5µm, 0.75µm to 2.5µm and most preferred 1µm in diameter. However, when using magnetic particles as solid support for the first binding agent that binds the hybrid, it is preferred to perform a final magnetic separation step prior to performing step e) in order to ensure that the magnetic particles do not interfere with the detection.

Preferably, the support that is used for immobilising the first binding agent which binds the generated hybrid is a reaction vessel. Preferably, the support is provided by a multi-well device such as a microtiter plate, wherein the wells are at least partially functionalized with the first binding agent. This embodiment is advantageous, as it allows to easily remove the particles that were used for binding the cells in the course of the assay, if said particles were not separated prior to step d), what is preferred. The generated hybrid is captured by the binding agent and thus is immobilized to the reaction vessel, so that the remaining sample including the particles that were used for cell binding can be easily removed e.g. by aspiration and/or washing.

The hybrids are incubated with the anti-hybrid binding agent attached to the support for a sufficient amount of time to allow capture of the double-stranded nucleic acid hybrids by the immobilized anti-hybrid binding agent. Thereby, a double-stranded nucleic acid hybrid/solid support complex is formed, which also comprises the first binding agent that is used for capturing the hybrid. As described above, in a preferred aspect, the support is a reaction vessel, preferably a microtiter plate functionalized with one or more anti-hybrid binding agents such as anti-hybrid antibodies. The anti-hybrid antibody may be monoclonal or polyclonal. In one aspect the antibody is monoclonal. In one aspect, the antibody is coupled to the support by an I-ethyl-3-[3- dimethylaminopropyl] carbodiimide hydrochloride (EDAC) linker.

In one aspect, the support is a polystyrene bead. In an aspect, the support or bead coupled to the binding agent, which preferably is an antibody, is diluted in a bead dilution buffer. The bead dilution buffer is helpful in minimizing protein denaturation on the bead. One example of a bead dilution buffer comprises 6 percent casein, 100 mM Tris-HCl, 300 mM NaCl, and 0.05 percent sodium azide.

In an aspect, the support coated with the anti-hybrid antibody is incubated with the sample. Incubation may be performed at room temperature or at elevated temperatures. The incubation time can range from about 5 to about 120 minutes, about 10 to about 100 minutes, or from about 20 to about 80 minutes, or from about 30 minutes to about 60 minutes, as well as any number within the recited ranges sufficient to allow capture. The same incubation times are suitable if the first binding agent that is used for binding the formed hybrid is not bound to a solid support. The sample can be shaked during said incubation. It will be understood by those skilled in the art that the incubation time, temperature and/or shaking conditions can be varied to achieve alternative capture kinetics as desired.

Following binding and thus capture of the target nucleic acid/probe hybrid, the captured hybrid may be separated from the rest of the sample. Separation is particularly easy if the first binding agent is immobilized to a solid support. In this case, the unbound sample can e.g. simply be aspirated as is also described in the examples. According to one embodiment, one or more washing steps are performed to wash away non-captured nucleic acids and sample remainders. As described above, if the particles that were used for binding the cells are still present during the generation and capture of the hybrid, they will at least be partially removed during said separation step and/or the subsequent washing steps if performed what is preferred. As described above, said particles are preferably magnetic particles which preferably carry PEI as anion exchange moiety at their surface. Advantageously, it is not necessary to specifically remove the particles e.g. by the aid of a magnet in case of magnetic particles as they will be automatically removed when separating the sample remainders and/or the subsequent washing steps which are preferably performed. This again saves handling steps.

According to one embodiment, a further binding agent is used in step d). The further binding agent may comprise a detectable label. The further binding agent is used to allow detecting the presence of double- stranded nucleic acid hybrids. The further binding agent can be bound directly or indirectly to the complex that is formed when the first binding agent binds and thus captures the formed hybrid, thereby providing a double-stranded nucleic acid hybrid/first binding agent/labelled binding agent complex or a double-stranded nucleic acid hybrid/solid support/labelled binding agent complex if the first binding agent was immobilized to a solid support. In one aspect, the further binding agent comprises a label that must react with a substrate to provide a signal that can be detected. The further binding agent may be dissolved in a suitable buffer. In one aspect the buffer comprises 100 mM TrisHCl, pH 7.4, 0.5 M NaCl, 0.1 mM ZnCl₂, 1.0 mM MgCl₂, 0.25 percent Tween 20, 0.2 mg/ml RNase A, 4 percent hydroxypropyl-b-cyclodextrin (cyclodextrin), 30 percent bead dilution buffer as discussed previously, 0.05 percent goat IgG, 0.05 percent sodium azide. Preferably, the further binding agent is an antibody or fragment thereof, preferably a monoclonal antibody.

According to one embodiment, the further binding agent comprises a detectable label and binds to the double-stranded nucleic acid hybrid. Alternatively, the further binding agent which comprises a detectable label binds the first binding agent. Alternatively, the formed double-stranded nucleic acid hybrids can be detected with a second binding agent that is not directly labelled. In this embodiment, a second binding agent is used which may bind to the double stranded nucleic acid hybrid or to the first binding agent and said second binding agent can be bound by a further binding agent which comprises a detectable label. For example, the second binding agent can be a mouse immunoglobulin that is detected by a labelled third antibody, e.g. a goat anti-mouse antibody. Respective binding strategies suitable for providing a complex with a detectable label are well known in the prior art and thus, do not need any further description here.

In an aspect, the binding reaction of the labelled binding agent to the complex comprising the captured hybrid takes place at room temperature. In an aspect, the binding reaction takes place at room temperature for between about 15 minutes and 120 minutes, 20minutes and 100 minutes, 25 minutes and 80 minutes, 30 minutes and 60 minutes or 30 minutes and 45 minutes. The binding reaction may take place at room temperature or at elevated temperatures.

It will be understood by those skilled in the art that any detectable label such as, but not limited to, an enzyme, radioactive molecule, fluorescent molecule, or metal particle such as gold particle can be used. In certain aspects, the detectable label is alkaline phosphatase. Methods of conjugating a label to an antibody are known. For example, an antibody can be reduced with dithiothreitol (DTT) to yield monovalent antibody fragments. The reduced antibody can then be directly conjugated to maleinated alkaline phosphatase by the methods of Ishikawa et al, J. Immunoassay 4:209-237 (1983) and Means et al, Chem. 1: 2-12 (1990), and the resulting conjugate can be purified by HPLC. The conjugate may also be purified using any type of size-exclusion chromatography. One benefit of purification is that the conjugates of one protein to one antibody can be separated from those conjugates with other ratios of protein to antibody.

### Wash

Following binding with the further binding agent comprising a detectable label, the sample can be washed with a wash buffer. The wash buffer may contain one or more detergents or may be free of a detergent. If the wash buffer contains a detergent, the detergent preferably is an ionic or a non-ionic detergent. One example of a non-ionic detergent is Triton-X. The detergent may be present in the wash buffer at a concentration of about 0.05 percent to about 1.5 percent, or from about 0.075 percent to about 1.0 percent, or from about 0.1 percent to about 0.75 percent, or about 0.5 percent or any number within the recited ranges. One example of a suitable wash buffer comprises 40 mM Tris, pH 8.2, 100 mM NaCl, 0.5 percent Triton-X 100 and 0.05 percent sodium azide.

The sample may be washed with the wash buffer from one to ten times, or from three to seven times, or from four to six times, or five times, or any number within the recited ranges. The sample may also be washed with a single wash buffer or with multiple wash buffers. Each wash may use the same wash buffer or a different wash buffer. For example, a detergent-containing wash buffer may be used for one wash while a detergent-free wash buffer may be used for another wash. In an aspect, one of the wash buffers does not include Triton.

Performing said one or more washing steps has the advantage that any remaining traces of magnetic particles that were used for binding the cells and which were present during hybrid capturing can be washed away efficiently and thus are removed prior to performing detection step e). The particles can be particularly easily washed away if they have a size of 7.5µm or less, preferably 5µm or less, more preferably 2.5µm or less. Therefore, according to a preferred embodiment, no magnetic separation step is performed in step d) for removing the magnetic particles that were used for collecting the cells.

### Step e) - Detection

In step e), the label present on the further binding agent is detected to thus indicate the presence or absence of the target nucleic acid molecule. Methods for detecting various labels are known in the art. For example, colorimetry, radioactive, surface plasmon resonance, or chemiluminescence methods are described by e.g., Coutlee et at., J. Clin. Microbiol. 27:1002-1007 (1989).

For example, a bound alkaline phosphatase conjugate can be detected by chemiluminescence with a reagent such as a LUMI-PHOS 530 reagent (Lumigen, Detroit, MI) or DR2 (Applied Biosystems, Foster City, CA) using a detector such as an FVLUMINA luminometer (Source Scientific Systems, Inc., Garden Grove, CA), an OPTOCOMP I Luminometer (MGM Instruments, Hamden, CT), or the like, such as a Veritas Microplate Luminometer by Turner Biosystems. Multiple detection techniques can also be used in sequence or in parallel. For example, the conjugate may be detected by chemiluminescence and/or fluorescence. In another aspect, the conjugate can be detected by chemiluminescence.

As described herein, detection of the label is indicative of the presence of one or more of the target nucleic acid molecules in the sample that are complementary to the one or more probes. Following washing (see above), the sample is suspended in a detection buffer that for example, contains the substrate for the label on the labelled binding agent.

One reason why the presence of HPV or other target nucleic acid molecules can be determined in short periods of time is because the method that is based on hybrid capturing does not require an amplification of the target nucleic acid molecule prior to detection. Instead of target amplification, signal amplification may be used to accurately detect the presence of HPV or other target nucleic acid molecules. In an aspect, the methods of the present invention may include a signal amplification step. In an aspect, the methods of the present invention do not include a target amplification step and in particular do not include a PCR amplification step in step d) or preferably, do not include a target amplification step at all. In another aspect, the methods of the disclosure may include a signal amplification step and no target nucleic acid amplification step.

The present disclosure also provides methods and assays for detecting cancer or determining the risk for developing cancer, for example cervical cancer, by detecting the presence of a target nucleic acid molecule, such as HPV, in a sample using the method discussed above.

It will be understood to those skilled in the art that the detection can be carried out on a number of platforms including, but not limited to, tubes, dipsticks, microarrays, microplates, 384 well plates, other microliter plates and microfluidic systems.

### Target nucleic acids

The target nucleic acid can be of any type and may be selected from cellular nucleic acids, preferably genomic DNA or cellular RNA, tumor derived nucleic acids, pathogen derived nucleic acids such as e.g. pathogen nucleic acids, in particular viral nucleic acids. In an exemplary embodiment, the target nucleic acid is derived from a pathogen such as a microorganism or virus. In a further example, the nucleic acid is derived from a virus. In a further aspect, the viral nucleic acid is a virally-derived DNA molecule association with the cell in an intact virus or a viral caspid; maintained in the cell episomally; or integrated in a cellular DNA molecule, such as a chromosome of the cell. In a further embodiment, the viral nucleic acid is an mRNA encoded by a viral gene or a cDNA molecule derived from such an mRNA. If the target nucleic acid is cDNA, a reverse transcription step is employed. In a further embodiment, the target nucleic acid is derived from a human papillomavirus. In a further embodiment, the automated method is a method of analyzing or screening clinical samples for the presence of a high-risk human papillomavirus.

In one embodiment, the target nucleic acid molecules are at least 70 percent, at least 80 percent, at least 85 percent, at least 90 percent, at least 95 percent, at least 96 percent, at least 97 percent, at least 98 percent, at least 98 percent, at least 99 percent, or 100 percent identical to nucleic acid molecules associated with or comprised in any one of cervical samples (e.g., a sample obtained from a cervical swab) or cervical cell samples, adenoid cells, anal epithelial cells, blood, saliva, cerebral spinal fluid, pleural fluid, milk, lymph, sputum, urine and semen, other viral, bacteria, mycobacteria or plasmodia containing cell samples, for example cytomegalovirus (CMV), HPV, herpes, HIV, HINI, chlamydia, gonorrhea, Neisseria gonorrhoeae (GC), Chlamydia trachomatis (CT), Trichomonas vaginalis, Staphylococcus aureus, tuberculosis, SARS-associated coronavirus or influenza or cell samples suspected of containing respective pathogens.

In one embodiment, the target nucleic acid molecules are derived from human papillomavirus (HPV), including genetic variants of HPV. A variant includes polymorphisms, mutants, derivatives, modified, altered, or the like forms of the target nucleic acid. In one aspect, the target nucleic acid is an HPV nucleic acid. In another aspect, the HPV nucleic acid is HPV DNA of a high risk HPV type. In another aspect, the HPV nucleic acid is HPV RNA of a high risk HPV type. In another aspect the target nucleic acids are any one of high risk HPV types 16, 18, 26, 31, 33, 35, 39, 45, 51, 52, 56, 58, 59, 66, 68, and 82 or any one of low risk HPV types 6, 1 1, 40, 43, 53, 61, 67, 69, 70, 71, 72, 81, and 83. In one aspect, the target nucleic acid molecule is at least 70 percent, at least 80 percent, at least 85 percent, at least 90 percent, at least 95 percent, at least 96 percent, at least 97 percent, at least 98 percent, at least 98 percent, at least 99 percent, or 100 percent identical to nucleic acid molecules associated with any one of HPV, genetic variants of HPV, HPV DNA of a high risk HPV type, or HPV RNA of a high risk HPV type. In another aspect the target nucleic acids are at least 70 percent, at least 80 percent, at least 85 percent, at least 90 percent, at least 95 percent, at least 96 percent, at least 97 percent, at least 98 percent, at least 98 percent, at least 99 percent, or 100 percent identical to nucleic acid molecules associated with any one of high risk HPV types 16, 18, 26, 31, 33, 35, 39, 45, 51, 52, 56, 58, 59, 66, 68, and 82 or any one of low risk HPV types 6, 11, 40, 43, 53, 6i, 67, 69, 70, 71, 72, 81, and 83.

The target nucleic acid molecule may be DNA or RNA. In one aspect, the target nucleic acid to be detected is DNA (e.g., HPV genomic DNA or cDNA) or RNA (e.g., mRNA, ribosomal RNA, nuclear RNA, transfer RNA, viral RNA, heterogeneous nuclear RNA, small non-coding RNA, siRNA, miRNA), wherein the one or more polynucleotide probes are polyribonucleotides or polydeoxyribonucleotides, respectively or mixtures thereof. The probes may also comprise modified nucleotides. When the target nucleic acid molecule is DNA, the polynucleotide probe is preferably RNA and when the target nucleic acid is RNA, the polynucleotide probe is preferably DNA. However, a DNA probe can be used with DNA target nucleic acid molecule and an RNA probe can be used with RNA target nucleic acid molecule. In a preferred aspect, the double-stranded nucleic acid hybrids are DNA-RNA hybrids formed by hybridization of target DNA and probe RNA, and can be detected using an antibody that is immunospecific to RNA-DNA hybrids.

In a preferred embodiment, the target nucleic acid is derived from a pathogen such as a microorganism or virus. In a further example, the target nucleic acid is derived from a virus. In a further aspect, the viral nucleic acid is a virally-derived DNA molecule and can be present in an intact virus or a viral capsid, can be comprised in the cell in an episomal form or can be present integrated in a cellular DNA molecule, such as a chromosome of the cell. In a further embodiment, the viral nucleic acid is an mRNA encoded by a viral gene or a cDNA molecule derived from such an mRNA. In one embodiment, the target nucleic acid is derived from a human papillomavirus. In one embodiment, the automated method is a method of analyzing or screening clinical samples for the presence of a high-risk human papillomavirus.

The method according to the present invention is particularly advantageous in that it can be fully automated. Using a surface comprising anion exchange moieties in particular in form of magnetic beads comprising anion exchange moieties such as PEI allows cell isolation, lysis, and nucleic acid analysis to be performed without a centrifugation step, thereby permitting a high-throughput sample processing and analysis.

In one embodiment, detection step e) comprises the use of on an analyzer comprising: (1) a heating element; and (2) a device for detecting a detectable signal, such as a fluorimeter or a luminometer.

In a further embodiment, an automated method for detecting a target nucleic acid is provided, said method comprising immobilizing a mammalian cell as set forth above, lysing the cell, thereby releasing the nucleic acid in a lysate; and detecting the nucleic acid by a method comprising generating a DNA:RNA hybrid.

In a further embodiment, an automated method for analyzing or screening clinical samples for a disease state is provided, said method comprising: (a) immobilizing a cell comprised in the samples to the surface comprising anion exchange moieties as set forth above; (b) isolating or concentrating the cell as set forth above; (c) lysing the cells to create a lysate; (d) and detecting the presence of a target nucleic acid in the lysate, wherein the presence or absence of the biomolecule in the lysate is indicative of the disease state. Any detection method compatible with automation may be used. By way of example and not limitation, the detection method may comprise hybridizing a nucleic acid probe to the nucleic acid from the lysate. By way of example and not limitation, hybridization results in a DNA:RNA hybrid. In a further embodiment, the DNA:RNA hybrid is detected by binding an antibody specific for DNA:RNA hybrids to the DNA:RNA hybrid between the nucleic acid probe and the nucleic acid from the lysate.

In a further embodiment, an automated method for analyzing or screening clinical samples for a disease state is provided, said method comprising performing steps a) to e) as described above, wherein the presence or absence of the target nucleic acid in the cell lysate is indicative of the disease state. Any detection method compatible with automation may be used. By way of example and not limitation, a nucleic acid probe is hybridized to the target nucleic acid from the lysate. By way of example and not limitation, hybridization results in a DNA:RNA hybrid. In a further embodiment, the DNA:RNA hybrid is captured by binding an antibody specific for DNA:RNA hybrids to the DNA:RNA hybrid between the nucleic acid probe and the target nucleic acid from the lysate. Said captured hybrid is then subsequently detected.

### PARTICULARLY PREFERRED EMBODIMENTS

According to a first particularly preferred embodiment, the method of determining the presence or absence of a target nucleic acid in a cell sample comprises the following steps:
a) contacting a surface comprising anion exchange moieties with the cell sample under conditions suitable to induce binding between the cells and said surface, wherein cell binding occurs at a pH value wherein anion exchange groups of the anion exchange moieties are positively charged, wherein preferably, cell binding occurs at a pH value that is selected from a pH range of 3 to 8.5, 3.5 to 8, 3.75 to 7.75, 4 to 7.5, 4.25 to 7.25 or 4.5 to 7;
b) separating the surface with the bound cells from the remaining sample to collect the cells;
c) releasing nucleic acids from the cells and denaturing the released nucleic acids;
d) generating a hybrid between the target nucleic acid and a probe specific for the target nucleic acid, wherein step d) comprises:
   - contacting the sample comprising the released and denatured nucleic acids with one or more probes specific for the target nucleic acid under conditions that allow the probes and single-stranded target nucleic acid molecules to hybridize forming double-stranded nucleic acid hybrids, wherein preferably RNA/DNA hybrids are formed;
   - capturing the double stranded nucleic acid hybrids by using a binding agent which binds the double stranded nucleic acid hybrid;
   - optionally separating the captured double-stranded nucleic acid hybrids from unbound nucleic acids;
   - optionally washing the captured double-stranded nucleic acid hybrids;
e) detecting the presence or absence of double-stranded nucleic acid hybrids thereby indicating the presence or absence of the target nucleic acid.

Suitable and preferred embodiments of the anion exchange surface, the cell sample and steps a) to e) were described in detail above and it is referred to the above disclosure which also applies here and can be combined with said first particularly preferred embodiment. E.g. as described in detail above, the anion exchange surface is preferably provided by magnetic particles, in particular PEI functionalized magnetic particles. Furthermore, said surface may additionally comprise additional functional groups, preferably acidic groups such as carboxyl groups. Said carboxyl groups may be used for PEI functionalization as described in detail above. It is referred to the above disclosure. Furthermore, as described above, if magnetic particles are used the magnetic particles are preferably not removed prior to step d) in order to save handling steps. Therefore, preferably, the anion exchange surface is resistant to degradation under the conditions used in step d). Furthermore, as described above, preferably the cell sample comprises cells, in particular cervix cells, comprised in a liquid based cytology medium. It is referred to the above disclosure.

According to a second particularly preferred embodiment, the method of determining the presence or absence of a target nucleic acid in a cell sample comprises the following steps:
a) contacting a surface comprising anion exchange moieties with the cell sample under conditions suitable to induce binding between the cells and said surface, wherein the anion exchange moieties comprise at least one primary, at least one secondary and/or at least one tertiary amino group, wherein cell binding occurs at a pH value that is selected from a pH range of 3.5 to 8, 3.75 to 7.75, 4 to 7.5, 4.25 to 7.25 or 4.5 to 7, wherein amino groups of the anion exchange moieties are positively charged at said pH value and wherein no adaption of the binding conditions occurs;
b) separating the surface with the bound cells from the remaining sample to collect the cells;
c) contacting the collected cells that are bound to the anion exchange surface with a liquid composition that assists the release of nucleic acids from the cells, wherein the liquid composition has one or more, preferably at least two, at least three and more preferred all of the following characteristics i. to v.:
   i. it comprises a chaotropic agent;
   ii. it has an alkaline pH value;
   iii. it has a pH value selected from pH 12 or above, pH 12.5 or above, pH 13 or above or pH 13.25 or above;
   iv. it comprises one or more additives, preferably
      aa) a chelating agent;
      bb) a buffering agent; and/or
      cc) a preservative
   v. the liquid composition is obtained by mixing two or more compositions, wherein one of said compositions comprises a chaotropic agent and one of said compositions is an alkaline solution comprising an alkaline agent, preferably NaOH or KOH, preferably in a concentration of 1.0N to 2.0N;
   and releasing nucleic acids from the cells and denaturing the released nucleic acids, wherein releasing and/or denaturing the nucleic acids involves heating and wherein preferably, the sample mixture comprising the liquid composition, cells and/or lysed cells and optionally the anion exchange surface or an aliquot of said mixture is transferred into a new vessel, preferably into a well of a multi-well device, prior to heating;
d) generating a hybrid between the target nucleic acid and a probe specific for the target nucleic acid, wherein step d) comprises:
   - contacting the sample comprising the released and denatured nucleic acids with one or more probes specific for the target nucleic acid under conditions that allow the probes and single-stranded target nucleic acid molecules to hybridize forming double-stranded nucleic acid hybrids, wherein preferably RNA/DNA hybrids are formed;
   - capturing the double stranded nucleic acid hybrids by using a binding agent which binds the double stranded nucleic acid hybrid;
   - optionally separating the captured double-stranded nucleic acid hybrids from unbound nucleic acids;
   - optionally washing the captured double-stranded nucleic acid hybrids;
e) detecting the presence or absence of double-stranded nucleic acid hybrids thereby indicating the presence or absence of the target nucleic acid.

Suitable and preferred embodiments of the anion exchange surface, the cell sample, the liquid composition and steps a) to e) were described in detail above and it is referred to the above disclosure which also applies here and can be combined with said second particularly preferred embodiment. E.g. as described in detail above, the anion exchange surface is preferably provided by magnetic particles, in particular PEI functionalized magnetic particles. Furthermore, said surface may additionally comprise additional functional groups, preferably acidic groups such as carboxyl groups. Said carboxyl groups may also be PEI functionalized as described in detail above. It is referred to the above disclosure. Furthermore, as described above, if magnetic particles are used the magnetic particles are preferably not removed prior to step d) in order to save handling steps. Therefore, preferably, the anion exchange surface is resistant to degradation under the conditions used in step d). Furthermore, as described above, preferably the cell sample comprises cells, in particular cervix cells, comprised in a liquid based cytology medium. It is referred to the above disclosure.

According to a third particularly preferred embodiment, the method of determining the presence or absence of a target nucleic acid in a cell sample comprises the following steps:
a) contacting a surface comprising anion exchange moieties with the cell sample under conditions suitable to induce binding between the cells and said surface, wherein the surface is provided by magnetic particles, wherein cell binding occurs at a pH value that is selected from a pH range of 3.5 to 8, 3.75 to 7.75, 4 to 7.5, 4.25 to 7.25 or 4.5 to 7, wherein anion exchange groups of the anion exchange moieties are positively charged at said pH value and wherein, preferably, no adaption of the binding conditions occurs;
b) separating the magnetic particles with the bound cells from the remaining sample to collect the cells;
c) contacting the collected cells that are bound to the magnetic particles with a liquid composition that assists the release of nucleic acids from the cells, wherein the liquid composition has one or more, preferably at least two, at least three and more preferred all of the following characteristics i. to v.:
   i. it comprises a chaotropic agent;
   ii. it has an alkaline pH value;
   iii. it has a pH value selected from pH 12 or above, pH 12.5 or above, pH 13 or above or pH 13.25 or above;
   iv. it comprises one or more additives, preferably
      aa) a chelating agent;
      bb) a buffering agent; and/or
      cc) a preservative
      and/or
   v. the liquid composition is obtained by mixing two or more compositions, wherein one of said compositions comprises a chaotropic agent and one of said compositions is an alkaline solution comprising an alkaline agent, preferably NaOH or KOH, preferably in a concentration of 1.0N to 2.0N;
   and releasing nucleic acids from the cells and denaturing the released nucleic acids; wherein preferably, releasing and/or denaturing the nucleic acids involves heating and wherein preferably, the sample mixture comprising the liquid composition, cells and/or lysed cells and magnetic particles or an aliquot of said mixture is transferred into a new vessel, preferably into a well of a multi-well device, prior to heating;
d) generating a hybrid between the target nucleic acid and a probe specific for the target nucleic acid, wherein the magnetic particles are not magnetically separated prior to step d) and wherein step d) comprises:
   - contacting the sample comprising the released and denatured nucleic acids and magnetic particles that were used for binding the cells with one or more probes specific for the target nucleic acid under conditions that allow the probes and single-stranded target nucleic acid molecules to hybridize forming double-stranded nucleic acid hybrids;
   - capturing the double stranded nucleic acid hybrids using a first binding agent binding the double-stranded nucleic acid hybrids, thereby forming a double-stranded nucleic acid hybrid/first binding agent complex;
   - separating the double-stranded nucleic acid hybrid/first binding agent complex from unbound nucleic acids thereby also removing at least a portion of the magnetic particles;
   - binding the complex with a further binding agent that is labelled with a detectable marker to form a double-stranded nucleic acid hybrid/first binding agent/labelled binding agent complex;
   - washing the double-stranded nucleic acid hybrid/first binding agent/labelled binding agent complex, wherein said washing removes remaining magnetic particles if still present;
e) detecting the presence or absence of double-stranded nucleic acid hybrids thereby indicating the presence or absence of the target nucleic acid.

Suitable and preferred embodiments of the magnetic particles, the cell sample, the liquid composition and steps a) to e) were described in detail above and it is referred to the above disclosure which also applies here and can be combined with said third particularly preferred embodiment. Preferably, the anion exchange surface that is provided by the magnetic particles is resistant to degradation under the conditions used in step d). E.g. as described in detail above, preferably PEI functionalized magnetic particles are used and more preferably, said magnetic particles have an average size of 7.5µm or less, more preferred 5µm or less. The magnetic particles may additionally carry acidic functional groups such as carboxyl groups at their surface and as described above said functional groups may also be used for PEI functionalization of the surface. For further details regarding the magnetic particles, the used concentration of magnetic particles and/or other reagents or components used in the method, it is referred to the above disclosure. Furthermore, as described above, preferably the cell sample comprises cells, in particular cervix cells, comprised in a liquid based cytology medium. It is referred to the above disclosure.

According to a fourth particularly preferred embodiment, steps d) and e) of the method according to the third particularly preferred embodiment comprises:
*Step d):*
   - contacting the sample comprising the released and denatured nucleic acids and magnetic particles that were used for binding the cells with one or more probes specific for the target nucleic acid under conditions that allow the probes and single-stranded target nucleic acid molecules to hybridize forming double-stranded nucleic acid hybrids;
   - capturing the double stranded nucleic acid hybrids on a solid support coated with a first binding agent binding the double-stranded nucleic acid hybrid, thereby forming a double-stranded nucleic acid hybrid/solid support complex;
   - separating the double-stranded nucleic acid hybrid/solid support complex from unbound nucleic acids thereby removing at least a portion of the magnetic particles;
   - binding the complex with a further binding agent that is labelled with a detectable marker to form a double-stranded nucleic acid hybrid/solid support/labelled binding agent complex;
   - washing the double-stranded nucleic acid hybrid/solid support/labelled binding agent complex with a wash buffer, wherein said washing removes remaining magnetic particles if still present;
*Step e):*
   - detecting the presence or absence of the label of the further binding agent thereby indicating the presence or absence of the target nucleic acid.

According to a fifth particularly preferred embodiment, the method according to the third or fourth particularly preferred embodiment has the characteristic that after contacting the magnetic particles with the cell sample in step a), the magnetic particles are comprised in the resulting mixture in a concentration selected from 50µg/ml to 2000 µg/ml, 75µg/ml to 1750 µg/ml, 100 µg/ml to 1500 µg/ml, 125 µg/ml to 1250 µg/ml and 150 µg/ml to 1100 µg/ml. Furthermore, as described above, it is preferred that the collected cells that are bound to the magnetic particles are contacted with a volume of said liquid composition that is selected from 50µl to 200µl, 60µl to 175µl, 70µl to 150µl, 80µl to 125µl and 85µl to 100µl.

As described above, the method and accordingly the method according to the first, second, third, fourth or fifth particularly preferred embodiment preferably is an automated method wherein the sample processing occurs on a robotic system. This is particularly feasible if magnetic particles are used to provide the anion exchange surface. Said method can be used to analyse a sample such as e.g. a clinical sample for a disease state and/or for the presence or absence of a pathogen nucleic acid. In particular, as is demonstrated by the examples, said method is useful in the field of HPV diagnostic in order to determine the presence or absence of HPV nucleic acids, in particular high risk HPV nucleic acids, in cervix samples collected in a liquid based cytology medium.

### KITS

Furthermore, disclosed are kits suitable for performing the method according to the present invention.

According to one embodiment, a kit is provided which comprises
a) magnetic particles, wherein the surface of the magnetic particles comprise anion exchange moieties;
b) a denaturation agent which is an alkaline solution comprising a base, preferably NaOH or KOH, preferably in a concentration selected from a range of 1.0N to 2.0N; and
c) a composition comprising a chaotropic agent and optionally one or more additives selected from the group of chelating agents, buffering agents and preservatives.

According to a preferred embodiment of said kit, the kit comprises a cartridge comprising multiple troughs, wherein
a) a first trough of said cartridge comprises magnetic particles, wherein the surface of the magnetic particles comprise anion exchange moieties;
b) a second trough of said cartridge comprises a denaturation agent which is an alkaline solution comprising a base, preferably NaOH or KOH, preferably in a concentration selected from 1.0N to 2.0N; and
c) a third trough of said cartridge comprises a composition comprising a chaotropic agent and optionally one or more additives selected from the group of chelating agents, buffering agents and preservatives.

Said cartridge can be sealed e.g. with a lid to avoid a contamination of the comprised reagents. A respective cartridge can be used in conjunction with automated systems such as robotic systems that are capable of processing magnetic particles. Suitable and preferred examples of respective automated systems were described above. A respective cartridge is inserted into the automated system, thereby providing reagents required for processing the samples on the automated system according to the method according to the first aspect. Therefore, the present disclosure also pertains to an automated system capable of processing magnetic particles comprising a cartridge as described above. The cartridge can be inserted into the robotic system. Details with respect to the magnetic particles, the denaturation agent, the composition comprising the chaotropic agent, the automated system and the method according to the first aspect were described in detail above. It is referred to the above disclosure. Preferably, the kit comprises in addition to the cartridge an elution plate comprising multiple wells, wherein the wells are suitable for receiving the collected cells. After processing the samples on the automated system using the reagent cartridge described above, the cells that were collected from an individual sample by binding them to the magnetic particles are transferred together with the magnetic particles into a well of the elution plate. A respective elution plate comprising the collected cells can then be subjected to a hybrid capturing assay as described herein.

Details regarding the magnetic particles, suitable and preferred anion exchange moieties, particle sizes, magnetism and the like were already described above in conjunction with the method. It is referred to the above disclosure which also applies here.

As described above, the magnetic particles are preferably functionalized with polyethylenimine as anion exchange moieties. The advantages were explained above. As described above, the magnetic particles are preferably provided in form of a particle suspension. Suitable and preferred embodiments of said particle suspension were described above, it is referred to the respective disclosure which also applies here. As described above, the particle suspension preferably has a pH value selected from 7.5 or less, 7 or less and 6.5 or less. Furthermore, as described above, the particles are preferably comprised in the suspension in a concentration selected from 5mg/ml to 50mg/ml, 10mg/ml to 25mg/ml, 10mg/ml to 20mg/ml and 12.5 mg/ml to 17.5mg/ml.

The kit comprises a denaturation agent which is an alkaline solution comprising a base, preferably NaOH or KOH, preferably in a concentration selected from the range of 1.0N to 2.0N. However, also higher concentrated alkaline solutions can be used. Furthermore, the kit comprises a composition comprising a chaotropic agent and optionally one or more additives selected from the group of chelating agents, buffering agents and preservatives. By mixing the denaturation agent and the composition comprising a chaotropic agent, a liquid composition is provided which assists the release of the nucleic acids and/or the denaturation of the nucleic acids. Details were described above, it is referred to the respective disclosure which also applies here. A respective mixing step is preferably performed on an automated system as described above.

Optionally, the kit may also comprise reagents for performing the hybrid capturing assay such as
- at least one probe suitable for hybridizing to a target nucleic acid; and
- at least one binding agent which is capable of binding a double-stranded nucleic acid hybrid.

Thus, the kit may comprise at least one probe suitable for hybridizing to a target nucleic acid. Details regarding suitable and preferred probes and target nucleic acids were described above in conjunction with the method according to the present method; it is referred to the respective disclosure which also applies here. Preferably, the probe is a polynucleotide probe, in particular a RNA probe which accordingly forms a DNA:RNA hybrid upon binding to the single stranded target DNA. Preferably, the at least one probe is suitable for hybridizing to a HPV target nucleic acids. Details were described above in conjunction with the method; it is referred to the above disclosure which also applies here. Furthermore, the kit may comprise at least one binding agent which is capable of binding a double-stranded nucleic acid hybrid. Said binding agent is preferably an anti-hybrid antibody or anti-hybrid binding fragment thereof. Suitable and preferred embodiments of said first binding agent were described above in conjunction with the method; it is referred to the above disclosure. As described above, the at least one binding agent is preferably attached to a solid support such as to the inner surface of a reaction vessel; it is referred to the above disclosure. Preferably, the kit comprises at least one further binding agent comprising a detectable label. Details regarding said further binding agent and suitable labels were described above in conjunction with the method; it is referred to the above disclosure. Preferably, the further binding agent is conjugated to alkaline phosphatase as detectable label. Furthermore, the kit may comprise one or more detection reagents. Details were described above in conjunction with the method, it is referred to the above disclosure. E.g. a chemiluminescent substrate can be comprised that can be processed by alkaline phosphatase, thereby producing light. Furthermore, the kit may comprise one or more washing buffers. Suitable embodiments were described above in conjunction with step d) of the method. It is referred to the respective disclosure which also applies here.

According to one aspect, a kit is provided which comprises:
a) magnetic particles, wherein the surface of the magnetic particles comprise anion exchange moieties;
a) at least one probe suitable for hybridizing to a target nucleic acid; and
b) at least one binding agent which is capable of binding a double-stranded nucleic acid hybrid.

The kit may also be provided by combining two separate kits, e.g. the two kits described above. The kit components were described above.

The term "solution" as used herein, e.g. as lysis solution in particular refers to a liquid composition, preferably an aqueous composition. It may be a homogenous mixture of only one phase but it is also within the scope of the present invention that a solution that is used according to the present invention comprises solid components such as e.g. precipitates.

According to one embodiment, subject matter described herein as comprising certain steps in the case of methods or as comprising certain ingredients in the case of compositions, solutions and/or buffers refers to subject matter consisting of the respective steps or ingredients.

Numeric ranges are inclusive of the numbers defining the range. The headings provided herein are not limitations of the various aspects or embodiments of this disclosure which can be read by reference to the specification as a whole. It is preferred to select and combine preferred embodiments described herein and the specific subject-matter arising from a respective combination of preferred embodiments also belongs to the present disclosure.

The method according to the present invention is now described by way of non-limiting examples. Examples not pertaining to the claimed invention are for illustrative purposes only.

### EXAMPLES

### Example 1: Comparison of manual AXpH-direct and manual conversion

### 1.1) Reference: Sample preparation by manual conversion (MC)

In case of MC, which is the standard prior art method, sample preparation was performed as follows: Samples from a HPV negative clinical pool and a HPV positive clinical pool, both in SUREPATH^{®} medium (see below), were used. SUREPATH® Post-gradient samples were obtained by density gradient centrifugation and the respectively obtained samples were further processed as described in the following.

First, the samples were vortexed thoroughly. 2.8 ml of each sample were transferred to a 15 ml centrifuge tube and centrifuged for 10 min at 800 g. After centrifugation, the supernatant was decanted and the centrifuge tube (with the opening downwards) was dabbed on a cloth 3 times. 200 µl of Specimen Transport Medium (STM, Digene - comprising a chaotropic agent) were added to each pellet, i.e. sample, and vortexed for 15 sec at maximum speed until the complete pellet was resuspended.

100 µl of Denaturation Reagent (DNR, 1.75 M NaOH) were added to each sample and vortexed for 5 sec at maximum speed. The samples were then incubated in a water bath for 90 min at 65 °C. Thereby, the cells were lysed to release the nucleic acids and the released nucleic acids were denatured. 75 µl of each sample (double determination) were transferred to a 96 well plate. The plate was stored at 4 °C until *digene* HC2 HPV DNA Test (Qiagen) was performed (see 2. below).

### 1.2) Sample preparation by manual AXpH-direct method (invention)

In case of the manual AXpH-direct method, sample preparation was performed as follows. SUREPATH® post-gradient samples from a HPV negative and a HPV positive clin. pool in SUREPATH^{®} medium were used. First, the samples were vortexed thoroughly. 2.8 ml of each sample were transferred to a 5 ml PP-Tube (Greiner, bio-one). 50 µl of a suspension of various types of magnetic beads carrying different anion exchange moieties were added. The suspensions comprised approx. 15mg beads/ml or 40mg/ml (see below).

**Tab. 1: Overview over the tested bead suspensions:**

| **Bead suspension** | **Description** |
|---|---|
| I | Approx. 15mg/ml beads in SNC buffer. The beads were made from carboxylated magnetic beads (Seradyn) which are functionalized at their surface with PEI as anion exchange moiety. |
| II | Approx. 40 mg/ml beads in SCN buffer. The beads were made from magnetic silica particles as carrier material. The silica surface is functionalized with N,N Diethylaminopropyltrimethoxysilan (DEAPS) as anion exchange moiety and (2,3-dihydroxypropyl)oxypropylsilane as inert ligand. |
| III | Approx. 40 mg/ml beads in fully desalinated water. The beads were made from magnetic silica particles as carrier material. The silica surface is functionalized with N,N Diethylaminopropyltrimethoxysilan (DEAPS) as anion exchange moiety and (2,3-dihydroxypropyl)oxypropylsilane as inert ligand. |
| IV | Approx. 40 mg/ml beads in SCN buffer. The beads were made from magnetic silica particles as carrier material. The silica surface is functionalized with N,N Diethylaminopropyltrimethoxysilan (DEAPS) as anion exchange moitey. |
| V | Approx. 40 mg/ml beads in fully desalinated water. The beads are made from magnetic silica particles as carrier material. The silica surface is functionalized with N,N Diethylaminopropyltrimethoxysilan (DEAPS) as anion exchange moitey. |
| VI | Approx. 40 mg/ml beads in SCN buffer. The beads were made from magnetic |
| | silica particles as carrier material. The silica surface is functionalized with N,N Diethylaminopropyltrimethoxysilan (DEAPS) as anion exchange moiety and (2,3-dihydroxypropyl)oxypropylsilane as inert ligand. |
| VII | Approx. 40 mg/ml beads in fully desalinated water. The beads were made from magnetic silica particles as carrier material. The silica surface is functionalized with N,N Diethylaminopropyltrimethoxysilan (DEAPS) as anion exchange moitey. |

The SCN buffer comprises mainly 50 mM of MES, azide, a non-ionic detergent and had a pH of 6.1.

11 samples were tested per setting. The tubes were locked with caps, cautiously inverted 10 times and vortexed for 30 sec. The samples were then incubated for 5 min at room temperature. After incubation, the samples were placed on a magnet in order to separate the beads. Subsequently, the supernatant was discarded, 200 µl of STM were added to the separated beads and vortexed for 15 sec at maximum speed. 100 µl of DNR were added to the sample and vortexed for 5 sec at maximum speed. 75 µl of each sample (double determination) including the magnetic beads were transferred to a 96 well plate. The samples were then incubated in a microplate heater for 90 min at 65 °C. This heating step ensures an efficient lysis and denaturation of the released nucleic acids. After incubation, the plate was stored at 4 °C until *digene* HC2 HPV DNA Test was performed (see 2. below).

### 2) Sample analysis by digene HC2 HPV DNA Test

The Digene HYBRID CAPTURE® System (Qiagen) was used, and the *digene* HC2 HPV DNA Test was performed according to the manufacturer's instructions.

A probe / diluent mix of 1 volume part of High-Risk HPV Probe and 25 volume parts of Probe Diluent was prepared. Thereby, the High-Risk HPV Probe was not pipetted directly into the Probe Diluent but merely onto the rim of the vessel. The probes used in said test are RNA probes. Then, thorough vortexing was performed and 25 µl of the probe / diluent mix were added to each sample obtained after following the procedure of 1.1) and 1.2) described above. After supplying the microplate with a lid, mixing was carried out for 3 min at 1100 rpm on a Digene HYBRID CAPTURE® System Rotary Shaker followed by incubation for 60 min at 65 °C on a Digene Microplate Heater I. In said incubation step, the RNA probes can hybridize to their DNA targets, thereby forming RNA:DNA hybrids.

After incubation, all samples were transferred to a Capture Microplate (white). The wells of said Capture Microplate are coated with antibodies specific for RNA:DNA hybrids. The samples were then mixed for 60 min at 1100 rpm on the rotary shaker to allow capture of the DNA:RNA hybrids by the antibodies. The liquid was rejected from the microplate and the latter was tapped on cellulose cloths. 75 µl of Detection Reagent 1 (pink) were added per well, the plate was covered with a lid and incubated for 30 min at room temperature. The Detection Reagent I comprises alkaline phosphatase conjugated antibodies specific for the RNA:DNA hybrids. Several alkaline phosphatase molecules are conjugated to each antibody. Multiple conjugated antibodies may bind to each captured RNA:DNA hybrid, resulting in signal amplification.

The samples were washed 6 times with 1x washing buffer. For this purpose, washing buffer was added to the wells until they overflow, the liquid was rejected from the microplate and, after the last washing step, the plate was tapped dry on cellulose clothes. 75 µl of Detection Reagent 2 were added per well and the plate was covered, in order to protect it from light. Said Detection Reagent 2 comprises the substrate for alkaline phosphatase. When the substrate is cleaved by the alkaline phosphatase molecules that are conjugated to the antibody comprised in Detection Reagent 1 (see above), light is emitted which can be measured as relative light units (RLU) on a luminometer. After incubation for 15 min at room temperature (protected from light), the plate was read out in a Digene DML 2000 luminometer. The intensity of the light emitted denotes the presence of absence of target DNA in the specimen.

In the AXpH-direct method (invention) the magnetic beads that were used for collecting the cells were present in the sample obtained after following the procedure described above under 1.2). Accordingly, magnetic particles were present during probe hybridisation and during capture of the RNA:DNA hybrids. Afterwards, the magnetic particles were automatically removed when following the instructions of the *digene* HC2 HPV DNA Test. In particular, the magnetic particles were removed when, after capturing the RNA:DNA hybrids, the liquid is removed prior to adding Detection Reagent 1. Furthermore, potentially remaining magnetic particles were efficiently removed during the washing steps that are performed after adding Detection Reagent 1 and prior to adding Detection Reagent 2. This example demonstrates that it is not necessary perform a separate magnetic particle removal step such as e.g. a magnetic separation step, prior to or during the hybrid capturing assay, as the magnetic particles surprisingly do not disturb the hybridisation and capture step and afterwards are automatically removed in the normal course of the procedure prior to detecting the emitted signals in the luminometer. It is advantageous that the magnetic particles are removed prior to the detection step in the luminometer, as the magnetic particles could disturb the read out of the emitted light.

### 3 Results

The obtained results are depicted in **Fig. 1** and **2****.** In case of the HPV negative samples **(****Fig. 1****),** all types of bead suspensions applied (see above) led to similar levels in RLU/CO values referred to the manual conversion (MC), i.e. to the reference method. Regarding MC, the mean value was increased due to an outlier. In case of the HPV positive samples **(****Fig. 2****),** the manual conversion showed the highest RLU/CO values followed by bead suspension I and bead suspension VII. This example demonstrates that various types of beads comprising different anion exchange moieties on their surface may be used in the method according to the present invention (AXpH-direct method).

### Exampe 2: Suitability of AXpH-direct for automation

In Example 2, the following samples were processed:
1) from a HPV negative clinical Pool in SUREPATH^{®} medium (RLU/CO approx. 0.2),
2) SiHa cells in SUREPATH^{®} medium (HPV positive; 1x10⁵ cells/ml),
3) from a HPV positive clinical Pool 1 in SUREPATH^{®} medium (RLU/CO approx. 186.4) and
4) from a HPV positive clinical Pool 2 in SUREPATH^{®} medium (RLU/CO approx. 530)

### 1.1) Reference: Manual Conversion (MC)

See **Example 1** for the manual conversion (MC) protocol.

### 1.2) Manual AXpH-direct method

First, the samples were vortexed thoroughly. 2.8 ml of each sample, i.e. 1.4 ml of the initialsample mixed with 1.4 ml of SUREPATH^{®} medium were transferred to a 5 ml PP-Tube. 50 µl of bead suspension I (see **Example 1)** were added. The tubes were locked with caps, cautiously inverted 10 times and vortexed for 30 sec. The samples were then incubated for 5 min at room temperature. After incubation, the samples were placed on a magnet in order to separate the beads. Subsequently, the supernatant was discarded, 100 µl of STM were added to the separated beads and vortexed for 15 sec at maximum speed. 50 µl of DNR were added to the sample and vortexed for 5 sec at maximum speed. 75 µl of each sample (double determination) comprising the magnetic beads were transferred to a 96 well plate. The samples were then incubated in a microplate heater for 90 min at 65 °C. After incubation, the plate was stored at 4 °C until *digene* HC2 HPV DNA Test (see below) was performed.

### 1.3) Automated AXpH-direct method (QIASYMPHONY)

The samples were subjected to an automated sample preparation using the QIASYMPHONY system (Qiagen) for processing the beads. 1.4 ml of each of the samples were supplied with 1.4 ml of SUREPATH^{®} medium (2.8ml in total). The sample prep cartridge was provided with 35-50 µl of a bead suspension I comprising 15 mg/ml of beads in SCN buffer (see **Example 1**) from the bead trough. Then, 0.7-1.0 ml of the samples in SUREPATH buffer were added to the beads and incubation was carried out for approx. 6 min at room temperature. In the meantime, a new sample cartridge was provided with 60 µl and 30 µl each of STM and DNR, respectively. The magnetic beads were collected and transferred into the STM/DNR mixture. Subsequently, the entire mixture of STM, DNR and magnetic beads was transferred to a 96 well plate. For release of the nucleic acids and denaturation, the samples were then incubated for 90 min at 65 °C in a microplate heater.

### 2) Sample analysis by digene HC2 HPV DNA Test

The *digene* HC2 HPV DNA Test was performed according to **Example** 1.

### 3 Results

The overall time required was 2 h 8 min for the automated AXpH-direct protocol (invention). This is a considerable time saving compared to other available automated protocols such as e.g. the standard automated SUREPATH protocol (QIAGEN) which requires 6 h 23 min.

The obtained results of the *digene* HC2 HPV DNA Test are depicted in **Fig. 3, 4****,** **5** and **6****.** Fig. 3 shows the results on the negative clinical pools. This test is important to determine the background. The results should be below 1, preferably below 0.5. This is achieved with the tested protocols. In case of the HPV positive samples **(****Fig. 4-6****),** when applying the automated AXpH-direct Protocol (invention), the RLU/CO values obtained were comparable with those obtained by the manual AXpH-direct method (invention) and by manual conversion (MC - prior art). Here, it is important to note that not necessarily the highest RLU/CO values are decisive. Rather, it is important that consistent and reliable results are obtained, i.e. that the amount of correct results is high. That this is achieved with the method according to the present invention is shown in the subsequent examples. However, high RLU/CO values are an advantage as it ensures that the method is very sensitive.

### Example 3: Suitability of the AXpH-direct method for PRESERVCYT^{®} and SUREPATH^{®} samples

The following samples were processed:
1. A HPV positive cell culture (SiHa cells) in PRESERVCYT^{®} (PC) medium;
2. A HPV positive cell culture (SiHa cells) in SUREPATH^{®} (SP) medium
3. A positive clinical sample pool in PRESERVCYT^{®} (PC) medium
4. A positive clinical sample pool in SUREPATH^{®} (SP) medium
5. A negative clinical sample pool in PRESERVCYT^{®} (PC) medium
6. A negative clinical sample pool in SUREPATH^{®} (SP) medium

### 1.1) Reference: Manual conversion (MC)

All samples 1 to 6 were processed with the MC protocol. See **Example 1** 1.1. for sample preparation.

### 1.2) Manual AXpH-direct method (invention)

Samples 1, 3, 4 and 5 were processed with this protocol. Here, the protocol described in **Example** 8 was followed, however, manually processing the PEI modified beads instead of using a robotic system.

### 1.3) Automated AXpH-direct method (QIASYMPHONY - invention)

Samples 2, 4 and 5 were processed with this protocol. For details, concerning sample preparation see **Example 2** (automated AXpH-direct protocol) for SUREPATH® samples and **Example 8** for PRESERVCYT® samples.

### 2) Sample analysis by digene HC2 HPV DNA Test

The *digene* HC2 HPV DNA Test was performed according to **Example 1.**

### 3 Results

The obtained results are depicted in **Fig. 7****.** When applying the manual AXpH-direct method, all obtained RLU/CO values were approx. as high as those obtained by manual conversion (MC), i.e. the reference method, as one may see in **Fig. 7** on the left. The same applies for the automated AXpH-direct method (**Fig.** 7, on the right)

### Example 4: The cells bind under various conditions to the beads

### 1) Sample preparation

In order to investigate which parameters and conditions are responsible or suitable for binding the cells to various beads, different binding conditions were tested. For this, aqueous solutions with different pH values as well as isopropanol were added to the samples. In this example, beads made from carboxylated magnetic Seradyn beads which are functionalized at their surface with PEI as anion exchange moiety were used (PEI beads) and beads that were made from magnetic silica particles as carrier material wherein the silica surface is functionalized with N,N Diethylaminopropyltrimethoxysilan (DEAPS) as anion exchange moiety (DEAPS beads). For comparison, magnetic silica beads (MAS G, QIAGEN - see EP 1 266 385) were tested.

8 post-gradient samples each of a HPV positive clinical pool in SUREPATH^{®} medium (RLU/CO approx. 70,6) were used. 700 µl each of each sample were mixed with
A: a 50 µl suspension of PEI beads (15mg/ml), 150 µl of water and
   1) 100 µl of Tris HCl pH = 5,
   2) 100 µl of Tris HCl pH = 7,
   3) 100 µl of Tris HCl pH = 9, or
   4) 500 µl of isopropanol,
B: a 50 µl suspension of DEAPS beads in water, 150 µl of water and
   1) 100 µl of Tris HCl pH = 5,
   2) 100 µl of Tris HCl pH = 7,
   3) 100 µl of Tris HCl pH = 9, or
   4) 500 µl of isopropanol
C: 50µl MAS G beads and
   1) 800µl isopropanol, 100µl NaCl (2M);
   2) 800µl H2O, 100µl NaCl (2M)

According to the manual AXpH-direct method, after incubation and magnetic separation of the beads with a Dynal^{®} MPC, two fractions were obtained per batch (1-8): a bead fraction and a supernatant fraction each. The bead fraction was resuspended in 80 µl of a STM/DNR mixture, whereas the supernatant fraction was subjected to manual conversion, i.e. the fraction was centrifuged, the resulting supernatant was discarded and the pellet was resuspended in 80 µl of a STM/DNR mixture, too (see **Example 1).** All fractions were incubated for 90 min at 65 °C in a water bath.

As reference, 700 µl of the initial samples were directly subjected to manual conversion (n = 16).

### 2) Sample analysis by digene HC2 HPV DNA Test

The *digene* HC2 HPV DNA Test HC 2.0 was performed according to **Example** 1.

### 3 Results

The obtained results are shown in **Fig. 8****,** **9** and **10****.** **Fig. 8** depicts the RLU/CO values obtained when using PEI beads. The binding performance with pH = 5 as well as pH = 7 in the binding medium was excellent, especially in comparison with the manual conversion protocol (MC). However, it is assumed that there was an error in the manual conversion protocol as the values are unusually low for the MC protocol. Usually, the values lie for the MC protocol in the range of the sample (approx. 70). However, the detected RLU/CO values are comparable with the method according to the present invention to the RLU/CO value of the sample as employed (approx. 70). Almost no signal was detected in case of the respective supernatant fractions, indicating proper and efficient binding of the cells to the beads. In contrast, when applying pH = 9 in the binding medium, the binding performance was considerably lower, i.e. not all cells bound to the tested beads. In this case, the signal detected in the supernatant fraction was approx. one forth of the one detected in the beads fraction. Using isopropanol (iPrOH) as binding medium results in excellent binding performance as well. The magnetic silica beads were under the tested conditions not suitable to achieve proper cell binding.

**Fig. 9** depicts the RLU/CO values obtained when applying DEAPS beads. The results are comparable to those obtained with the PEI beads in **Fig. 8****.** Applying pH = 5 as well as pH = 7 or iPrOH resulted in excellent binding performance, also in comparison with MC, whereas the performance at pH = 9 was considerably lower.

In **Fig. 10** the binding performances of the PEI beads and the DEAPS beads, expressed by the respective RLU/CO values in the bead fractions, are compared. In all cases, the performance of the PEI beads was higher. Therefore, it is preferred to use a PEI functionalized surface.

### Example 5: Process scheme for sample preparation and analysis

**Tab. 2** shows the steps of the Hybrid Capture (HC) assay on Rapid Capture System (RCS, Digene) using AXpH direct eluates obtained according to the invention (comprising magnetic beads, preferably PEI functionalized magnetic beads). The AxpH direct eluates may be obtained e.g. as described in **Example 1** 1.2), **Example 2** 1.3), **Example 3** or **Example 8** wherein, the processing of SUREPATH® as well as PRESERVCYT® samples is described.

**Tab. 2: Steps on the Rapid Capture System (RCS)**

| |
|---|
| Prepare the RCS: fill up all plastic consumables; prepare reagents and wash buffer; fill up system liquid etc. |
| Place the C-Rack only with non-denatured Cals&Cons on RCS |
| Place the plate with AXpH eluates on the shaker position with lid |
| Start run: RCS takes AXpH plate and pipets the Cals & Cons in 1^{st} column → Addition of 25 µl DNR1 to Cals&Cons; Addition of 25 µl DNR2 to AXpH eluates |
| Shaking of plate with lid for 3 min at 1000 rpm |
| RCS puts plate in "Incubator" at 65°C for 45 min (denaturation of both the Cals&Cons and AXpH eluates) |
| Addition of 25 µl Probe/Diluent-Mix to each well |
| Incubation for 60 min at 65 °C |
| Transfer of all samples including Cals&Cons to a hybrid capture plate |
| Shaking of capture plate for 60 min at 1000 rpm with lid |
| Aspiration of liquid from capture plate with plate washer. N.B: in this step at least a portion of the magnetic particles is removed. |
| Addition of 75 µl "Detection Reagent 1" into each well |
| Incubation for 45 min at RT |
| Washing of each plate with plate washer on RCS with wash buffer. N.B: in this step remaining traces of magnetic particles are removed. |
| Addition of 75 µl "Detection Reagent 2" into each well |
| Incubation for 15 min at RT |
| Signal of RCS to put Capture Microplate into DML 2000 (Luminometer) and start readout |

### Example 6: Reproducibility of assay results

The methods manual conversion (MC - prior art) and manual AXpH-direct (invention) were compared. Therefore, 72 individual clinical samples - HPV positive and negative - were analysed and two replicates each from the same cell pellet were used. For each pair of replicates, the detected pairs of RLU/CO values were depicted in a diagram. Comparisons were made between the individual methods **(****Fig. 11****),** in order to determine the reproducibility respectively the conformity of the HPV HC assay results. Perfect reproducibility / conformity, i.e.100 % identical results, would be the case with an intercept of 0, a slope of 1 and a correlation coefficient r² of 1.

### Results

In comparison with manual conversion (MC), using the AXpH-direct method led to a high conformity of the HPV HC assay results.

### Example 7: Influence of different parameters of sample preparation on performance of HPV HC assay

To demonstrate the influence of different parameters of sample preparation on the performance of the HPV HC assay, the method according to the invention was carried out applying a QIASYMPHONY system (automated AXpH-direct protocol) or using the manual AXpH-direct method (for both see **Example 2**). The manual conversion (MC - prior art) always served as the reference method (see **Example 1**).

### 1) Influence of elution buffer

2.8 ml each of samples in SUREPATH^{®} medium were diluted with 5.2 ml of water. 2000 µl each of these diluted samples or, alternatively, 700 µl each of the respective undiluted samples in SUREPATH^{®} medium were employed in the QIASYMPHONY system (automated AXpH-direct protocol: QS-SP-2000 µl protocol and QS-SP-700 µl protocol, respectively). In both cases, the total amount of sample applied was the same. Elution respectively resuspension of the cells/particles was performed with STM/DNR, i.e. the elution buffer of the *digene* HC2 HPV DNA Test, or an elution buffer comprising 75mM NaOH.

### Results:

As can be seen in **Fig. 12****,** both buffers could be used adequately to receive the collected cells, indicated by the obtained RLU/CO values being almost the same.

### 2) Influence of pH on cell binding

Different pH values and binding conditions were tested as indicated in **Fig. 13****.** As can be seen, the PEI beads I (see above) performed better that the DEAPS beads (see above) and thus are preferably used. For the DEAPS beads binding could be improved by adding isopropanol.

### 3) Effect of conductivity during cell binding

Applying the manual AXpH-direct method, 0.7 ml each of undiluted samples in SUREPATH^{®} medium were used. The conditions for binding to the beads were adjusted with decreasing amounts of KH₂PO₄, i.e. 1000 to 0 mM. PEI beads were used.

### Results:

As can be seen from **Fig. 14****,** low conductivity improved the binding performance of the PEI beads. The best results were obtained with 16 or 8 mM KH₂PO₄. At these concentrations the highest RLU/CO signals in the eluate fraction were found, whereas no signal was detected in the supernatant fraction.

### 4) Influence of sample acidification and dilution

2.8 ml each of samples in SUREPATH^{®} medium were diluted with 5.2 ml of water or, alternatively, with 5.2 ml of acetate. 2000 µl each of these diluted samples or, alternatively, 700 µl each of the respective undiluted samples in SUREPATH^{®} medium were subjected to the manual AXpH-direct method. PEI beads (see above) were used.

### Results:

No positive effect neither of sample dilution nor of acidification could be observed **(****Fig. 15****).** This shows that cell binding is very efficient also under different binding conditions.

### 5) Optimizing bead amounts I

Manual conversion was performed 50-fold and various amounts of PEI beads, in form of a 15 mg/ml suspension, were spiked to the resulting pool. After sample preparation, *digene* HC2 HPV DNA Test was performed.

### Results:

As shown in **Fig. 16****,** rising amounts of the PEI beads did not result in lower assay performances. This example confirms that the magnetic particles do not need to be separated prior to performing the hybrid capture assay.

### 6) Optimizing bead amounts II

700 µl each of undiluted samples in SUREPATH^{®} medium were employed in the QIASYMPHONY system (automated AXpH-direct protocol). Various amounts of PEI beads (see above) were applied in form of a 15 mg/ml suspension. As a reference, manual conversion was performed with 700 µl of the sample. After sample preparation, *digene* HC2 HPV DNA Test was performed with the eluate and with the supernatant fractions as well.

### Results:

As shown in **Fig.17****,** amounts of 40 and 50 µl of PEI beads dispersion yield the best ratio of the RLU/CO signals in the eluate / supernatant fraction, i.e. the best binding performance of the beads.

### 7) Binding of cell culture controls

Cell culture samples - SiHa or HeLa cells, alternatively- in SUREPATH^{®} medium were employed in order to investigate whether these cell cultures may be used as a positive HPV control in the method of the invention. Various amounts of PEI beads were applied in form of a 15 mg/ml suspension. The QIASYMPHONY® system was used for sample preparation. As a reference, manual conversion was carried out. After sample preparation, *digene* HC2 HPV DNA Test was performed with the eluate and with the supernatant fractions as well.

### Results:

As shown in **Fig. 18****,** decreasing amounts of PEI beads result in lower ratios of the RLU/CO signals in the eluate / supernatant fraction. Best binding performance is again found at 50 µl of bead suspension. Thus, applying PEI beads, it is possible to use both, SiHa and HeLa cells as a positive control for the HPV HC assay.

### 8) Interfering substances

Several substances, which are capable of potentially interfering with the HPV HC assay, were tested. A BD density gradient was employed to receive a postquot. The samples generated from this postquot were spiked with various interfering substances: contraceptive jelly (CJ), lubricant jelly (LJ), antifungal cream (AC), contraceptive spermicide (CS) and douche (D) or blood 1x and blood 0.3x **(****Fig. 19****).** Subsequently, sample preparation was performed by applying the AXpH-direct method.

### Results:

As shown in **Fig. 19****,** when using the AXpH-direct method only in case of contraceptive jelly (CJ) interference was found for the spiked samples compared to the negative control (w/o Inh.). Slight interference was detected for blood 1 x. Thus, the method according to the present invention is very robust and not susceptible to interference.

### 9) Limit of detection

In order to determine the limit of detection (LOD) for the AXpH-direct method, a HPV positive 8x sample pool (pool) was prepared by isolating the cells from a 8 ml postquot pool and resuspending them in 1 ml of aqueous SUREPATH^{®} medium. This 8x sample pool was subjected to a series of 1:2 dilutions. The following dilutions were obtained this way (see **Fig. 20****):** 4x (dil. 1), 2x (dil. 2), 1x (dil. 3), 1/2x (dil. 4), 1/4x (dil. 5). 12 replicates each were analysed by *digene* HC2 HPV DNA Test. As negative control, a respective negative sample pool (neg. pool) was employed. As reference method, manual conversion (MC) was performed.

### Results:

As shown in the table of **Fig. 20****,** the LOD of the AXpH-direct method is comparable to the one of MC. For the 4^{th} dilution (dil. 4), both methods still detected 4 of 12 replicates as positive, whereas for the 5^{th} dilution (dil. 5) both methods detected no more positive replicates. Moreover, the signal strength of the AXpH-direct method is approx. 85 % of the one of MC.

### 10) Influence of cellularity on cell binding

In order to determine the influence of cellularity on cell binding for the automated AXpH-direct method (QIASYMPHONY), a HPV positive 8x sample pool was prepared by isolating the cells from a 8 ml postquot pool and resuspending them in 1 ml of aqueous SUREPATH^{®} medium. This 8x sample pool was subjected to a series of 1:2 dilutions. The following dilutions were obtained this way (see **Fig. 21****):** 4x, 2x, 1 x, 1/2x, 1/4x, 1/16x, 1/32x, 1/64x, 1/128x.

### Results

As one may see in **Fig. 21****,** for automated AXpH-direct method (QIASYMPHONY) high cellularity, i.e. a high cell density has minor effect on cell binding, comparable to MC. Furthermore, the signal strength of the automated AXpH-direct method (QIASYMPHONY) is approx. 85 % of the one of MC.

### 11) Influence of elution volume

In order to investigate, whether it was possible to reduce the elution volume, the automated AXpH direct method (QIASYMPHONY) was performed and compared to manual conversion (MC). For elution/resuspension of the collected particles/cells, mixtures of 40 µl of STM with 20 µl of DNR (60 µl in total), 50 µl of STM with 25 µl of DNR (75 µl in total) and 60 µl of STM with 30 µl of DNR (90 µl in total) were used alternatively.

### Results:

As one may see in **Fig.** 22, reduced elution/resuspension volumes reduced the performance of the HPV HC assay. The best performance was found with a total volume of 90 µl.

### 12) Influence of sample input volume

In order to find out, whether it was beneficial to increase the sample input volumes, the automated the AXpH-direct method (QIASYMPHONY) was perfomed and compared to manual conversion (MC). For MC 2.8 ml samples were used, whereas for AXpH-direct 0.7 ml, 0.8 ml and 1 ml samples were used, alternatively. Samples from two independent pools (Pool 1 and Pool 2) were applied.

### Results:

As may be seen in **Fig. 23****,** increased AXpH-direct sample volumes of at least 0.8-1.0 ml are beneficial.

### Example 8: Automated AXpH-direct method with samples in PRESERVCYT^{®} medium

The following experiments were performed to further demonstrate the suitability of the automated AXpH-direct method for the preparation of samples contained in PRESERVCYT^{®} medium (see also **Example 3**). If not stated otherwise, for all of the following experiments the *digene* HC2 HPV DNA Test HC 2.0 was performed according to **Example 1.**

### 1) Comparison of HPV HC assay results when using PEI functionalized beads

Sample preparation was performed with samples in PRESERVCYT^{®} medium from two different positive clinical pools (pool 1 and pool 2) according to **Example 2** with some modifications. In case of the automated AXpH-direct protocol, 35-50 µl of a bead suspension comprising 15 mg/ml of PEI beads in SCN buffer (see **Example 1**) was obtained from the bead trough. Then, 2-3 ml of the samples in PRESERVCYT^{®} medium was added and incubation was carried out for 6 min at room temperature to bind the cells to the beads. In the meantime, a new sample cartridge was provided with 60 µl and 30 µl each of STM and DNR, respectively. The magnetic beads were collected and transferred into the STM/DNR mixture. Subsequently, the entire mixture of STM, DNR and magnetic beads was transferred to a 96 well plate. To assist the release of the nucleic acids and for denaturation, the samples were then incubated for 45 min at 65 °C in a microplate heater and the assay was performed.

Manual conversion (MC) (see **Example 1**) with a sample input of 2.0 ml served as reference.

### Results:

The results are shown in **Fig. 24****.** Regarding the application of PEI beads, the RLU/CO values obtained with the automated AXpH-direct method were comparable to or even higher than those obtained with manual conversion (MC) even though the magnetic beads were present during hybridisation and capture of the generated hybrids.

### 2) Analysis of binding efficiency for PEI beads

Sample preparation was performed according to experiment 1) of this example, wherein, however, different volumes of bead suspensions were used in the QIASYMPHONY runs. Both, the obtained eluates and the binding supernatants were analysed. Thereby, the supernatants were analysed by manual conversion (MC). Additionally, MC with a sample input of 2.0 ml served as reference.

### Results:

The results are shown in **Fig. 25****.** Regarding the PEI beads (see **Fig.25**), increasing bead amounts led to stronger HPV HC assay signals, almost comparable to MC. However, as is shown in the previous example, also higher signals can be achieved. Furthermore, the results show that increasing amounts of PEI beads led to weaker signals derived from the binding supernatants, indicating a more efficient cell binding. However, at a turning point above 30/40µl beads, only slight improvements/variations in the results were observed. Thus, higher amounts of beads are not necessary. Thus, a bead volume between 30µl to 75µl, preferably 35µl to 50µl is preferred also due to cost reasons. Using a volume of 35µl or more is beneficial because small variations in the pipetting process have no substantial influence. Using 75µl or preferable 50µl or less is beneficial because materials and hence costs can be saved.

### 3) Fine tuning of bead amounts for automated sample preparation

In order to find out the optimal bead amounts for the automated preparation of PRESERVCYT^{®} samples, samples from various cell cultures or from various clinical pools, alternatively, were prepared according to experiment 6) of **Example 7.**

In detail, the following cell cultures and clinical pools were employed:
1) SiHa cells, HPV low positive
2) HeLa cells, HPV low positive
3) SiHa cells, HPV medium/high positive
4) HeLa cells, HPV medium/high positive
5) clinical pool 1, HPV high positive
6) clinical pool 2, HPV medium positive
7) clinical pool 3, HPV low positive

Different amounts of PEI beads were tested: 25 µl, 35 µl or 50 µl of a suspension containing 15 mg/ml of beads. Manual conversion (MC) with a sample input of 2.0 ml served as reference. Regarding the cell culture samples, a number of 22-23 replicates was analysed per batch, whereas in case of the clinical pool samples, up to 66 replicates were analysed per batch.

### Results:

The results are depicted in **Fig. 26****,** **Fig. 27** and **Fig. 28****.** **Fig. 26** shows the obtained RLU/CO values for HPV low positive SiHa (top) and HeLa cells (bottom). Increasing bead amounts led to better binding performance, indicated by higher RLU/CO values. 50 µl of PEI beads performed best in this setting. **Fig. 27** shows the obtained RLU/CO values for HPV medium/high positive SiHa (top) and HeLa cells (bottom). Increasing bead amounts also led to better binding performance, indicated by higher RLU/CO values. 35 and 50 µl of PEI beads performed similar and best. **Fig. 28** shows the obtained RLU/CO values for a HPV high (top), medium (middle) and low (bottom) positive clinical pool (pool 1, 2 and 3, respectively). No significant difference in performance was found when using 25 µl to 50 µl of beads.

### 5) Link between automated AXpH-direct method and RCS

Sample preparation was performed with samples contained PRESERVCYT^{®} medium from a HPV negative and from a HPV positive clinical pool as well as from HPV positive SiHa cell culture according to experiment 1) of this example. Manual conversion (MC) with a sample input of 4 ml and the automated AXpH-direct method (QIASYMPHONY) were applied alternatively. The obtained eluate plates were then subjected to manual HPV HC 2.0 Assay, or to an automated HPV HC 2.0 Assay (see **Example 5**) using a Rapid Capture System (RCS). Optionally, the HPV HC 2.0 Assay on the RCS, was interrupted by 3 min of shaking on a rotary shaker outside the RCS to allow a complete transfer of the beads, which settle down during hybridisation, into the capture plate.

### Results:

The results for the HPV negative and positive clinical pools are shown in **Fig. 29** (top and middle, respectively). The RLU/CO values obtained when applying the AXpH-direct method are comparable to or slightly lower than those obtained when applying manual conversion (MC). Furthermore, the RLU/CO values obtained when applying automated HPV HC 2.0 Assay on the RCS are comparable to those obtained when applying the manual HPV HC 2.0 Assay. The additional shaking step had no significant impact on the performance of the HPV HC 2.0 Assay.

The results for the HPV positive SiHa cell culture are shown in **Fig. 29** on the bottom. The performance was slightly better when applying the HPV HC 2.0 Assay on the RCS than manually. However, applying the HPV HC 2.0 Assay on the RCS with an additional shaking step led to RLU/CO values comparable to those obtained with the manual assay.

### 6) Limit of detection (LOD)

In order to determine the limit of detection (LOD) for the automated AXpH-direct method (see experiment 1 of this example), a HPV positive sample pool (dilution 1) was subjected to a series of 11 1:2 dilutions (dilution 2-12). For each dilution level, 24 replicates were analysed by *digene* HC2 HPV DNA Test.. As FDA approved reference method, manual conversion (MC) was performed.

### Results:

As shown in **Tab. 3,** the LOD when applying the automated AXpH-direct method is comparable to or even slightly lower than the one obtained with MC (see no. of hits in the table). For both methods, with dilution 11 none of the replicates was longer detected as positive. Respectively, the signal strength achieved with the automated AXpH-direct method is comparable to or even slightly higher than the one with MC.

**Tab. 3: LOD**

| Dil. | No of hits MC | No of hits AXpH-direct |
|---|---|---|
| 1 | 24 | 24 |
| 2 | 24 | 24 |
| 3 | 24 | 24 |
| 4 | 24 | 24 |
| 5 | 24 | 24 |
| 6 | 15 | 22 |
| 7 | 6 | 10 |
| 8 | 2 | 3 |
| 9 | 1 | 1 |
| 10 | 1 | 1 |
| 11 | 0 | 0 |
| 12 | 0 | 1 |

### 7) Conformity with manual conversion (MC) referring to HPV HC assay results

The automated AXpH-direct method (see experiment 1 of this example: 35 µl of AXpH bead suspension) and manual conversion (MC) were compared concerning the conformity of the HPV HC assay results (ASCUS study). Therefore, 394 individual clinical samples - HPV positive (179) and negative (215) - were analysed.

In detail the percentages were:
Positive RLU/CO > 1: 179 (45 %)
High positive RLU/CO > 10: 118 (30 %)
Positive RLU/CO > 2.5: 44 (11 %)
Retest zone RLU/CO = 1-2.5: 17 (4 %)
Negative RLU/CO < 1: 215 (55 %)

Two replicates each from the same cell pellet were used. The experiment was performed by two different operators. The sample input was 2.5 ml (2.0 ml thereof being processed) and 4.0 ml each for the automated AXpH-direct method and MC, respectively. For each pair of replicates, the detected pairs of RLU/CO values were depicted in a diagram **(****Fig. 30****).** Perfect conformity, i.e.100 % identical results, would be the case with an intercept of 0, a slope of 1 and a correlation coefficient r² of 1.

### Results:

The obtained data are depicted in **Fig. 30** in 2x2 tables (complete and excluding all retest-results) and in a scatter plot. No false positive results, i.e. negative with MC but positive with the automated AXpH-direct method, were obtained outside the retest zone, the specificity of the HPV HC assay achieved thus being high. However, the sensitivity of the HPV HC assay was found to be lower in case of the automated AXpH-direct method as indicated by 21 out of 179 HPV positive samples (by MC) detected as negative. The conformity of the results obtained with the two methods was rather high, indicated by an intercept of -0.14, a slope of 1 and a correlation coefficient r² of 1.

### 8) Increased sensitivity of HPV HC assay by increased sample input

In order to investigate the influence of sample input on the sensitivity of the HPV HC assay, the automated AXpH-direct method (see experiment 1 of this example) was carried out with various volumes of sample input - 1.5 ml, 2.5 ml, 3.5 ml and 4.0 ml . Samples derived from HPV positive culture of Caski cells as well as from a HPV low positive clinical pool were employed. Manual conversion (MC) served as reference.

### Results:

The results are shown in **Fig. 31****.** Both, in case of the Caski cells and in case of the low positive pool, higher RLU/CO values, i.e. also an increased sensitivity, could be achieved by increasing the sample input volumes. Applying 4.0 ml of sample led to the highest RLU/CO values, even higher than those obtained with MC.

### Example 9: Automated AXpH-direct method with SUREPATH® pre-quot samples

Clinical performance of SUREPATH® (SP) Prequot specimens was evaluated by processing them on the QIAsymphony (QS) via the AXpH-direct method and comparing them to the same sample prepared by the PrepMate ("Postgradient" or "post-quot") and processed using the currently approved Manual Conversion method. A clinical cervical sample collected in 10ml SUREPATH® sample was obtained. A 1000µl aliquot was first taken from the full 10ml specimen (Prequot) and processed on the QS as described in **Example 2** (see 1.3). Approximately 8ml of the same specimen was then prepared on the PrepMate (Postgradient) and processed by Manual Conversion (MC). In addition, from the remaining volume not used for the PrepMate gradient, a third aliquot (Residual Prequot), also 1000 µl, was taken and processed on the QS to compare to both of the previous aliquots. All three processed samples were run in the hc2 assay on the Rapid Capture System (RCS).

QSSP AXpH-direct extracted samples were denatured on an external plate heater for 90 minutes at 70°C and stored over night at 4°C. On the next day the sample extracts were processed on the RCSusing the "C" scripts. Manually converted samples were processed on the RCS using the "C" scripts in a separate run. Separate analyses were performed for prequot and residual prequot materials. Positive, negative, and total agreement between the AXpH-direct method of the invention (SP Prequot) and the current approved Manual Conversion method was calculated. Scatter plots were generated comparing the method of the invention to current approved Manual Conversion method. The results are shown in **Fig. 32** and **Fig. 33****.** The data demonstrate the equivalent performance of the AXpH-direct sample preparation and Manual Conversion Methods. The Chi square value indicated no significant difference between the AXpH-direct and Manual Conversion methods. The agreements are ≥ 90% when comparing both prequot and residual prequot.

## Claims

1. A method of determining the presence or absence of a target nucleic acid in a cell sample, said method comprising:
a) contacting magnetic particles having a surface comprising anion exchange moieties with the sample under conditions suitable to induce binding between the cells and said surface;
b) separating the magnetic particles with the bound cells from the remaining sample to collect the cells, wherein the magnetic particles with the bound cells are processed by the aid of a magnetic field;
c) releasing nucleic acids from the cells;
d) generating a hybrid between the target nucleic acid and a probe specific for the target nucleic acid in the presence of the magnetic particles; and
e) detecting the presence or absence of the hybrid.

2. The method according to claim 1, wherein the surface comprising anion exchange moieties has at least one, at least two, at least three, at least four, or all of the following characteristics:
i. the anion exchange moieties comprise amine groups;
ii. the surface comprises polyethylenimine as anion exchange moiety;
iii. the anion exchange surface is resistant to degradation under the conditions used in step d);
iv. the surface is provided by magnetic particles having an average size selected from 5µm or less, 3µm or less, 2µm or less and 1.5µm or less, and/or
v. the surface is provided by magnetic particles which comprise carboxyl groups at the particle surface, wherein at least a portion of said carboxyl groups is functionalized with polyethylenimine to provide anion exchange moieties.

3. The method according to claim 1 or 2, wherein in the cell sample, cells are comprised in a liquid medium, preferably a liquid-based cytology medium;
and wherein optionally, after contacting the magnetic particles with the sample, the magnetic particles are comprised in the resulting mixture in a concentration selected from 50µg/ml to 2000 µg/ml, 75µg/ml to 1750 µg/ml, 100 µg/ml to 1500 µg/ml, 125 µg/ml to 1250 µg/ml and 150 µg/ml to 1100 µg/ml; and/or
wherein optionally, in step a) cell binding occurs at a pH value selected from a pH range of 3 to 8.5, 3.5 to 8.0, 3.5 to 7.75, 3.5 to 7.5, 3.5 to 7.25 or 3.75 to 7.

4. The method according to one or more of claims 1 to 3, wherein the collected cells that are bound to the anion exchange surface are contacted with a liquid composition that assists the release of nucleic acids from the cells in step c) and wherein optionally, a heating step is performed in step c) to assist the release and/or denaturation of the nucleic acids.

5. The method according to claim 4, wherein the liquid composition has one or more, preferably at least two, at least three and more preferred all of the following characteristics i. to v.:
i. it comprises a chaotropic agent;
ii. it has an alkaline pH value;
iii. it has a pH selected from pH 12 or above, pH 12.5 or above, pH 13 or above or pH 13.25 or above;
iv. it comprises one or more additives selected from
aa. a chelating agent;
bb. a buffering agent; and/or
cc. a preservative
and/or
v. the liquid composition is obtained by mixing two or more compositions, wherein one of said compositions comprises a chaotropic agent and one of said compositions is an alkaline solution comprising an alkaline agent.

6. The method according to one or more of claims 1 to 5, wherein a hybrid capturing assay is performed, and wherein step d) and e) comprise the following steps:
step d)
- contacting the sample comprising the released and denatured nucleic acids and the magnetic particles with one or more probes specific for the target nucleic acid under conditions that allow the probes and single-stranded target nucleic acid molecules to hybridize forming double-stranded nucleic acid hybrids, wherein preferably RNA/DNA hybrids are formed;
- capturing the double stranded nucleic acid hybrids by using a binding agent which binds the double stranded nucleic acid hybrid;
- optionally separating the captured double-stranded nucleic acid hybrids from un-bound nucleic acids;
step e)
- detecting the presence or absence of double-stranded nucleic acid hybrids thereby indicating the presence or absence of the target nucleic acid;
and wherein the magnetic particles are present during the generation and capture of the hybrid and are not removed in step d) by the aid of a magnet but are removed when separating the sample remainders and/or when performing one or more washing steps prior to performing detection step e).

7. The method according to one or more of claims 1 to 6, wherein the method comprises the following steps d) and e)
Step d)
- contacting the sample comprising the released and denatured nucleic acids and magnetic particles that were used for binding the cells with one or more probes specific for the target nucleic acid under conditions that allow the probes and single-stranded target nucleic acid molecules to hybridize forming double-stranded nucleic acid hybrids;
- capturing the double stranded nucleic acid hybrids;
- separating the double-stranded nucleic acid hybrids from un-bound nucleic acids thereby also removing at least a portion of the magnetic particles;
Step e)
- detecting the presence or absence of double-stranded nucleic acid hybrids.

8. The method according to one or more of claims 1 to 7, wherein the method comprises the following steps d) and e)
Step d)
- contacting the sample comprising the released and denatured nucleic acids and magnetic particles that were used for binding the cells with one or more probes specific for the target nucleic acid under conditions that allow the probes and single-stranded target nucleic acid molecules to hybridize forming double-stranded nucleic acid hybrids;
- capturing the double stranded nucleic acid hybrids using a first binding agent binding the double-stranded nucleic acid hybrids, thereby forming a double-stranded nucleic acid hybrid/first binding agent complex;
- separating the double-stranded nucleic acid hybrid/first binding agent complex from un-bound nucleic acids thereby also removing at least a portion of the magnetic particles;
- binding the complex with a further binding agent that is labelled with a detectable marker to form a double-stranded nucleic acid hybrid/first binding agent/labelled binding agent complex;
- washing the double-stranded nucleic acid hybrid/first binding agent/labelled binding agent complex, wherein said washing removes remaining magnetic particles if still present;
Step e)
- detecting the presence or absence of the label of the further binding agent thereby indicating the presence or absence of the target nucleic acid.

9. The method according to one or more of claims 1 to 8, comprising the following steps:
a) contacting magnetic particles having a surface comprising anion exchange moieties with the cell sample under conditions suitable to induce binding between the cells and said surface, wherein cell binding occurs at a pH value wherein anion exchange groups of the anion exchange moieties are positively charged, wherein preferably, cell binding occurs at a pH value that is selected from a pH range of 3 to 8.5, 3.5 to 8, 3.75 to 7.75, 4 to 7.5, 4.25 to 7.25 or 4.5 to 7;
b) separating the magnetic particles with the bound cells from the remaining sample to collect the cells, wherein the magnetic particles with the bound cells are processed by the aid of a magnetic field;
c) releasing nucleic acids from the cells and denaturing the released nucleic acids;
d) generating a hybrid between the target nucleic acid and a probe specific for the target nucleic acid, wherein step d) comprises:
- contacting the sample comprising the released and denatured nucleic acids and magnetic particles with one or more probes specific for the target nucleic acid under conditions that allow the probes and single-stranded target nucleic acid molecules to hybridize forming double-stranded nucleic acid hybrids, wherein preferably RNA/DNA hybrids are formed;
- capturing the double stranded nucleic acid hybrids by using a binding agent which binds the double stranded nucleic acid hybrid;
- optionally separating the captured double-stranded nucleic acid hybrids from un-bound nucleic acids;
- optionally washing the captured double-stranded nucleic acid hybrids;
e) detecting the presence or absence of double-stranded nucleic acid hybrids thereby indicating the presence or absence of the target nucleic acid.

10. The method according to one or more of claims 1 to 9, in particular according to claim 3, comprising the following steps:
a) contacting magnetic particles having a surface comprising anion exchange moieties with the cell sample under conditions suitable to induce binding between the cells and said surface, wherein the anion exchange moieties comprise at least one primary, at least one secondary and/or at least one tertiary amino group, wherein cell binding occurs at a pH value that is selected from a pH range of 3.5 to 8, 3.75 to 7.75, 4 to 7.5, 4.25 to 7.25 or 4.5 to 7, wherein amino groups of the anion exchange moieties are positively charged at said pH value and wherein no adaption of the binding conditions occurs;
b) separating the magnetic particles with the bound cells from the remaining sample to collect the cells, wherein the magnetic particles with the bound cells are processed by the aid of a magnetic field;
c) contacting the collected cells that are bound to the anion exchange surface with a liquid composition that assists the release of nucleic acids from the cells, wherein the liquid composition has one or more, preferably at least two, at least three and more preferred all of the following characteristics i. to v.:
i. it comprises a chaotropic agent;
ii. it has an alkaline pH value;
iii. it has a pH value selected from pH 12 or above, pH 12.5 or above, pH 13 or above or pH 13.25 or above;
iv. it comprises one or more additives selected from
aa) a chelating agent;
bb) a buffering agent; and/or
cc) a preservative
and/or
v. the liquid composition is obtained by mixing two or more compositions, wherein one of said compositions comprises a chaotropic agent and one of said compositions is an alkaline solution comprising an alkaline agent, preferably NaOH or KOH, preferably in a concentration of 1.0N to 2.0N;
and releasing nucleic acids from the cells and denaturing the released nucleic acids, wherein preferably a heating step is performed to assist the release and/or denaturation of the nucleic acids;
d) generating a hybrid between the target nucleic acid and a probe specific for the target nucleic acid, wherein step d) comprises:
- contacting the sample comprising the released and denatured nucleic acids and magnetic particles with one or more probes specific for the target nucleic acid under conditions that allow the probes and single-stranded target nucleic acid molecules to hybridize forming double-stranded nucleic acid hybrids, wherein preferably RNA/DNA hybrids are formed;
- capturing the double stranded nucleic acid hybrids by using a binding agent which binds the double stranded nucleic acid hybrid;
- optionally separating the captured double-stranded nucleic acid hybrids from un-bound nucleic acids;
- optionally washing the captured double-stranded nucleic acid hybrids;
e) detecting the presence or absence of double-stranded nucleic acid hybrids thereby indicating the presence or absence of the target nucleic acid.

11. The method according to one or more of claims 1 to 10, in particular according to claim 3, comprising the following steps:
a) contacting magnetic particles having a surface comprising anion exchange moieties with the cell sample under conditions suitable to induce binding between the cells and said surface, wherein cell binding occurs at a pH value that is selected from a pH range of 3.5 to 8, 3.75 to 7.75, 4 to 7.5, 4.25 to 7.25 or 4.5 to 7, wherein anion exchange groups of the anion exchange moieties are positively charged at said pH value and wherein, preferably, no adaption of the binding conditions occurs;
b) separating the magnetic particles with the bound cells from the remaining sample to collect the cells, wherein the magnetic particles with the bound cells are processed by the aid of a magnetic field;
c) contacting the collected cells that are bound to the magnetic particles with a liquid composition that assists the release of nucleic acids from the cells, wherein the liquid composition has one or more, preferably at least two, at least three and more preferred all of the following characteristics i. to v.:
i. it comprises a chaotropic agent;
ii. it has an alkaline pH value;
iii. it has a pH value selected from pH 12 or above, pH 12.5 or above, pH 13 or above or pH 13.25 or above;
iv. it comprises one or more additives selected from
aa) a chelating agent;
bb) a buffering agent; and/or
cc) a preservative
and/or
v. the liquid composition is obtained by mixing two or more compositions, wherein one of said compositions comprises a chaotropic agent and one of said compositions is an alkaline solution comprising an alkaline agent, preferably NaOH or KOH, preferably in a concentration of 1.0N to 2.0N;
and releasing nucleic acids from the cells and denaturing the released nucleic acids, wherein preferably a heating step is performed to assist the release and/or denaturation of the nucleic acids;
d) generating a hybrid between the target nucleic acid and a probe specific for the target nucleic acid, wherein the magnetic particles are not magnetically separated prior to step d) and wherein step d) comprises:
- contacting the sample comprising the released and denatured nucleic acids and magnetic particles that were used for binding the cells with one or more probes specific for the target nucleic acid under conditions that allow the probes and single-stranded target nucleic acid molecules to hybridize forming double-stranded nucleic acid hybrids;
- capturing the double stranded nucleic acid hybrids using a first binding agent binding the double-stranded nucleic acid hybrids, thereby forming a double-stranded nucleic acid hybrid/first binding agent complex;
- separating the double-stranded nucleic acid hybrid/first binding agent complex from un-bound nucleic acids thereby also removing at least a portion of the magnetic particles;
- binding the complex with a further binding agent that is labelled with a detectable marker to form a double-stranded nucleic acid hybrid/first binding agent/labelled binding agent complex;
- washing the double-stranded nucleic acid hybrid/first binding agent/labelled binding agent complex, wherein said washing removes remaining magnetic particles if still present;
e) detecting the presence or absence of double-stranded nucleic acid hybrids thereby indicating the presence or absence of the target nucleic acid.

12. The method according to one or more of claims 1 to 11, in particular claims 9 to 11, wherein the surface comprising anion exchange moieties is provided by magnetic particles comprising polyethylenimine as anion exchange moiety and wherein after contacting the magnetic particles with the cell sample in step a), the magnetic particles are preferably comprised in the resulting mixture in a concentration selected from 50µg/ml to 2000 µg/ml, 75µg/ml to 1750 µg/ml, 100 µg/ml to 1500 µg/ml, 125 µg/ml to 1250 µg/ml and 150 µg/ml to 1100 µg/ml;
and wherein optionally, the collected cells that are bound to the magnetic particles are contacted in step c) with a volume of said liquid composition that is selected from 50µl to 200µl, 60µl to 175µl, 70µl to 150µl, 80µl to 125µl and 85µl to 100µl and wherein the liquid composition comprises a chaotropic agent and has a pH value selected from pH 12 or above, pH 12.5 or above, pH 13 or above or pH 13.25 or above.

13. The method according to one or more of claims 1 to 12, having one or more, preferably at least two, more preferably at least three of the following characteristics i. to ix.:
i. said method is an automated method for analysing a clinical sample for a disease state and/or for determining the presence or absence of a pathogen in a clinical sample;
ii. the target nucleic acid to be detected is a pathogen nucleic acid;
iii. the target nucleic acid to be detected is a viral nucleic acid;
iv. the target nucleic acid to be detected is a HPV nucleic acid;
v. the method is used for HPV diagnostic;
vi. the cells comprised in the cell sample are epithelial cells;
vii. the cells comprised in the sample are cervix cells;
viii.the cell sample is a cervical sample collected in a liquid based cytology medium selected from SUREPATH® and PRESERVCYT®; and/or
ix. said method is an automated method for determining the presence or absence of HPV nucleic acids, preferably high risk HPV nucleic acids, in cervix samples collected in a liquid based cytology medium.

14. Use of a kit in the method according to one or more of claims 1 to 13, wherein the kit comprises:
a) magnetic particles, wherein the surface of the magnetic particles comprise anion exchange moieties;
b) a denaturation agent which is an alkaline solution comprising a base; and
c) a composition comprising a chaotropic agent.

15. The use according to claim 14, wherein the kit comprises a cartridge comprising multiple troughs, wherein
a) a first trough of said cartridge comprises magnetic particles, wherein the surface of the magnetic particles comprise anion exchange moieties;
b) a second trough of said cartridge comprises a denaturation agent which is an alkaline solution comprising a base, preferably NaOH or KOH, preferably in a concentration selected from 1.0N to 2.0N; and
c) a third trough of said cartridge comprises composition comprising a chaotropic agent and optionally one or more additives selected from the group of chelating agents, buffering agents and preservatives,
wherein the cartridge is optionally sealed.

16. The use according to claim 14 or 15, wherein the magnetic particles have one or more of the following characteristics:
i) the magnetic particles are functionalized with polyethylenimine as anion exchange moieties;
ii) the magnetic particles are comprised in a particle suspension having a pH value selected from 7.5 or less, 7 or less and 6.5 or less; and/or
iii) the magnetic particles are comprised in a particle suspension, wherein the particles are comprised in the suspension in a concentration selected from 5mg/ml to 50mg/ml, 10mg/ml to 20mg/ml and 12.5 mg/ml to 17.5mg/ml.

17. The use according to one or more of claims 14 to 16, wherein the kit has one or more of the following characteristics:
a) the kit comprises an elution plate comprising multiple wells;
b) the kit comprises at least one probe suitable for hybridizing to a target nucleic acid wherein said probe preferably is a RNA probe;
c) the kit comprises at least one binding agent which is capable of binding a double-stranded nucleic acid hybrid wherein said binding agent is preferably attached to a solid support;
d) the kit comprises one or more washing buffers;
e) the kit comprises at least one further binding agent comprising a detectable label, preferably alkaline phosphatase;
f) the kit comprises one or more detection reagents; and/or
g) the kit comprises at least one probe is suitable for hybridizing with a HPV target nucleic acids;
h) the denaturation agent is NaOH or KOH;
i) the denaturation agent comprises the base in a concentration selected from 1.0N to 2.0N;
j) the composition comprises one or more additives selected from the group of chelating agents, buffering agents and preservatives.

## Patentansprüche

1. Verfahren zur Bestimmung der Anwesenheit oder Abwesenheit einer Zielnukleinsäure in einer Zellprobe, das Verfahren umfassend:
a) Kontaktieren von magnetischen Partikeln, die eine Oberfläche mit Anionenaustauschern aufweisen, mit der Probe unter Bedingungen, die geeignet sind, um eine Bindung zwischen den Zellen und der Oberfläche zu induzieren;
b) Trennen der magnetischen Partikel mit den gebundenen Zellen von der restlichen Probe, um die Zellen zu sammeln, wobei die magnetischen Partikel mit den gebundenen Zellen mithilfe eines magnetischen Feldes prozessiert werden;
c) Freisetzen von Nukleinsäuren aus den Zellen;
d) Erzeugen eines Hybrids zwischen der Zielnukleinsäure und einer für die Zielnukleinsäure spezifischen Sonde in Anwesenheit der magnetischen Partikel; und
e) Detektion der Anwesenheit oder Abwesenheit des Hybrids.

2. Verfahren nach Anspruch 1, wobei die Anionenaustauscher umfassende Oberfläche zumindest eine, zumindest zwei, zumindest drei, zumindest vier oder alle der folgenden Eigenschaften aufweist:
i. die Anionenaustauscher umfassen Amingruppen;
ii. die Oberfläche umfasst Polyethylenimin als Anionenaustauscher;
iii. die Anionenaustauscheroberfläche ist unter den in Schritt d) verwendeten Bedingungen gegenüber Abbau resistent;
iv. die Oberfläche wird durch magnetische Partikel bereitgestellt, die eine durchschnittliche Größe aufweisen, die ausgewählt ist aus 5 µm oder weniger, 3 µm oder weniger, 2 µm oder weniger und 1,5 µm oder weniger, und/oder
v. die Oberfläche wird durch magnetische Partikel bereitgestellt, die Carboxylgruppen an der Partikeloberfläche aufweisen, wobei zumindest ein Teil der Carboxylgruppen mit Polyethylenimin funktionalisiert ist, um Anionenaustauscher bereitzustellen.

3. Verfahren nach Anspruch 1 oder 2, wobei in der Zellprobe Zellen in einem flüssigen Medium, vorzugsweise in einem flüssigkeitsbasierten Zytologiemedium, enthalten sind;
und wobei optional die magnetischen Partikel nach dem Kontaktieren der magnetischen Partikel mit der Probe in der resultierenden Mischung in einer Konzentration enthalten sind, die ausgewählt ist aus 50µg/ml bis 2000 µg/ml, 75µg/ml bis 1750 µg/ml, 100 µg/ml bis 1500 µg/ml, 125 µg/ml bis 1250 µg/ml und 150 µg/ml bis 1100 µg/ml; und/oder
wobei optional in Schritt a) die Zellbindung bei einem pH-Wert erfolgt, der ausgewählt ist aus einem pH-Bereich von 3 bis 8,5, 3,5 bis 8,0, 3,5 bis 7,75, 3,5 bis 7,5, 3,5 bis 7,25 oder 3,75 bis 7.

4. Verfahren nach einem oder mehreren der Ansprüche 1 bis 3, wobei die gesammelten Zellen, die an die Anionenaustauscheroberfläche gebunden sind, mit einer flüssigen Zusammensetzung kontaktiert werden, die die Freisetzung von Nukleinsäuren von den Zellen in Schritt c) unterstützt, und wobei optional ein Erwärmungsschritt in Schritt c) durchgeführt wird, um die Freisetzung und/oder Denaturierung der Nukleinsäuren zu unterstützen.

5. Verfahren nach Anspruch 4, wobei die flüssige Zusammensetzung eine oder mehrere, vorzugsweise zumindest zwei, zumindest drei und noch bevorzugter alle der folgenden Eigenschaften i. bis v. aufweist:
i. sie weist ein chaotropes Agens auf;
ii. sie weist einen alkalischen pH-Wert auf;
iii. sie weist einen pH auf, der ausgewählt ist aus 12 oder höher, pH 12,5 oder höher, pH 13 oder höher oder pH 13,25 oder höher;
iv. sie umfasst ein oder mehrere Zusatzstoffe ausgewählt aus:
aa. einem Chelatbildner;
bb. einer Puffersubstanz; und/oder
cc. einem Konservierungsmittel
und/oder
v. die flüssige Zusammensetzung wird durch Mischen von zwei oder mehr Zusammensetzungen erhalten, wobei eine der Zusammensetzungen ein chaotropes Agens aufweist und eine der Zusammensetzungen eine alkalische Lösung ist, die ein alkalisches Agens umfasst.

6. Verfahren nach einem oder mehreren der Ansprüche 1 bis 5, wobei ein Assay zum Fangen von Hybriden durchgeführt wird und wobei Schritt d) und e) die folgenden Schritte umfassen:
Schritt d):
- Kontaktieren der Probe, die die freigesetzten und denaturierten Nukleinsäuren und die magnetischen Partikel umfasst, mit einer oder mehreren für die Zielnukleinsäure spezifischen Sonden unter Bedingungen, die es den Sonden und einzelsträngigen Zielnukleinsäuremolekülen ermöglichen zu hybridisieren, so dass doppelsträngige Nukleinsäurehybride ausgebildet werden, wobei vorzugsweise RNA/DNA-Hybride gebildet werden;
- Fangen der doppelsträngigen Nukleinsäurehybride unter Verwendung eines Bindungsmittels, das das doppelsträngige Nukleinsäurehybrid bindet;
- optional Trennen der gefangenen doppelsträngigen Nukleinsäurehybride von ungebundenen Nukleinsäuren;
Schritt e):
- Detektion der Anwesenheit oder Abwesenheit von doppelsträngigen Nukleinsäurehybriden, wodurch die Anwesenheit oder Abwesenheit der Zielnukleinsäure angezeigt wird;
und wobei die magnetischen Partikel während dem Erzeugen und Fangen des Hybrids anwesend sind und in Schritt d) nicht mithilfe eines Magneten entfernt werden, sondern entfernt werden, wenn die Probenrückstände abgetrennt werden und/oder wenn ein oder mehr Waschschritte vor dem Durchführen des Detektionsschrittes e) durchgeführt werden.

7. Verfahren nach einem oder mehreren der Ansprüche 1 bis 6, wobei das Verfahren die folgenden Schritte d) und e) umfasst:
Schritt d):
- Kontaktieren der Probe, die die freigesetzten und denaturierten Nukleinsäuren und magnetische Partikel, die zum Binden der Zellen verwendet wurden, umfasst, mit einer oder mehr für die Zielnukleinsäure spezifischen Sonden unter Bedingungen, die es den Sonden und einzelsträngigen Zielnukleinsäuremolekülen ermöglichen zu hybridisieren, so dass doppelsträngige Nukleinsäurehybride ausgebildet werden;
- Fangen der doppelsträngigen Nukleinsäurehybride;
- Trennen der doppelsträngigen Nukleinsäurehybride von ungebundenen Nukleinsäuren, wodurch zumindest ein Teil der magnetischen Partikel ebenfalls entfernt wird;
Schritt e):
- Detektion der Anwesenheit oder Abwesenheit von doppelsträngigen Nukleinsäurehybriden.

8. Verfahren nach einem oder mehreren der Ansprüche 1 bis 7, wobei das Verfahren die folgenden Schritte d) und e) umfasst:
Schritt d):
- Kontaktieren der Probe, die die freigesetzten und denaturierten Nukleinsäuren und magnetische Partikel, die zum Binden der Zellen verwendet wurden, umfasst, mit einer oder mehr für die Zielnukleinsäure spezifischen Sonden unter Bedingungen, die es den Sonden und einzelsträngigen Zielnukleinsäuremolekülen ermöglichen zu hybridisieren, so dass doppelsträngige Nukleinsäurehybride ausgebildet werden;
- Fangen der doppelsträngigen Nukleinsäure-Hybride unter Verwendung eines ersten Bindungsmittels, das die doppelsträngigen Nukleinsäurehybride bindet, wodurch ein Komplex aus doppelsträngigem Nukleinsäurehybrid und erstem Bindungsmittel gebildet wird;
- Trennen des Komplexes aus doppelsträngigem Nukleinsäurehybrid und erstem Bindungsmittel von ungebundenen Nukleinsäuren, wodurch zumindest ein Teil der magnetischen Partikel ebenfalls entfernt wird;
- Binden des Komplexes mit einem weiteren Bindungsmittels, das mit einem detektierbaren Marker markiert ist, um einen Komplex aus doppelsträngigem Nukleinsäurehybrid, erstem Bindungsmittel und markierten Bindungsmittel zu bilden;
- Waschen des Komplexes aus einem doppelsträngigen Nukleinsäurehybrid, einem ersten Bindungsmittel und einem markierten Bindungsmittel, wobei das Waschen restliche magnetische Partikel, falls noch anwesend, entfernt;
Schritt e):
- Detektion der Anwesenheit oder Abwesenheit der Markierung des weiteren Bindungsmittels, wodurch die Anwesenheit oder Abwesenheit der Zielnukleinsäure angezeigt wird.

9. Verfahren nach einem oder mehreren der Ansprüche 1 bis 8, umfassend die folgenden Schritte:
a) Kontaktieren von magnetischen Partikel, die eine Oberfläche mit Anionenaustauschern aufweisen mit der Zellprobe unter Bedingungen, die geeignet sind, um eine Bindung zwischen den Zellen und der Oberfläche zu induzieren, wobei die Zellbindung bei einem pH-Wert erfolgt, bei dem Anionenaustauschergruppen der Anionenaustauscher positiv geladen sind, wobei die Zellbindung vorzugsweise bei einem pH-Wert erfolgt, der ausgewählt ist aus einem pH-Bereich von 3 bis 8,5, 3,5 bis 8, 3,75 bis 7,75, 4 bis 7,5, 4,25 bis 7,25 oder 4,5 bis 7;
b) Trennen der magnetischen Partikel mit den gebundenen Zellen von der restlichen Probe, um die Zellen zu sammeln, wobei die magnetischen Partikel mit den gebundenen Zellen mithilfe eines magnetischen Feldes prozessiert werden;
c) Freisetzen von Nukleinsäuren von den Zellen und Denaturieren der freigesetzten Nukleinsäuren;
d) Erzeugen eines Hybrids zwischen der Zielnukleinsäure und einer für die Zielnukleinsäure spezifischen Sonde, wobei Schritt d) umfasst:
- Kontaktieren der Probe, die die freigesetzten und denaturierten Nukleinsäuren und magnetische Partikel umfasst, mit einer oder mehreren für die Zielnukleinsäure spezifischen Sonden unter Bedingungen, die es den Sonden und einzelsträngigen Zielnukleinsäuremolekülen ermöglichen zu hybridisieren, so dass doppelsträngige Nukleinsäurehybride ausgebildet werden, wobei vorzugsweise RNA-/DNA-Hybride gebildet werden;
- Fangen der doppelsträngigen Nukleinsäurehybride unter Verwendung eines Bindungsmittels, das das doppelsträngige Nukleinsäurehybrid bindet;
- optional Trennen der gefangenen doppelsträngigen Nukleinsäurehybride von ungebundenen Nukleinsäuren;
- optional Waschen der gefangenen doppelsträngigen Nukleinsäurehybride;
e) Detektion der Anwesenheit oder Abwesenheit von doppelsträngigen Nukleinsäurehybriden, wodurch die Anwesenheit oder Abwesenheit der Zielnukleinsäure angezeigt wird.

10. Verfahren nach einem oder mehreren der Ansprüche 1 bis 9, insbesondere nach Anspruch 3, umfassend die folgenden Schritte:
a) Kontaktieren magnetischer Partikel, die eine Oberfläche mit Anionenaustauschern aufweisen, mit der Zellprobe unter Bedingungen, die geeignet sind, um eine Bindung zwischen den Zellen und der Oberfläche zu induzieren, wobei die Anionenaustauscher zumindest eine primäre, zumindest eine sekundäre und/oder zumindest eine tertiäre Aminogruppe umfassen, wobei die Zellbindung bei einem pH-Wert erfolgt, der ausgewählt ist aus einem pH-Bereich von 3,5 bis 8, 3,75 bis 7,75, 4 bis 7,5, 4,25 bis 7,25, oder 4,5 bis 7, wobei die Aminogruppen der Anionenaustauscher bei dem pH-Wert positiv geladen sind, und wobei keine Anpassung der Bindebedingungen erfolgt;
b) Trennen der magnetischen Partikel mit den gebundenen Zellen von der restlichen Probe, um die Zellen zu sammeln, wobei die magnetischen Partikel mit den gebundenen Zellen mithilfe eines magnetischen Feldes prozessiert werden;
c) Kontaktieren der gesammelten Zellen, die an die Anionenaustauscheroberfläche gebunden sind, mit einer flüssigen Zusammensetzung, die die Freisetzung von Nukleinsäuren von den Zellen unterstützt, wobei die flüssige Zusammensetzung eine oder mehr, vorzugsweise zumindest zwei, zumindest drei und bevorzugter alle der folgenden Eigenschaften i. bis v. aufweist:
i. sie umfasst ein chaotropes Agens;
ii. sie weist einen alkalischen pH-Wert auf;
iii. sie weist einen aus pH 12 oder höher, pH 12,5 oder höher, pH 13 oder höher oder pH 13,25 oder höher ausgewählten pH-Wert auf;
iv. sie umfasst einen oder mehr aus Zusatzstoffe ausgewählt aus:
aa) einem Chelatbildner;
bb) einer Puffersubstanz; und/oder
cc) einem Konservierungsmittel
und/oder
v. die flüssige Zusammensetzung wird durch Mischen von zwei oder mehr Zusammensetzungen erhalten, wobei eine der Zusammensetzungen ein chaotropes Agens umfasst und eine der Zusammensetzungen eine alkalische Lösung ist, die ein alkalisches Agens, vorzugsweise NaOH oder KOH, vorzugsweise in einer Konzentration von 1,0 N bis 2,0 N, umfasst;
und Freisetzen von Nukleinsäuren aus den Zellen und Denaturieren der freigesetzten Nukleinsäuren, wobei vorzugsweise ein Erwärmungsschritt durchgeführt wird, um die Freisetzung und/oder Denaturierung der Nukleinsäuren zu unterstützen;
d) Erzeugen eines Hybrids zwischen der Zielnukleinsäure und einer für die Zielnukleinsäure spezifischen Sonde, wobei Schritt d) umfasst:
- Kontaktieren der Probe, die die freigesetzten und denaturierten Nukleinsäuren und magnetische Partikel umfasst, mit einer oder mehreren für die Zielnukleinsäure spezifischen Sonden unter Bedingungen, die es den Sonden und einzelsträngigen Zielnukleinsäuremolekülen ermöglichen zu hybridisieren, so dass doppelsträngige Nukleinsäurehybride ausgebildet werden, wobei vorzugsweise RNA/DNA-Hybride gebildet werden;
- Fangen der doppelsträngigen Nukleinsäurehybride unter Verwendung eines Bindungsmittels, das das doppelsträngige Nukleinsäurehybrid bindet;
- optional Trennen der gefangenen doppelsträngigen Nukleinsäurehybride von ungebundenen Nukleinsäuren;
- optional Waschen der gefangenen doppelsträngigen Nukleinsäurehybride;
e) Detektion der Anwesenheit oder Abwesenheit von doppelsträngigen Nukleinsäurehybriden, wodurch die Anwesenheit oder Abwesenheit der Zielnukleinsäure angezeigt wird.

11. Verfahren nach einem oder mehreren der Ansprüche 1 bis 10, insbesondere nach Anspruch 3, umfassend die folgenden Schritte:
a) Kontaktieren von magnetischen Partikeln, die eine Oberfläche mit Anionenaustauschern aufweisen, mit der Zellprobe unter Bedingungen, die geeignet sind, um das Binden zwischen den Zellen und der Oberfläche zu induzieren, wobei die Zellbindung bei einem pH-Wert erfolgt, der ausgewählt ist aus einem pH-Bereich von 3,5 bis 8, 3,75 bis 7,75, 4 bis 7,5, 4,25 bis 7,25, oder 4,5 bis 7, und wobei die Anionenaustauschgruppen der Anionenaustauscher bei dem pH-Wert positiv geladen sind, wobei vorzugsweise keine Anpassung der Bindebedingungen erfolgt;
b) Trennen der magnetischen Partikel mit den gebundenen Zellen von der restlichen Probe, um die Zellen zu sammeln, wobei die magnetischen Partikel mit den gebundenen Zellen mithilfe eines magnetischen Feldes prozessiert werden;
c) Kontaktieren der gesammelten Zellen, die an die magnetischen Partikel gebunden sind, mit einer flüssigen Zusammensetzung, die die Freisetzung von Nukleinsäuren von den Zellen unterstützt, wobei die flüssige Zusammensetzung eine oder mehr, vorzugsweise zumindest zwei, zumindest drei und noch bevorzugter alle der folgenden Eigenschaften i. bis v. aufweist:
i. sie umfasst ein chaotropes Agens;
ii. sie weist einen alkalischen pH-Wert auf;
iii. sie weist einen aus pH 12 oder höher, pH 12,5 oder höher, pH 13 oder höher oder pH 13,25 oder höher ausgewählten pH-Wert auf;
iv. sie umfasst einen oder mehrere Zusatzstoffe ausgewählt aus:
aa) einem Chelatbildner;
bb) einer Puffersubstanz; und/oder
cc) einem Konservierungsmittel
und/oder
v. die flüssige Zusammensetzung wird durch Mischen von zwei oder mehreren Zusammensetzungen erhalten, wobei eine der Zusammensetzungen ein chaotropes Agens umfasst und eine der Zusammensetzungen eine alkalische Lösung ist, die ein alkalisches Agens, vorzugsweise NaOH oder KOH, vorzugsweise in einer Konzentration von 1,0 N bis 2,0 N, umfasst;
und Freisetzen von Nukleinsäuren von den Zellen und Denaturieren der freigesetzten Nukleinsäuren, wobei vorzugsweise ein Erwärmungsschritt durchgeführt wird, um die Freisetzung und/oder Denaturierung der Nukleinsäuren zu unterstützen;
d) Erzeugen eines Hybrids zwischen der Zielnukleinsäure und einer für die Zielnukleinsäure spezifischen Sonde, wobei die magnetischen Partikel vor Schritt d) nicht magnetisch abgetrennt werden und wobei Schritt d) umfasst:
- Kontaktieren der Probe, die die freigesetzten und denaturierten Nukleinsäuren und magnetische Partikel, die zum Binden der Zellen verwendet wurden, umfasst mit einer oder mehrere für die Zielnukleinsäure spezifischen Sonden unter Bedingungen, die es den Sonden und einzelsträngigen Zielnukleinsäuremolekülen ermöglichen zu hybridisieren, so dass doppelsträngige Nukleinsäurehybride ausgebildet werden;
- Fangen der doppelsträngigen Nukleinsäure-Hybride unter Verwendung eines ersten Bindungsmittels, das die doppelsträngigen Nukleinsäurehybride bindet, wodurch ein Komplex aus doppelsträngigem Nukleinsäurehybrid und erstem Bindungsmittel gebildet wird;
- Trennen des Komplexes aus doppelsträngigem Nukleinsäurehybrid und erstem Bindungsmittel von ungebundenen Nukleinsäuren, wodurch zumindest ein Teil der magnetischen Partikel ebenfalls entfernt wird;
- Binden des Komplexes mit einem weiteren Bindungsmittel, das mit einem detektierbaren Marker markiert ist, um einen Komplex aus doppelsträngigem Nukleinsäurehybrid, erstem Bindungsmittel und markiertem Bindungsmittel zu bilden;
- Waschen des Komplexes aus doppelsträngigem Nukleinsäurehybrid, erstem Bindungsmittel und markiertem Bindungsmittel, wobei das Waschen restliche magnetische Partikel, falls noch anwesend, entfernt;
e) Detektion der Anwesenheit oder Abwesenheit von doppelsträngigen Nukleinsäurehybriden, wodurch die Anwesenheit oder Abwesenheit der Zielnukleinsäure angezeigt wird.

12. Verfahren nach einem oder mehreren der Ansprüche 1 bis 11, insbesondere nach den Ansprüchen 9 bis 11, wobei die Anionenaustauscher aufweisende Oberfläche durch magnetische Partikel bereitgestellt wird, die Polyethylenimin als Anionenaustauscher umfassen, und wobei nach dem Kontaktieren der magnetischen Partikel mit der Zellprobe in Schritt a) die magnetischen Partikel vorzugsweise in einer Konzentration in der resultierenden Mischung enthalten sind, die ausgewählt ist aus 50 µg/ml bis 2000 µg/ml, 75 µg/ml bis 1750 µg/ml, 100 µg/ml bis 1500 µg/ml, 125 µg/ml bis 1250 µg/ml und 150 µg/ml bis 1100 µg/ml;
und wobei optional die gesammelten Zellen, die an die magnetischen Partikel gebunden sind, in Schritt c) mit einem Volumen der flüssigen Zusammensetzung kontaktiert werden, das ausgewählt ist aus 50µl bis 200µl, 60µl bis 175µl, 70µl bis 150µl, 80µl bis 125µl und 85µl bis 100µl, und wobei die flüssige Zusammensetzung ein chaotropes Mittel umfasst und einen aus pH 12 oder höher, pH 12,5 oder höher, pH 13 oder höher oder pH 13,25 oder höher ausgewählten pH-Wert aufweist.

13. Verfahren nach einem oder mehreren der Ansprüche 1 bis 12, das eines oder mehrere, vorzugsweise zumindest zwei, noch bevorzugter zumindest drei der folgenden Eigenschaften i. bis ix. aufweist:
i. das Verfahren ist ein automatisiertes Verfahren zum Analysieren einer klinischen Probe in Bezug auf einen Krankheitszustand und/oder zum Bestimmung der Anwesenheit oder Abwesenheit eines Krankheitserregers in einer klinischen Probe;
ii. die zu detektierende Nukleinsäure ist eine Pathogen-Nukleinsäure;
iii. die zu detektierende Nukleinsäure ist eine virale Nukleinsäure;
iv. die zu detektierende Nukleinsäure ist eine HPV-Nukleinsäure;
v. das Verfahren wird für HPV-Diagnostik verwendet;
vi. die in der Zellprobe enthaltenen Zellen sind epitheliale Zellen;
vii. die in der Zellprobe enthaltenen Zellen sind Zervixzellen;
viii. die Zellprobe ist eine in einem flüssigkeitsbasierten Zytologiemedium ausgewählt aus SUREPATH® und PRESERVCYT® gesammelte zervikale Probe; und/oder
ix. das Verfahren ist ein automatisiertes Verfahren zum Bestimmten der Anwesenheit oder Abwesenheit von HPV-Nukleinsäuren, vorzugsweise Hochrisiko-HPV-Nukleinsäuren, in in einem flüssigkeitsbasierten Zytologiemedium gesammelten Zervixproben.

14. Verwendung eines Kits in dem Verfahren nach einem oder mehreren der Ansprüche 1 bis 13, wobei das Kit umfasst:
a) magnetische Partikel, wobei die Oberfläche der magnetischen Partikel Anionenaustauscher aufweist;
b) ein Denaturierungsmittel, das eine alkalische Lösung ist, die eine Base umfasst; und
c) eine Zusammensetzung, die ein chaotropes Agens umfasst.

15. Verwendung nach Anspruch 14, wobei das Kit eine mehrere Vertiefungen aufweisende Kartusche umfasst, wobei
a) eine erste Vertiefung der Kartusche magnetische Partikel umfasst, wobei die Oberfläche der magnetischen Partikel Anionenaustauscher aufweist;
b) eine zweite Vertiefung der Kartusche ein Denaturierungsmittel umfasst, das eine alkalische Lösung ist, die eine Base, vorzugsweise NaOH oder KOH, vorzugsweise in einer von 1,0 N bis 2,0 N ausgewählten Konzentration, umfasst; und
c) eine dritte Vertiefung der Kartusche eine Zusammensetzung umfasst, die ein chaotropes Agens und optional ein oder mehr Zusatzstoffe umfasst, die ausgewählt sind aus der Gruppe von Chelatbildnern, Puffersubstanzen und Konservierungsmitteln,
wobei die Kartusche optional abgedichtet ist.

16. Verwendung nach Anspruch 14 oder 15, wobei die magnetischen Partikel eine oder mehrere der folgenden Eigenschaften aufweisen:
i) die magnetischen Partikel sind mit Polyethylenimin als Anionenaustauscher funktionalisiert;
ii) die magnetischen Partikel sind in einer Partikelsuspension enthalten, die einen aus 7,5 oder weniger, 7 oder weniger und 6,5 oder weniger ausgewählten pH-Wert aufweist; und/oder
iii) die magnetischen Partikel sind in einer Partikelsuspension enthalten, wobei die Partikel in der Suspension in einer aus 5 mg/ml bis 50 mg/ml, 10 mg/ml bis 20 mg/ml und 12,5 mg/ml bis 17,5 mg/ml ausgewählten Konzentration enthalten sind.

17. Verwendung nach einem oder mehreren der Ansprüche 14 bis 16, wobei das Kit eine oder mehrere der folgenden Eigenschaften aufweist:
a) das Kit umfasst eine Elutionsplatte, die mehrere Vertiefungen aufweist;
b) das Kit umfasst zumindest eine für die Hybridisierung an eine Zielnukleinsäure geeignete Sonde, wobei die Sonde vorzugsweise eine RNA-Sonde ist;
c) das Kit umfasst zumindest ein Bindungsmittel, das ein doppelsträngiges Nukleinsäurehybrid binden kann, wobei das Bindungsmittel vorzugsweise an einem festen Träger befestigt ist;
d) das Kit umfasst einen oder mehrere Waschpuffer;
e) das Kit umfasst zumindest ein weiteres Bindungsmittel, das eine detektierbare Markierung umfasst, vorzugsweise alkalische Phosphatase;
f) das Kit umfasst ein oder mehrere Detektionsreagenzien; und/oder
g) das Kit umfasst zumindest eine für die Hybridisierung mit einer HPV-Zielnukleinsäure geeignete Sonde;
h) das Denaturierungsmittel ist NaOH oder KOH;
i) das Denaturierungsmittel umfasst die Base in einer aus von 1,0 N bis 2,0 N ausgewählten Konzentration;
j) die Zusammensetzung umfasst ein oder mehr Zusatzstoffe, die ausgewählt sind aus der Gruppe von Chelatbildnern, Puffersubstanzen und Konservierungsmitteln.

## Revendications

1. Procédé de détermination de la présence ou de l'absence d'un acide nucléique cible dans un échantillon de cellules, ledit procédé comprenant :
a) mettre en contact des particules magnétiques, ayant une surface comprenant des entités échangeuses d'anions, avec l'échantillon dans des conditions appropriées pour induire une liaison entre les cellules et ladite surface ;
b) séparer les particules magnétiques, avec les particules fixées, d'avec l'échantillon restant, afin de recueillir les cellules, les particules magnétiques avec les cellules fixées étant traitées à l'aide d'un champ magnétique ;
c) libérer les acides nucléiques d'avec les cellules ;
d) former un hybride entre l'acide nucléique cible et une sonde spécifique de l'acide nucléique cible en présence des particules magnétiques ; et
e) détecter la présence ou l'absence d'hybride.

2. Procédé selon la revendication 1, dans lequel la surface comprenant des entités échangeuses d'anions présente au moins une, au moins deux, au moins trois, au moins quatre ou la totalité des caractéristiques suivantes :
i. les entités échangeuses d'anion comprennent des groupes amino ;
ii. la surface comprend de la polyéthylène-imine en tant qu'entité échangeuse d'anions ;
iii. la surface d'échange d'anions est résistante à la dégradation dans les conditions utilisées dans l'étape d) ;
iv. la surface est fournie par des particules magnétiques ayant une taille moyenne choisie parmi 5 µm ou moins, 3 µm ou moins, 2 µm ou moins et 1,5 µm ou moins, et/ou
v. la surface est fournie par des particules magnétiques qui comprennent des groupes carboxy à la surface des particules, au moins une partie desdits groupes carboxy étant fonctionnalisée avec de la polyéthylène-imine pour donner des entités échangeuses d'anions.

3. Procédé selon la revendication 1 ou 2, dans lequel les cellules dans l'échantillon de cellules sont contenues dans un milieu liquide, de préférence un milieu pour cytologie à base liquide ;
et dans lequel en option, après la mise en contact des particules magnétiques avec l'échantillon, les particules magnétiques sont contenues dans le milieu résultant à une concentration choisie parmi des concentrations de 50 µg/ml à 2 000 µg/ml, 75 µg/ml à 1 750µg/ml, 100 µg/ml à 1 500 µg/ml, 125 µg/ml à 1 250 µg/ml et 150 µg/ml à 1 100 µg/ml ; et/ou
dans lequel en option, dans l'étape a), la liaison des cellules a lieu à un pH choisi dans un intervalle de pH de 3 à 8,5, 3,5 à 8,0, 3,5 à 7,75, 3,5 à 7,5, 3,5 à 7,25 ou 3,75 à 7.

4. Procédé selon une ou plusieurs des revendications 1 à 3, dans lequel les cellules recueillies qui sont fixées à la surface d'échange d'anions sont mises en contact avec une composition liquide qui aide la libération des acides nucléiques d'avec les cellules dans l'étape c) et dans lequel en option on effectue une étape de chauffage dans l'étape c) pour faciliter la libération et/ou la dénaturation des acides nucléiques.

5. Procédé selon la revendication 4, dans lequel la composition liquide présente une ou plusieurs, de préférence au moins deux, au moins trois et de façon plus particulièrement préférée la totalité des caractéristiques i. à v. suivantes :
i. elle comprend un agent chaotropique ;
ii. elle présente un pH alcalin ;
iii. elle présente un pH choisi parmi pH 12 ou plus, pH 12,5 ou plus, pH 13 ou plus ou pH 13,25 ou plus ;
iv. elle comprend un ou plusieurs additifs choisis parmi
aa. un agent chélateur ;
bb. un agent tampon ; et/ou
cc. un conservateur
et/ou
v. la composition liquide est obtenue par mélange de deux ou plus de deux compositions, une desdites compositions comprenant un agent chaotropique et une desdites compositions étant une solution alcaline comprenant un agent alcalin.

6. Procédé selon une ou plusieurs des revendications 1 à 5, dans lequel on effectue un essai de capture d'hybrides, et dans lequel les étapes d) et e) comprennent les étapes suivantes :
étape d)
- mettre en contact l'échantillon comprenant les acides nucléiques libérés et dénaturés et les particules magnétiques avec une ou plusieurs sondes spécifiques de l'acide nucléique cible, dans des conditions permettant aux sondes et aux molécules d'acide nucléique cible simple brin de s'hybrider en formant des hybrides d'acides nucléiques double brin, de préférence des hybrides ARN/ADN étant formés ;
- capturer les hybrides d'acides nucléiques double brin à l'aide d'un agent de liaison qui lie l'hybride d'acides nucléiques double brin ;
- en option séparer les hybrides d'acides nucléiques double brin capturés d'avec les acides nucléiques non liés ;
étape e)
- détecter la présence ou l'absence d'hybrides d'acides nucléiques double brin, ce qui indique la présence ou l'absence de l'acide nucléique cible ;
et dans lequel les particules magnétiques sont présentes pendant la formation et la capture de l'hybride et ne sont pas éliminées dans l'étape d) à l'aide d'un aimant mais sont éliminées lors de la séparation des restes d'échantillon et/ou lors de l'exécution d'une ou de plusieurs étapes de lavage avant l'excéution de l'étape de détection e).

7. Procédé selon une ou plusieurs des revendications 1 à 6, le procédé comprenant les étapes d) et e) suivantes :
étape d)
- mettre en contact l'échantillon comprenant les acides nucléiques libérés et dénaturés et les particules magnétiques, qui ont été utilisées pour la fixation des cellules, avec une ou plusieurs sondes spécifiques de l'acide nucléique cible, dans des conditions permettant aux sondes et aux molécules d'acide nucléique cible simple brin de s'hybrider en formant des hybrides d'acides nucléiques double brin ;
- capturer les hybrides d'acides nucléiques double brin ;
- séparer les hybrides d'acides nucléiques double brin d'avec les acides nucléiques non liés, ce qui élimine également au moins une partie des particules magnétiques ;
étape e)
- détecter la présence ou l'absence d'hybrides d'acides nucléiques double brin.

8. Procédé selon une ou plusieurs des revendications 1 à 7, le procédé comprenant les étapes d) et e) suivantes :
étape d)
- mettre en contact l'échantillon comprenant les acides nucléiques libérés et dénaturés et les particules magnétiques, qui ont été utilisées pour la fixation des cellules, avec une ou plusieurs sondes spécifiques de l'acide nucléique cible, dans des conditions permettant aux sondes et aux molécules d'acide nucléique cible simple brin de s'hybrider en formant des hybrides d'acides nucléiques double brin ;
- capturer les hybrides d'acides nucléiques double brin à l'aide d'un premier agent de liaison qui lie les hybrides d'acides nucléiques double brin, ce qui forme un complexe hybride d'acides nucléiques double brin/premier agent de liaison ;
- séparer du complexe hybride d'acides nucléiques double brin/premier agent de liaison d'avec les acides nucléiques non liés, ce qui élimine également au moins une partie des particules magnétiques ;
- lier le complexe avec un agent de liaison supplémentaire qui est marqué avec un marqueur détectable, pour former un complexe hybride d'acides nucléiques double brin/premier agent de liaison/agent de liaison marqué ;
- laver le complexe hybride d'acides nucléiques double brin/premier agent de liaison/agent de liaison marqué, ledit lavage éliminant les particules magnétiques restantes si elles sont encore présentes ;
étape e)
- détecter la présence ou l'absence du marqueur de l'agent de liaison supplémentaire, ce qui indique la présence ou l'absence de l'acide nucléique cible.

9. Procédé selon une ou plusieurs des revendications 1 à 8, comprenant les étapes suivantes :
a) mettre en contact des particules magnétiques, présentant une surface comprenant des entités échangeuses d'anions, avec l'échantillon de cellules, dans des conditions appropriées pour induire une liaison entre les cellules et ladite surface, la fixation des cellules ayant lieu à un pH auquel les groupes d'échange d'anions des entités échangeuses d'anions sont chargés positivement, de préférence la fixation des cellules ayant lieu à un pH qui est choisi dans un intervalle de pH de 3 à 8,5, 3,5 à 8, 3,75 à 7,75, 4 à 7,5, 4,25 à 7,25 ou 4,5 à 7 ;
b) séparer les particules magnétiques, avec les cellules fixées, d'avec l'échantillon restant, pour recueillir les cellules, les particules magnétiques avec les cellules fixées étant traitées à l'aide d'un champ magnétique ;
c) libérer les acides nucléiques d'avec les cellules et dénaturer les acides nucléiques libérés ;
d) former un hybride entre l'acide nucléique cible et une sonde spécifique de l'acide nucléique cible, l'étape d) comprenant :
- mettre en contact l'échantillon comprenant les acides nucléiques libérés et dénaturés et les particules magnétiques avec une ou plusieurs sondes spécifiques de l'acide nucléique cible, dans des conditions permettant aux sondes et aux molécules d'acide nucléique cible simple brin de s'hybrider en formant des hybrides d'acides nucléiques double brin, de préférence des hybrides ARN/ADN étant formés ;
- capturer les hybrides d'acides nucléiques double brin à l'aide d'un agent de liaison qui lie l'hybride d'acides nucléiques double brin ;
- en option, séparer les hybrides d'acides nucléiques double brin capturés d'avec les acides nucléiques non liés ;
- en option, laver les hybrides d'acides nucléiques double brin capturés ;
e) détecter la présence ou l'absence d'hybrides d'acides nucléiques double brin, ce qui indique la présence ou l'absence de l'acide nucléique cible.

10. Procédé selon une ou plusieurs des revendications 1 à 9, en particulier selon la revendication 3, comprenant les étapes suivantes :
a) mettre en contact des particules magnétiques, présentant une surface comprenant des entités échangeuses d'anions, avec l'échantillon de cellules, dans des conditions appropriées pour induire une liaison entre les cellules et ladite surface, les entités échangeuses d'anions comprenant au moins un groupe amino primaire, au moins un groupe amino secondaire et/ou au moins un groupe amino tertiaire, la fixation des cellules ayant lieu à un pH qui est choisi dans un intervalle de pH de 3,5 à 8, 3,75 à 7,75, 4 à 7,5, 4,25 à 7,25 ou 4,5 à 7, les groupes amino des entités échangeuses d'anions étant chargés positivement audit pH et aucune adaptation des conditions de liaison n'ayant lieu ;
b) séparer les particules magnétiques, avec les cellules fixées, d'avec l'échantillon restant, pour recueillir les cellules, les particules magnétiques avec les cellules fixées étant traitées à l'aide d'un champ magnétique ;
c) mettre en contact les cellules recueillies, qui sont fixées à la surface d'échange d'anions, avec une composition liquide qui aide la libération des acides nucléiques d'avec les cellules, la composition liquide présentant une ou plusieurs, de préférence au moins deux, au moins trois et de façon plus particulièrement préférée la totalité des caractéristiques i. à v. suivantes :
i. elle comprend un agent chaotropique ;
ii. elle présente un pH alcalin ;
iii. elle présente un pH choisi parmi pH 12 ou plus, pH 12,5 ou plus, pH 13 ou plus ou pH 13,25 ou plus ;
iv. elle comprend un ou plusieurs additifs choisis parmi
aa. un agent chélateur ;
bb. un agent tampon ; et/ou
cc. un conservateur
et/ou
v. la composition liquide est obtenue par mélange de deux ou plus de deux compositions, une desdites compositions comprenant un agent chaotropique et une desdites compositions étant une solution alcaline comprenant un agent alcalin, de préférence NaOH ou KOH, de préférence à une concentration de 1,0 N à 2,0 N ;
et libérer les acides nucléiques d'avec les cellules et dénaturation des acides nucléiques libérés, de préférence une étape de chauffage étant effectuée pour faciliter la libération et/ou la dénaturation des acides nucléiques ;
d) former un hybride entre l'acide nucléique cible et une sonde spécifique de l'acide nucléique cible, l'étape d) comprenant :
- mettre en contact l'échantillon, comprenant les acides nucléiques libérés et dénaturés et les particules magnétiques, avec une ou plusieurs sondes spécifiques de l'acide nucléique cible, dans des conditions permettant aux sondes et aux molécules d'acide nucléique cible simple brin de s'hybrider en formant des hybrides d'acides nucléiques double brin, de préférence des hybrides ARN/ADN étant formés ;
- capturer les hybrides d'acides nucléiques double brin à l'aide d'un agent de liaison qui lie l'hybride d'acides nucléiques double brin ;
- en option, séparer les hybrides d'acides nucléiques double brin capturés d'avec les acides nucléiques non liés ;
- en option, laver les hybrides d'acides nucléiques double brin capturés ;
e) détecter la présence ou l'absence d'hybrides d'acides nucléiques double brin, ce qui indique la présence ou l'absence de l'acide nucléique cible.

11. Procédé selon une ou plusieurs des revendications 1 à 10, en particulier selon la revendication 3, comprenant les étapes suivantes :
a) mettre en contact les particules magnétiques, présentant une surface comprenant des entités échangeuses d'anions, avec l'échantillon de cellules, dans des conditions appropriées pour induire une liaison entre les cellules et ladite surface, la fixation des cellules ayant lieu à un pH qui est choisi dans un intervalle de pH de 3,5 à 8, 3,75 à 7,75, 4 à 7,5, 4,25 à 7,25 ou 4,5 à 7, les groupes d'échange d'anions des entités échangeuses d'anions étant chargés positivement audit pH et, de préférence, aucune adaptation des conditions de liaison n'ayant lieu ;
b) séparer les particules magnétiques, avec les cellules fixées, d'avec l'échantillon restant, pour recueillir les cellules, les particules magnétiques avec les cellules fixées étant traitées à l'aide d'un champ magnétique ;
c) mettre en contact les cellules recueillies, qui sont fixées aux particules magnétiques, avec une composition liquide qui facilite la libération des acides nucléiques d'avec les cellules, la composition liquide présentant une ou plusieurs, de préférence au moins deux, au moins trois et de façon plus particulièrement préférée la totalité des caractéristiques i. à v. suivantes :
i. elle comprend un agent chaotropique ;
ii. elle présente un pH alcalin ;
iii. elle présente un pH choisi parmi pH 12 ou plus, pH 12,5 ou plus, pH 13 ou plus ou pH 13,25 ou plus ;
iv. elle comprend un ou plusieurs additifs choisis parmi
aa. un agent chélateur ;
bb. un agent tampon ; et/ou
cc. un conservateur
et/ou
v. la composition liquide est obtenue par mélange de deux ou plus de deux compositions, une desdites compositions comprenant un agent chaotropique et une desdites compositions étant une solution alcaline comprenant un agent alcalin, de préférence NaOH ou KOH, de préférence à une concentration de 1,0 N à 2,0 N ;
et libérer les acides nucléiques d'avec les cellules et dénaturation des acides nucléiques libérés, de préférence une étape de chauffage étant effectuée pour faciliter la libération et/ou la dénaturation des acides nucléiques ;
d) former un hybride entre l'acide nucléique cible et une sonde spécifique de l'acide nucléique cible, les particules magnétiques n'étant pas séparées magnétiquement avant l'étape d) et l'étape d) comprenant :
- mettre en contact l'échantillon, comprenant les acides nucléiques libérés et dénaturés et les particules magnétiques qui ont été utilisées pour la fixation des cellules, avec une ou plusieurs sondes spécifiques de l'acide nucléique cible, dans des conditions permettant aux sondes et aux molécules d'acide nucléique cible simple brin de s'hybrider en formant des hybrides d'acides nucléiques double brin ;
- capturer les hybrides d'acides nucléiques double brin à l'aide d'un premier agent de liaison qui lie les hybrides d'acides nucléiques double brin, ce qui forme un complexe hybride d'acides nucléiques double brin/premier agent de liaison ;
- séparer le complexe hybride d'acides nucléiques double brin/premier agent de liaison d'avec les acides nucléiques non liés, ce qui élimine également au moins une partie des particules magnétiques ;
- lier le complexe avec un agent de liaison supplémentaire qui est marqué avec un marqueur détectable, pour former un complexe hybride d'acides nucléiques double brin/ premier agent de liaison/agent de liaison marqué ;
- laver le complexe hybride d'acides nucléiques double brin/premier agent de liaison/agent de liaison marqué, ledit lavage éliminant les particules magnétiques restantes si elles sont encore présentes ;
e) détecter la présence ou l'absence d'hybrides d'acides nucléiques double brin, ce qui indique la présence ou l'absence de l'acide nucléique cible.

12. Procédé selon une ou plusieurs des revendications 1 à 11, en particulier des revendications 9 à 11, dans lequel la surface comprenant des entités échangeuses d'anions est fournie par des particules magnétiques comprenant de la polyéthylèneimine en tant qu'entité échangeuse d'anions et dans lequel après la mise en contact des particules magnétiques avec l'échantillon de cellules dans l'étape a), les particules magnétiques sont de préférence contenues dans le mélange résultant à une concentration choisie parmi des concentrations de 50 µg/ml à 2 000 µg/ml, 75 µg/ml à 1 750 µg/ml, 100 µg/ml à 1 500 µg/ml, 125 µg/ml à 1 250 µg/ml et 150 µg/ml à 1 100 µg/ml ;
et dans lequel en option les cellules recueillies qui sont fixées au particules magnétiques sont mises en contact dans l'étape c) avec un volume de ladite composition liquide qui est choisi parmi 50 µl à 200 µl, 60 µl à 175 µl, 70 µl à 150 µl, 80 µl à 125 µl et 85 µl à 100 µl et dans lequel la composition liquide comprend un agent chaotropique et a un pH choisi parmi pH 12 ou plus, pH 12,5 ou plus, pH 13 ou plus ou pH 13,25 ou plus.

13. Procédé selon une ou plusieurs des revendications 1 à 12, ayant une ou plusieurs, de préférence au moins deux, de façon plus particulièrement préférée au moins trois des caractéristiques i. à ix. suivantes :
i. ledit procédé est un procédé automatisé pour l'analyse d'un échantillon clinique pour un état pathologique et/ou pour la détermination de la présence ou de l'absence d'un agent pathogène dans un échantillon clinique ;
ii. l'acide nucléique cible à détecter est un acide nucléique d'un agent pathogène ;
iii. l'acide nucléique cible à détecter est un acide nucléique viral ;
iv. l'acide nucléique cible à détecter est un acide nucléique de HPV ;
v. le procédé est utilisé pour le diagnostic de HPV ;
vi. les cellules contenues dans l'échantillon de cellules sont des cellules épithéliales ;
vii. les cellules contenues dans l'échantillon sont des cellules de col utérin ;
viii. l'échantillon de cellules est un échantillon de col utérin recueilli dans un milieu pour cytologie à base liquide choisi parmi SUREPATH® et PRESERVCYT® ; et/ou
ix. ledit procédé est un procédé automatisé pour la détermination de la présence ou de l'absence d'acides nucléiques de HPV, de préférence d'acides nucléiques de HPV à haut risque, dans des échantillons de col utérin recueillis dans un milieu pour cytologie à base liquide.

14. Utilisation d'un nécessaire dans le procédé selon une ou plusieurs des revendications 1 à 13, le nécessaire comprenant :
a) des particules magnétiques, la surface des particules magnétiques comprenant des entités échangeuses d'anions ;
b) un agent de dénaturation qui est une solution alcaline comprenant une base ; et
c) une composition comprenant un agent chaotropique.

15. Utilisation selon la revendication 14, dans laquelle le nécessaire comprend une cartouche comprenant de multiples réceptacles,
a) un premier réceptacle de ladite cartouche comprenant des particules magnétiques, la surface des particules magnétiques comprenant des entités échangeuses d'anions ;
b) un deuxième réceptacle de ladite cartouche comprenant un agent de dénaturation qui est une solution alcaline comprenant une base, de préférence NaOH ou KOH, de préférence à une concentration choisie parmi des concentrations de 1,0 N à 2,0 N ; et
c) un troisième réceptacle de ladite cartouche comprenant une composition comprenant un agent chaotropique et en option un ou plusieurs additifs choisis dans le groupe des agents chélateurs, agents tampon et conservateurs,
la cartouche étant en option scellée.

16. Utilisation selon la revendication 14 ou 15, dans laquelle les particules magnétiques présentent une ou plusieurs des caractéristiques suivantes :
i) les particules magnétiques son fonctionnalisées avec une polyéthylène-imine en tant qu'entités échangeuses d'anions ;
ii) les particules magnétiques sont contenues dans une suspension de particules ayant un pH choisi parmi 7,5 ou moins, 7 ou moins et 6,5 ou moins ; et/ou
iii) les particules magnétiques sont contenues dans une suspension de particules, les particules étant contenues dans la suspension à une concentration choisie parmi des concentrations de 5 mg/ml à 50 mg/ml, 10 mg/ml à 20 mg/ml et 12,5 mg/ml à 17,5 mg/ml.

17. Utilisation selon une ou plusieurs des revendications 14 à 16, dans laquelle le nécessaire présente une ou plusieurs des caractéristiques suivantes :
a) le nécessaire comprend une plaque d'élution comprenant de multiple puits ;
b) le nécessaire comprend au moins une sonde appropriée à l'hybridation avec un acide nucléique cible, ladite sonde étant de préférence une sonde d'ARN ;
c) le nécessaire comprend au moins un agent de liaison qui est capable de lier un hybride d'acides nucléiques double brin, ledit agent de liaison étant de préférence fixé à un support solide ;
d) le nécessaire comprend un ou plusieurs tampons de lavage ;
e) le nécessaire comprend au moins un agent de liaison supplémentaire comprenant un marqueur détectable, de préférence une phosphatase alcaline ;
f) le nécessaire comprend un ou plusieurs réactifs de détection ; et/ou
g) le nécessaire comprend au moins une sonde qui est appropriée à l'hybridation avec un acide nucléique cible de HPV ;
h) l'agent de dénaturation est NaOH ou KOH ;
i) l'agent de dénaturation comprend la base à une concentration choisie parmi des concentrations de 1,0 N à 2,0 N ;
j) la composition comprend un ou plusieurs additifs choisis dans le groupe des agents chélateurs, agents tampon et conservateurs.
